(19) Europäisches Patentamt
European Patent Office
Office européen des brevets

(11) **EP 4 245 321 A1**

(12) **EUROPEAN PATENT APPLICATION**
published in accordance with Art. 153(4) EPC

(43) Date of publication:
**20.09.2023 Bulletin 2023/38**

(21) Application number: **21891880.3**

(22) Date of filing: **10.11.2021**

(51) International Patent Classification (IPC):
***A61K 47/68*** (2017.01) ***A61K 31/4745*** (2006.01)
***A61K 39/395*** (2006.01) ***A61K 47/55*** (2017.01)
***A61P 35/00*** (2006.01) ***A61P 35/02*** (2006.01)
***A61P 43/00*** (2006.01)

(52) Cooperative Patent Classification (CPC):
**A61K 31/4745; A61K 39/395; A61K 47/55; A61K 47/68; A61P 35/00; A61P 35/02; A61P 43/00**

(86) International application number:
**PCT/JP2021/041255**

(87) International publication number:
**WO 2022/102634 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.11.2020 JP 2020188084**

(71) Applicant: **Daiichi Sankyo Company, Limited**
**Tokyo 103-8426 (JP)**

(72) Inventors:
- **SUE, Mayumi**
  **Tokyo 103-8426 (JP)**
- **TSUBAKI, Takuya**
  **Tokyo 103-8426 (JP)**
- **ISHIMOTO, Yoko**
  **Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) COMBINATION OF ANTIBODY-DRUG CONJUGATE WITH ANTI-SIRPALPHA ANTIBODY

(57) A pharmaceutical composition and a method of treatment having excellent antitumor effect and safety are provided. A pharmaceutical composition and a method of treatment wherein an antibody-drug conjugate, in which a drug-linker and an antibody are bound by a thioether bond represented by the following formula (A represents the binding position with an antibody), and a SIRPα-CD47 interaction inhibitor, such as an anti-SIRPα antibody, are administrated in combination. Alternatively, a pharmaceutical composition and a method of treatment wherein said antibody-drug conjugate and SIRPα-CD47 interaction inhibitor, such as an anti-SIRPα antibody, are administrated in combination and are used for the treatment of a disease improved by an action of activating antitumor immunity.

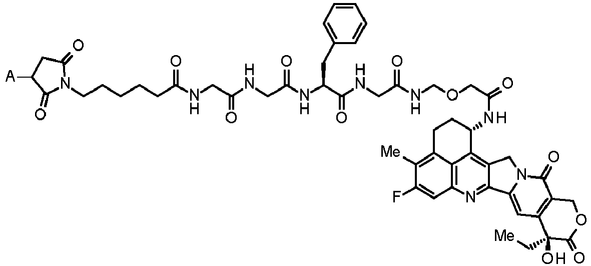

EP 4 245 321 A1

A—

## Description

[Technical Field]

The present invention relates to a pharmaceutical composition, wherein a specific antibody-drug conjugate and an anti-SIRPα antibody are administered in combination, and/or a method of treatment comprising administering a specific antibody-drug conjugate and an anti-SIRPα antibody in combination to a subject.

[Background Art]

SIRPα (SHPS-1) is a single transmembrane molecule belonging to the Ig superfamily present in myeloid cells such as macrophages, dendritic cells and neutrophils, and glial cells (Non-Patent Reference 1). The extracellular region thereof consists of a single IgV domain and two IgC domains. The IgV domain, which is a connecting position to CD47, is reported to have 10 variants in humans (Non-Patent Reference 2). On the other hand, the intracellular region thereof contains immunoreceptor tyrosine-based inhibition motifs (ITIM). When ITIM binds to CD47, binding to tyrosine dephosphorylation enzymes, SHP-1 and SHP-2, is induced, and a suppressive signal is transmitted.

It is reported that SIRPα-CD47 interaction causes a physiological phenomenon, i.e., CD47 on red blood cells binds to SIRPα on macrophages to transmit a "Don't eat me" signal, with the result that unwanted phagocytosis by red blood cells can be avoided (Non-Patent Reference 3). Also, in the tumor microenvironment, it is suggested that, when CD47, which is highly expressed on tumor cells, binds to SIRPα on macrophages and dendritic cells, phagocytic activity to engulf tumor cells is suppressed. When phagocytic activity is suppressed, the subsequent tumor antigen presentation to T cells and further subsequent tumor immune response will be suppressed. Thus, an immune phenomenon, that is, phagocytosis of tumor cells, is considered as a checkpoint of entry of a tumor antigen.

SIRPα-CD47 is the only phagocytosis suppressive molecule presently verified. An inhibitory antibody against this molecule is expected to have the potential to serve as a novel checkpoint inhibitor against targets other than T cells and to be widely effective in patients resistant to conventional immune checkpoint inhibitors.

Recently, patents relating to anti-SIRPα antibodies have been reported one after another by various companies (Patent References 1, 2 and 3). In the Examples of the individual references, differences in binding activity to various variants and family molecules (e.g. SIRPβ1, SIRPγ) and differences in antibody IgG subclasses are disclosed. For example, OSE-172, which is an IgG4Pro antibody, binds to SIRPα V1 and SIRPβ1 and neither to SIRPα V2 nor SIRPy; KWAR23, which is a IgG1N279A antibody, binds to all 10 types of SIRPα variant and family molecules, SIRPβ1 and SIRPγ; and ADU-1805, which is an IgG2 antibody, binds to all 10 types of SIRPα variant and SIRPγ. It is still unknown which of the antibodies is the most appropriate as a medicine. Efforts are underway to obtain an excellent antibody. As an example of such efforts, the antibody described in Patent Reference 4 can be exemplified.

An antibody-drug conjugate (ADC) having a drug with cytotoxicity conjugated to an antibody capable of binding to an antigen expressed on the surface of cancer cells and cellular internalization, can deliver the drug selectively to cancer cells and can thus be expected to cause accumulation of the drug within cancer cells and to kill the cancer cells.

As one such antibody-drug conjugate, an antibody-drug conjugate comprising an antibody and a derivative of exatecan, which is a topoisomerase I inhibitor, as its components is known (Patent References 5 to 11, Non-Patent References 4 to 7).

Patent References 5 to 11 disclose that an antibody-drug conjugate as mentioned above can be administered in combination with any one of various cancer therapeutic agents.

However, no test results showing a superior combined effect when the foregoing antibody-drug conjugate is used in combination with an anti-SIRPα antibody, nor any scientific basis for suggesting such a test result, has yet been published.

[Citation List]

[Patent References]

[Patent Reference1] International Publication No. WO 2017/178653
[Patent Reference2] International Publication No. WO 2018/026600
[Patent Reference3] International Publication No. WO 2018/190719
[Patent Reference4] International Publication No. WO 2020/013170
[Patent Reference5] International Publication No. WO 2014/057687
[Patent Reference6] International Publication No. WO 2014/061277

[Patent Reference7] International Publication No. WO 2015/098099
[Patent Reference8] International Publication No. WO 2015/115091
[Patent Reference9] International Publication No. WO 2015/146132
[Patent Reference10] International Publication No. WO 2015/155976
[Patent Reference11] International Publication No. WO 2015/155998

[Non-Patent References]

[Non-Patent Reference1] Matozaki et al. Trends in cell biol. 2009 (19) 2, 72-80
[Non-Patent Reference2] Takenaka et al. Nat Immunol. 2007 (8) 12, 1313-1323
[Non-Patent Reference3] Matozaki et al. J. Biochem. 2014 (155) 6, 335-344
[Non-Patent Reference4] Ogitani Y. et al., Clinical Cancer Research (2016) 22 (20), 5097-5108.
[Non-Patent Reference5] Ogitani Y. et al., Cancer Science (2016) 107, 1039-1046.
[Non-Patent Reference6] Doi T, et al., Lancet Oncol 2017; 18: 1512-22.
[Non-Patent Reference7] Takegawa N, et al., Int. J. Cancer: 141, 1682-1689 (2017)

[Summary of Invention]

[Technical Problem]

The antibody-drug conjugates used in the present invention (antibody-drug conjugates containing an exatecan derivative as a component) have been confirmed to exert a superior antitumor effect even as a single agent. However, there has been a need for obtaining a method of treatment which can suppress growth of cancer cells in multiple manners and exert a further superior antitumor effect by using the antibody-drug conjugate in combination with another anticancer agent having a different mechanism of action.

An object of the present invention is to provide a pharmaceutical composition wherein a specific antibody-drug conjugate and an anti-SIRPα antibody are administrated in combination, and/or a method of treatment comprising administering a specific antibody-drug conjugate and an anti-SIRPα antibody in combination to a subject.

[Solution to Problem]

As a result of diligent studies in order to solve the above problems, the present inventors have found that combined administration of a specific antibody-drug conjugate and an anti-SIRPα antibody exhibits a superior combined effect, and thereby completed the present invention.

Thus, the present invention provides the following [1] to [232]

[1] A pharmaceutical composition, wherein an antibody-drug conjugate and an anti-SIRPα antibody are administered in combination, and

the antibody-drug conjugate is an antibody-drug conjugate in which a drug-linker represented by the following formula:

[Formula 1]

wherein A represents the connecting position to an antibody, is conjugated to the antibody via a thioether bond.

[2] The pharmaceutical composition according to [1], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[3] The pharmaceutical composition according to [2], wherein the antibody in the antibody-drug conjugate is an anti-HER2 antibody.
[4] The pharmaceutical composition according to [3], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[5] The pharmaceutical composition according to [3], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[6] The pharmaceutical composition according to any one of [3] to [5], wherein the average number of units of the drug-linker conjugated per antibody molecule in the antibody-drug conjugate is in the range of from 7 to 8.
[7] The pharmaceutical composition according to [2], wherein the antibody in the antibody-drug conjugate is an anti-HER3 antibody.
[8] The pharmaceutical composition according to [7], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[9] The pharmaceutical composition according to [8], wherein the anti-HER3 antibody lacks a lysine residue at the carboxyl terminus of the heavy chain.
[10] The pharmaceutical composition according to any one of [7] to [9], wherein the average number of units of the drug-linker conjugated per antibody molecule in the antibody-drug conjugate is in the range of from 7 to 8.
[11] The pharmaceutical composition according to [2], wherein the antibody in the antibody-drug conjugate is an anti-TROP2 antibody.
[12] The pharmaceutical composition according to [11], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[13] The pharmaceutical composition according to [12], wherein the anti-TROP2 antibody lacks a lysine residue at the carboxyl terminus of the heavy chain.
[14] The pharmaceutical composition according to any one of [11] to [13], wherein the average number of units of the drug-linker conjugated per antibody molecule in the antibody-drug conjugate is in the range of from 3.5 to 4.5.
[15] The pharmaceutical composition according to [2], wherein the antibody in the antibody-drug conjugate is an anti-B7-H3 antibody.
[16] The pharmaceutical composition according to [15], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[17] The pharmaceutical composition according to [16], wherein the anti-B7-H3 antibody lacks a lysine residue at the carboxyl terminus of the heavy chain.
[18] The pharmaceutical composition according to any one of [15] to [17], wherein the average number of units of the drug-linker conjugated per antibody molecule in the antibody-drug conjugate is in the range of from 3.5 to 4.5.
[19] The pharmaceutical composition according to [2], wherein the antibody in the antibody-drug conjugate is an anti-GPR20 antibody.
[20] The pharmaceutical composition according to [19], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[21] The pharmaceutical composition according to [20], wherein the anti-GPR20 antibody lacks a lysine residue at the carboxyl terminus of the heavy chain.
[22] The pharmaceutical composition according to any one of [19] to [21], wherein the average number of units of the drug-linker conjugated per antibody molecule in the antibody-drug conjugate is in the range of from 7 to 8.
[23] The pharmaceutical composition according to [2], wherein the antibody in the antibody-drug conjugate is an anti-CDH6 antibody.
[24] The pharmaceutical composition according to [23], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[25] The pharmaceutical composition according to [24], wherein the anti-CDH6 antibody lacks a lysine residue at the carboxyl terminus of the heavy chain.
[26] The pharmaceutical composition according to any one of [23] to [25], wherein the average number of units of the drug-linker conjugated per antibody molecule in the antibody-drug conjugate is in the range of from 7 to 8.
[27] The pharmaceutical composition according to any one of [1] to [26], wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[28] The pharmaceutical composition according to any one of [1] to [27], wherein the antibody-drug conjugate and the anti-SIRPα antibody are separately contained as active components in different formulations, and are administered simultaneously or at different times.
[29] The pharmaceutical composition according to any one of [1] to [28], wherein the composition is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[30] A pharmaceutical composition, wherein an antibody-drug conjugate and an anti-SIRPα antibody are administered in combination, and

the antibody-drug conjugate is an antibody-drug conjugate represented by the following formula:

[Formula 2]

wherein a drug-linker is conjugated to an antibody via a thioether bond; and n represents the average number of units of the drug-linker conjugated per antibody molecule.

[31] The pharmaceutical composition according to [30], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[32] The pharmaceutical composition according to [31], wherein the antibody in the antibody-drug conjugate is an anti-HER2 antibody.
[33] The pharmaceutical composition according to [32], wherein the anti-HER2 antibody is an antibody comprising a heavy chain consisting of an amino acid sequence consisting of amino acid residues 1 of 449 of SEQ ID NO: 1 and a light chain consisting of an amino acid sequence represented by amino acid residues 1 to 214 of SEQ ID NO: 2.
[34] The pharmaceutical composition according to [32], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[35] The pharmaceutical composition according to any one of [32] to [34], wherein the average number of units of the drug-linker conjugated per antibody molecule in the antibody-drug conjugate is in the range of from 7 to 8.
[36] The pharmaceutical composition according to [31], wherein the antibody in the antibody-drug conjugate is an anti-HER3 antibody.
[37] The pharmaceutical composition according to [36], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[38] The pharmaceutical composition according to [37], wherein the anti-HER3 antibody lacks a lysine residue at the carboxyl terminus of the heavy chain.
[39] The pharmaceutical composition according to any one of [36] to [38], wherein the average number of units of the drug-linker conjugated per antibody molecule in the antibody-drug conjugate is in the range of from 7 to 8.
[40] The pharmaceutical composition according to [31], wherein the antibody in the antibody-drug conjugate is an anti-TROP2 antibody.
[41] The pharmaceutical composition according to [40], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[42] The pharmaceutical composition according to [41], wherein the anti-TROP2 antibody lacks a lysine residue at the carboxyl terminus of the heavy chain.
[43] The pharmaceutical composition according to any one of [40] to [42], wherein the average number of units of the drug-linker conjugated per antibody molecule in the antibody-drug conjugate is in the range of from 3.5 to 4.5.
[44] The pharmaceutical composition according to [31], wherein the antibody in the antibody-drug conjugate is an anti-B7-H3 antibody.
[45] The pharmaceutical composition according to [44], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[46] The pharmaceutical composition according to [45], wherein the anti-B7-H3 antibody lacks a lysine residue at the carboxyl terminus of the heavy chain.

[47] The pharmaceutical composition according to any one of [44] to [46], wherein the average number of units of the drug-linker conjugated per antibody molecule in the antibody-drug conjugate is in the range of from 3.5 to 4.5.
[48] The pharmaceutical composition according to [31], wherein the antibody in the antibody-drug conjugate is an anti-GPR20 antibody.
[49] The pharmaceutical composition according to [48], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[50] The pharmaceutical composition according to [49], wherein the anti-GPR20 antibody lacks a lysine residue at the carboxyl terminus of the heavy chain.
[51] The pharmaceutical composition according to any one of [48] to [50], wherein the average number of units of the drug-linker conjugated per antibody molecule in the antibody-drug conjugate is in the range of from 7 to 8.
[52] The pharmaceutical composition according to [31], wherein the antibody in the antibody-drug conjugate is an anti-CDH6 antibody.
[53] The pharmaceutical composition according to [52], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[54] The pharmaceutical composition according to [53], wherein the anti-CDH6 antibody lacks a lysine residue at the carboxyl terminus of the heavy chain.
[55] The pharmaceutical composition according to any one of [52] to [54], wherein the average number of units of the drug-linker conjugated per antibody molecule in the antibody-drug conjugate is in the range of from 7 to 8.
[56] The pharmaceutical composition according to any one of [30] to [55], wherein the anti-SIRP$\alpha$ antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[57] The pharmaceutical composition according to any one of [30] to [56], wherein the antibody-drug conjugate and the anti-SIRP$\alpha$ antibody are separately contained as active components in different formulations, and are administered simultaneously or at different times.
[58] The pharmaceutical composition according to any one of [30] to [57], wherein the composition is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[59] A method of treatment, comprising administering an antibody-drug conjugate and an anti-SIRP$\alpha$ antibody in combination to a subject in need of treatment, wherein the antibody-drug conjugate is an antibody-drug conjugate in which a drug-linker represented by the following formula:

[Formula 3]

wherein A represents the connecting position to an antibody, is conjugated to the antibody via a thioether bond.

[60] The method of treatment according to [59], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[61] The method of treatment according to [60], wherein the antibody in the antibody-drug conjugate is an anti-HER2 antibody.

[62] The method of treatment according to [61], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[63] The method of treatment according to [61], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[64] The method of treatment according to any one of [61] to [63], wherein the average number of units of the drug-linker conjugated per antibody molecule in the antibody-drug conjugate is in the range of from 7 to 8.

[65] The method of treatment according to [60], wherein the antibody in the antibody-drug conjugate is an anti-HER3 antibody.

[66] The method of treatment according to [65], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[67] The method of treatment according to [66], wherein the anti-HER3 antibody lacks a lysine residue at the carboxyl terminus of the heavy chain.

[68] The method of treatment according to any one of [65] to [67], wherein the average number of units of the drug-linker conjugated per antibody molecule in the antibody-drug conjugate is in the range of from 7 to 8.

[69] The method of treatment according to [60], wherein the antibody in the antibody-drug conjugate is an anti-TROP2 antibody.

[70] The method of treatment according to [69], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[71] The method of treatment according to [70], wherein the anti-TROP2 antibody lacks a lysine residue at the carboxyl terminus of the heavy chain.

[72] The method of treatment according to any one of [69] to [71], wherein the average number of units of the drug-linker conjugated per antibody molecule in the antibody-drug conjugate is in the range of from 3.5 to 4.5.

[73] The method of treatment according to [60], wherein the antibody in the antibody-drug conjugate is an anti-B7-H3 antibody.

[74] The method of treatment according to [73], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[75] The method of treatment according to [74], wherein the anti-B7-H3 antibody lacks a lysine residue at the carboxyl terminus of the heavy chain.
[76] The method of treatment according to any one of [73] to [75], wherein the average number of units of the drug-linker conjugated per antibody molecule in the antibody-drug conjugate is in the range of from 3.5 to 4.5.
[77] The method of treatment according to [60], wherein the antibody in the antibody-drug conjugate is an anti-GPR20 antibody.
[78] The method of treatment according to [77], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[79] The method of treatment according to [78], wherein the anti-GPR20 antibody lacks a lysine residue at the carboxyl terminus of the heavy chain.
[80] The method of treatment according to any one of [77] to [79], wherein the average number of units of the drug-linker conjugated per antibody molecule in the antibody-drug conjugate is in the range of from 7 to 8.
[81] The method of treatment according to [60], wherein the antibody in the antibody-drug conjugate is an anti-CDH6 antibody.
[82] The method of treatment according to [81], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[83] The method of treatment according to [82], wherein the anti-CDH6 antibody lacks a lysine residue at the carboxyl terminus of the heavy chain.
[84] The method of treatment according to any one of [81] to [83], wherein the average number of units of the drug-linker conjugated per antibody molecule in the antibody-drug conjugate is in the range of from 7 to 8.
[85] The method of treatment according to any one of [59] to [84], wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[86] The method of treatment according to any one of [59] to [85], wherein the antibody-drug conjugate and the anti-SIRPα antibody are separately contained as active components in different formulations, and are administered simultaneously or at different times.
[87] The method of treatment according to any one of [59] to [86], wherein the method is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[88] A method of treatment, comprising administering an antibody-drug conjugate and an anti-SIRPα antibody in combination to a subject in need of treatment, wherein the antibody-drug conjugate is an antibody-drug conjugate represented by the formula:

[Formula 4]

wherein a drug-linker is conjugated to an antibody via a thioether bond; and n represents the average number of units of the drug-linker conjugated per antibody molecule.

[89] The method of treatment according to [88], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[90] The method of treatment according to [89], wherein the antibody in the antibody-drug conjugate is an anti-HER2 antibody.

[91] The method of treatment according to [90], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[92] The method of treatment according to [90], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[93] The method of treatment according to any one of [90] to [92], wherein the average number of units of the drug-linker conjugated per antibody molecule in the antibody-drug conjugate is in the range of from 7 to 8.

[94] The method of treatment according to [89], wherein the antibody in the antibody-drug conjugate is an anti-HER3 antibody.

[95] The method of treatment according to [94], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[96] The method of treatment according to [95], wherein the anti-HER3 antibody lacks a lysine residue at the carboxyl terminus of the heavy chain.

[97] The method of treatment according to any one of [94] to [96], wherein the average number of units of the drug-linker conjugated per antibody molecule in the antibody-drug conjugate is in the range of from 7 to 8.

[98] The method of treatment according to [89], wherein the antibody in the antibody-drug conjugate is an anti-TROP2 antibody.

[99] The method of treatment according to [98], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[100] The method of treatment according to [99], wherein the anti-TROP2 antibody lacks a lysine residue at the carboxyl terminus of the heavy chain.

[101] The method of treatment according to any one of [98] or [100], wherein the average number of units of the drug-linker conjugated per antibody molecule in the antibody-drug conjugate is in the range of from 3.5 to 4.5.

[102] The method of treatment according to [89], wherein the antibody in the antibody-drug conjugate is an anti-B7-H3 antibody.

[103] The method of treatment according to [102], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[104] The method of treatment according to [103], wherein the anti-B7-H3 antibody lacks a lysine residue at the carboxyl terminus of the heavy chain.

[105] The method of treatment according to any one of [102] to [104], wherein the average number of units of the

drug-linker conjugated per antibody molecule in the antibody-drug conjugate is in the range of from 3.5 to 4.5.

[106] The method of treatment according to [89], wherein the antibody in the antibody-drug conjugate is an anti-GPR20 antibody.

[107] The method of treatment according to [106], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[108] The method of treatment according to [107], wherein the anti-GPR20 antibody lacks a lysine residue at the carboxyl terminus of the heavy chain.

[109] The method of treatment according to any one of [106] to [108], wherein the average number of units of the drug-linker conjugated per antibody molecule in the antibody-drug conjugate is in the range of from 7 to 8.

[110] The method of treatment according to [89], wherein the antibody in the antibody-drug conjugate is an anti-CDH6 antibody.

[111] The method of treatment according to [110], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

[112] The method of treatment according to [111], wherein the anti-CDH6 antibody lacks a lysine residue at the carboxyl terminus of the heavy chain.

[113] The method of treatment according to any one of [110] to [112], wherein the average number of units of the drug-linker conjugated per antibody molecule in the antibody-drug conjugate is in the range of from 7 to 8.

[114] The method of treatment according to any one of [88] to [113], wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[115] The method of treatment according to any one of [88] to [114], wherein the antibody-drug conjugate and the anti-SIRPα antibody are separately contained as active components in different formulations, and are administered simultaneously or at different times.

[116] The method of treatment according to any one of [88] to [115], wherein the method is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

[117] An antibody-drug conjugate for treating a disease through being administered in combination with an anti-SIRPα antibody, wherein a drug-linker represented by the following formula:

[Formula 5]

wherein A represents the connecting position to an antibody,
is conjugated to the antibody via a thioether bond in the antibody-drug conjugate.

[118] The antibody-drug conjugate according to [117], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[119] The antibody-drug conjugate according to [118], wherein the antibody in the antibody-drug conjugate is an anti-HER2 antibody
[120] The antibody-drug conjugate according to [119], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[121] The antibody-drug conjugate according to [119], wherein the anti-HER2 antibody is an antibody comprising a heavy chain consisting of an amino acid sequence represented by SEQ ID NO: 1 and a light chain consisting of an amino acid sequence represented by SEQ ID NO: 2
[122] The antibody-drug conjugate according to any one of [119] to [121], wherein the average number of units of the drug-linker conjugated per antibody molecule in the antibody-drug conjugate is in the range of from 7 to 8.
[123] The antibody-drug conjugate according to [118], wherein the antibody in the antibody-drug conjugate is an anti-HER3 antibody.
[124] The antibody-drug conjugate according to [123], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[125] The antibody-drug conjugate according to [124], wherein the anti-HER3 antibody lacks a lysine residue at the carboxyl terminus of the heavy chain.
[126] The antibody-drug conjugate according to any one of [123] to [125], wherein the average number of units of the drug-linker conjugated per antibody molecule in the antibody-drug conjugate is in the range of from 7 to 8.
[127] The antibody-drug conjugate according to [118], wherein the antibody in the antibody-drug conjugate is an anti-TROP2 antibody.
[128] The antibody-drug conjugate according to [127], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[129] The antibody-drug conjugate according to [128], wherein the anti-TROP2 antibody lacks a lysine residue at the carboxyl terminus of the heavy chain.
[130] The antibody-drug conjugate according to any one of [127] to [129], wherein the average number of units of the drug-linker conjugated per antibody molecule in the antibody-drug conjugate is in the range of from 3.5 to 4.5.
[131] The antibody-drug conjugate according to [130], wherein the antibody in the antibody-drug conjugate is an anti-B7-H3 antibody.
[132] The antibody-drug conjugate according to [131], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[133] The antibody-drug conjugate according to [132], wherein the anti-B7-H3 antibody lacks a lysine residue at the carboxyl terminus of the heavy chain.

[134] The antibody-drug conjugate according to any one of [131] to [133], wherein the average number of units of the drug-linker conjugated per antibody molecule in the antibody-drug conjugate is in the range of from 3.5 to 4.5.

[135] The antibody-drug conjugate according to [118], wherein the antibody in the antibody-drug conjugate is an anti-GPR20 antibody.

[136] The antibody-drug conjugate according to [135], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[137] The antibody-drug conjugate according to [136], wherein the anti-GPR20 antibody lacks a lysine residue at the carboxyl terminus of the heavy chain.

[138] The antibody-drug conjugate according to any one of [135] to [137], wherein the average number of units of the drug-linker conjugated per antibody molecule in the antibody-drug conjugate is in the range of from 7 to 8.

[139] The antibody-drug conjugate according to [118], wherein the antibody in the antibody-drug conjugate is an anti-CDH6 antibody.

[140] The antibody-drug conjugate according to [139], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

[141] The antibody-drug conjugate according to [140], wherein the anti-CDH6 antibody lacks a lysine residue at the carboxyl terminus of the heavy chain.

[142] The antibody-drug conjugate according to any one of [139] to [141], wherein the average number of units of the drug-linker conjugated per antibody molecule in the antibody-drug conjugate is in the range of from 7 to 8.

[143] The antibody-drug conjugate according to any one of [117] to [142], wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[144] The antibody-drug conjugate according to any one of [117] to [143], wherein the antibody-drug conjugate and the anti-SIRPα antibody are separately contained as active components in different formulations, and are administered simultaneously or at different times.

[145] The antibody-drug conjugate according to any one of [117] to [144], wherein the conjugate is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

[146] An antibody-drug conjugate for treating a disease through being administered in combination with an anti-SIRPα antibody, and represented by the following formula:

[Formula 6]

wherein a drug-linker is conjugated to an antibody via a thioether bond; and n represents the average number of units of the drug-linker conjugated per antibody molecule.

[147] The antibody-drug conjugate according to [146], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[148] The antibody-drug conjugate according to [147], wherein the antibody in the antibody-drug conjugate is an anti-HER2 antibody.

[149] The antibody-drug conjugate according to [148], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[150] The antibody-drug conjugate according to [148], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[151] The antibody-drug conjugate according to any one of [148] to [150], wherein the average number of units of the drug-linker conjugated per antibody molecule in the antibody-drug conjugate is in the range of from 7 to 8.

[152] The antibody-drug conjugate according to [147], wherein the antibody in the antibody-drug conjugate is an anti-HER3 antibody.

[153] The antibody-drug conjugate according to [152], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[154] The antibody-drug conjugate according to [153], wherein the anti-HER3 antibody lacks a lysine residue at the carboxyl terminus of the heavy chain.

[155] The antibody-drug conjugate according to any one of [152] to [154], wherein the average number of units of the drug-linker conjugated per antibody molecule in the antibody-drug conjugate is in the range of from 7 to 8.

[156] The antibody-drug conjugate according to [147], wherein the antibody in the antibody-drug conjugate is an anti-TROP2 antibody.

[157] The antibody-drug conjugate according to [156], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[158] The antibody-drug conjugate according to [157], wherein the anti-TROP2 antibody lacks a lysine residue at the carboxyl terminus of the heavy chain.

[159] The antibody-drug conjugate according to any one of [156] to [158], wherein the average number of units of the drug-linker conjugated per antibody molecule in the antibody-drug conjugate is in the range of from 3.5 to 4.5.

[160] The antibody-drug conjugate according to [147], wherein the antibody in the antibody-drug conjugate is an anti-B7-H3 antibody.

[161] The antibody-drug conjugate according to [160], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[162] The antibody-drug conjugate according to [161], wherein the anti-B7-H3 antibody lacks a lysine residue at the carboxyl terminus of the heavy chain.

[163] The antibody-drug conjugate according to any one of [160] to [162], wherein the average number of units of

the drug-linker conjugated per antibody molecule in the antibody-drug conjugate is in the range of from 3.5 to 4.5.
[164] The antibody-drug conjugate according to [147], wherein the antibody in the antibody-drug conjugate is an anti-GPR20 antibody.
[165] The antibody-drug conjugate according to [164], wherein the anti-GPR20 is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[166] The antibody-drug conjugate according to [165], wherein the anti-GPR20 antibody lacks a lysine residue at the carboxyl terminus of the heavy chain.
[167] The antibody-drug conjugate according to any one of [164] to [166], wherein the average number of units of the drug-linker conjugated per antibody molecule in the antibody-drug conjugate is in the range of from 7 to 8.
[168] The antibody-drug conjugate according to [147], wherein the antibody in the antibody-drug conjugate is an anti-CDH6 antibody.
[169] The antibody-drug conjugate according to [168], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[170] The antibody-drug conjugate according to [169], wherein the anti-CDH6 antibody lacks a lysine residue at the carboxyl terminus of the heavy chain.
[171] The antibody-drug conjugate according to any one of [168] to [170], wherein the average number of units of the drug-linker conjugated per antibody molecule in the antibody-drug conjugate is in the range of from 7 to 8.
[172] The antibody-drug conjugate according to any one of [146] to [171], wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[173] The antibody-drug conjugate according to any one of [146] to [172], wherein the antibody-drug conjugate and the anti-SIRPα antibody are separately contained as active components in different formulations, and are administered simultaneously or at different times.
[174] The antibody-drug conjugate according to any one of [146] to [173], wherein the conjugate is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[175] Use of an antibody-drug conjugate for the manufacture of a medicament for treating a disease through being administered in combination with an anti-SIRPα antibody, wherein a drug-linker represented by the following formula:

[Formula 7]

wherein A represents the connecting position to an antibody,
is conjugated to the antibody via a thioether bond in the antibody-drug conjugate.

[176] The use according to [175], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[177] The use according to [176], wherein the antibody in the antibody-drug conjugate is an anti-HER2 antibody.
[178] The use according to [177], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[179] The use according to [177], wherein the anti-HER2 antibody is an antibody comprising a heavy chain consisting of an amino acid sequence represented by SEQ ID NO: 1 and a light chain consisting of an amino acid sequence represented by SEQ ID NO: 2
[180] The use according to any one of [177] to [179], wherein the average number of units of the drug-linker conjugated per antibody molecule in the antibody-drug conjugate is in the range of from 7 to 8.
[181] The use according to [176], wherein the antibody in the antibody-drug conjugate is an anti-HER3 antibody.
[182] The use according to [181], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[183] The use according to [182], wherein the anti-HER3 antibody lacks a lysine residue at the carboxyl terminus of the heavy chain.
[184] The use according to any one of [181] to [183], wherein the average number of units of the drug-linker conjugated per antibody molecule in the antibody-drug conjugate is in the range of from 7 to 8.
[185] The use according to [176], wherein the antibody in the antibody-drug conjugate is an anti-TROP2 antibody.
[186] The use according to [185], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[187] The use according to [186], wherein the anti-TROP2 antibody lacks a lysine residue at the carboxyl terminus of the heavy chain.
[188] The use according to any one of [185] to [187], wherein the average number of units of the drug-linker conjugated per antibody molecule in the antibody-drug conjugate is in the range of from 3.5 to 4.5.
[189] The use according to [176], wherein the antibody in the antibody-drug conjugate is an anti-B7-H3 antibody.
[190] The use according to [189], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[191] The use according to [190], wherein the anti-B7-H3 antibody lacks a lysine residue at the carboxyl terminus of the heavy chain.
[192] The use according to any one of [189] to [191], wherein the average number of units of the drug-linker conjugated per antibody molecule in the antibody-drug conjugate is in the range of from 3.5 to 4.5.

[193] The use according to [176], wherein he antibody in the antibody-drug conjugate is an anti-GPR20 antibody.
[194] The use according to [193], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[195] The use according to [194], wherein the anti-GPR20 antibody lacks a lysine residue at the carboxyl terminus of the heavy chain.
[196] The use according to any one of [193] to [195], wherein the average number of units of the drug-linker conjugated per antibody molecule in the antibody-drug conjugate is in the range of from 7 to 8.
[197] The use according to [176], wherein the antibody in the antibody-drug conjugate is an anti-CDH6 antibody.
[198] The use according to [197], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[199] The use according to [198], wherein the anti-CDH6 antibody lacks a lysine residue at the carboxyl terminus of the heavy chain.
[200] The use according to any one of [197] to [199], wherein the average number of units of the drug-linker conjugated per antibody molecule in the antibody-drug conjugate is in the range of from 7 to 8.
[201] The use according to any one of [175] to [200], wherein the anti-SIRPα antibody is any one of the following (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[202] The use according to any one of [175] to [201], wherein the antibody-drug conjugate and the anti-SIRPα antibody are separately contained as active components in different formulations, and are administered simultaneously or at different times.
[203] The use according to any one of [175] to [202], wherein the use is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.
[204] Use of an antibody-drug conjugate for the manufacture of a medicament for treating a disease through being administered in combination with an anti-SIRPα antibody, and represented by the following formula:

[Formula 8]

wherein a drug-linker is conjugated to an antibody via a thioether bond; and n represents the average number of units of the drug-linker conjugated per antibody molecule.

[205] The use according to [204], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[206] The use according to [205], wherein the antibody in the antibody-drug conjugate is an anti-HER2 antibody.

[207] The use according to [206], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[208] The use according to [206], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[209] The use according to any one of [206] to [208], wherein the average number of units of the drug-linker conjugated per antibody molecule in the antibody-drug conjugate is in the range of from 7 to 8.

[210] The use according to [205], wherein the antibody in the antibody-drug conjugate is an anti-HER3 antibody.

[211] The use according to [210], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[212] The use according to [211], wherein the anti-HER3 antibody lacks a lysine residue at the carboxyl terminus of the heavy chain.

[213] The use according to any one of [210] to [212], wherein the average number of units of the drug-linker conjugated per antibody molecule in the antibody-drug conjugate is in the range of from 7 to 8.

[214] The use according to [205], wherein the antibody in the antibody-drug conjugate is an anti-TROP2 antibody.

[215] The use according to [214], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[216] The use according to [215], wherein the anti-TROP2 antibody lacks a lysine residue at the carboxyl terminus of the heavy chain.

[217] The use according to any one of [214] to [216], wherein the average number of units of the drug-linker conjugated per antibody molecule in the antibody-drug conjugate is in the range of from 3.5 to 4.5.

[218] The use according to [205], wherein the antibody in the antibody-drug conjugate is an anti-B7-H3 antibody.

[219] The use according to [218], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[220] The use according to [219], wherein the anti-B7-H3 antibody lacks a lysine residue at the carboxyl terminus of the heavy chain.

[221] The use according to any one of [218] to [220], wherein the average number of units of the drug-linker conjugated per antibody molecule in the antibody-drug conjugate is in the range of from 3.5 to 4.5.

[222] The use according to [205], wherein the antibody in the antibody-drug conjugate is an anti-GPR20 antibody.

[223] The use according to [222], wherein the anti-GPR20 antibody is an antibody comprising a heavy chain consisting of an amino acid sequence consisting of amino acid residues 20 to 472 of SEQ ID NO: 9 and a light chain consisting

of an amino acid sequence consisting of amino acid residues 21 to 234 of SEQ ID NO: 10.
[224] The use according to [223], wherein the anti-GPR20 antibody lacks a lysine residue at the carboxyl terminus of the heavy chain.
[225] The use according to any one of [222] to [224], wherein the average number of units of the drug-linker conjugated per antibody molecule in the antibody-drug conjugate is in the range of from 7 to 8.
[226] The use according to [205], wherein the antibody in the antibody-drug conjugate is an anti-CDH6 antibody.
[227] The use according to [226], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[228] The use according to [227], wherein the anti-CDH6 antibody lacks a lysine residue at the carboxyl terminus of the heavy chain.
[229] The use according to any one of [226] to [228], wherein the average number of units of the drug-linker conjugated per antibody molecule in the antibody-drug conjugate is in the range of from 7 to 8.
[230] The use according to any one of [204] to [229], wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

[231] The use according to any one of [204] to [230], wherein the antibody-drug conjugate and the anti-SIRPα antibody are separately contained as active components in different formulations, and are administered simultaneously or at different times.
[232] The use according to any one of [204] to [231], wherein the use is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, and uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

[Advantageous Effects of Invention]

The present invention provides a pharmaceutical composition, wherein a specific antibody-drug conjugate and an anti-SIRPα antibody are administered in combination, and/or a method of treatment comprising administering a specific antibody-drug conjugate and an anti-SIRPα antibody in combination to a subject.

[Brief Description of Drawings]

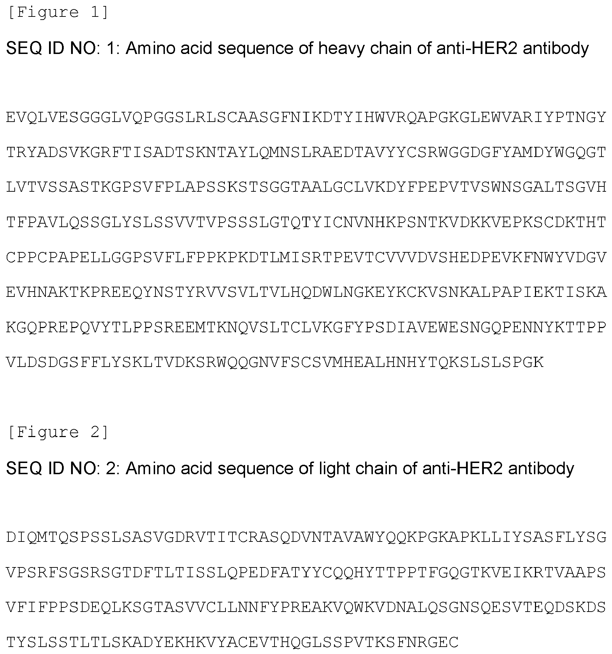

[Figure 1] Figure 1 is a diagram showing the amino acid sequence of a heavy chain of an anti-HER2 antibody (SEQ ID NO: 1).
[Figure 2] Figure 2 is a diagram showing the amino acid sequence of a light chain of an anti-HER2 antibody (SEQ ID NO: 2).

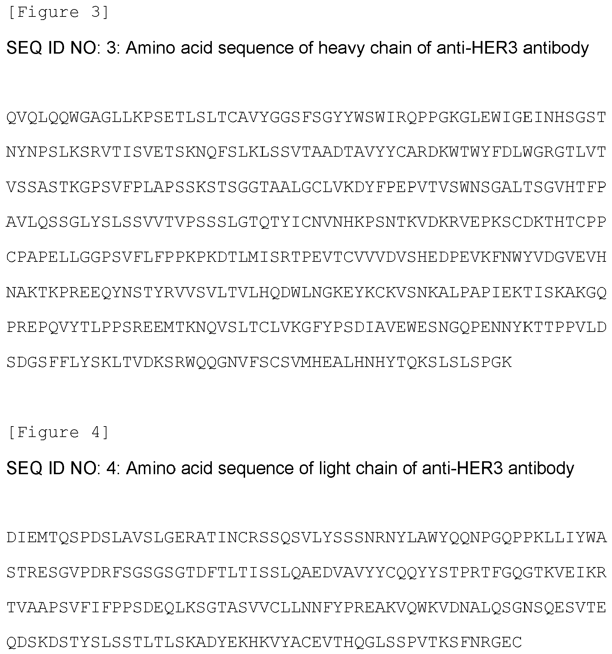

[Figure 3] Figure 3 is a diagram showing the amino acid sequence of a heavy chain of an anti-HER3 antibody (SEQ ID NO: 3).
[Figure 4] Figure 4 is a diagram showing the amino acid sequence of a light chain of an anti-HER3 antibody (SEQ

ID NO: 4).

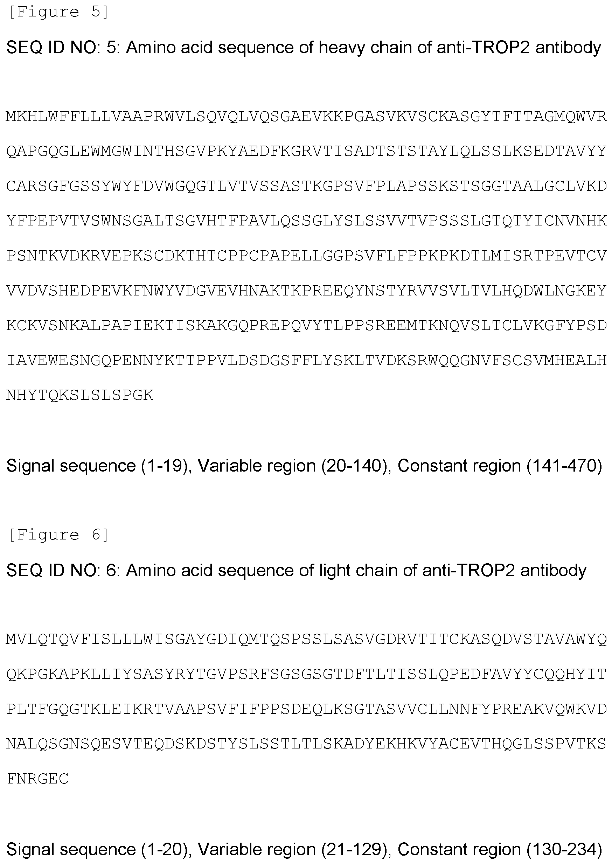

[Figure 5] Figure 5 is a diagram showing the amino acid sequence of a heavy chain of an anti-TROP2 antibody (SEQ ID NO: 5).

[Figure 6] Figure 6 is a diagram showing the amino acid sequence of a light chain of an anti-TROP2 antibody (SEQ ID NO: 6).

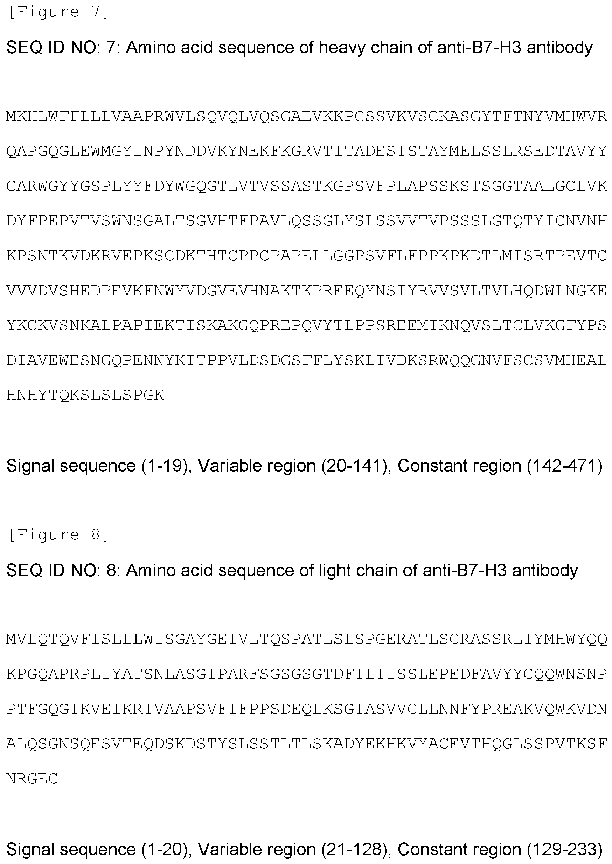

[Figure 7] Figure 7 is a diagram showing the amino acid sequence of a heavy chain of an anti-B7-H3 antibody (SEQ ID NO: 7).

[Figure 8] Figure 8 is a diagram showing the amino acid sequence of a light chain of an anti-B7-H3 antibody (SEQ ID NO: 8).

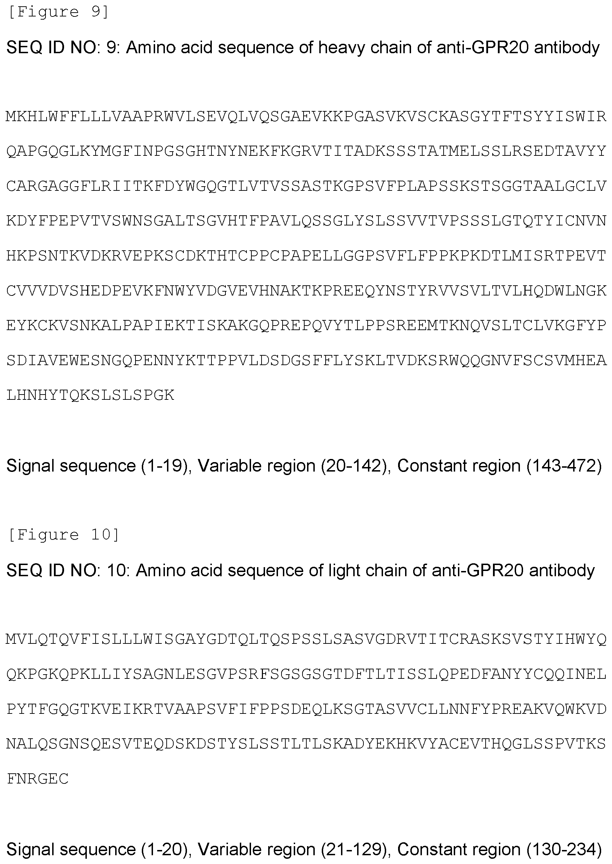

[Figure 9] Figure 9 is a diagram showing the amino acid sequence of a heavy chain of an anti-GPR20 antibody (SEQ ID NO: 9).

[Figure 10] Figure 10 is a diagram showing the amino acid sequence of a light chain of an anti-GPR20 antibody (SEQ ID NO: 10).

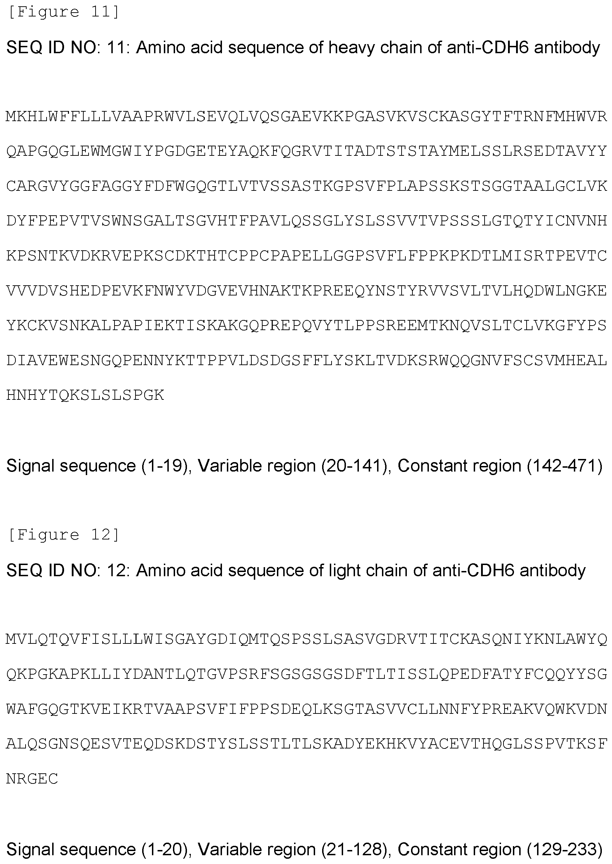

[Figure 11] Figure 11 is a diagram showing the amino acid sequence of a heavy chain of an anti-CDH6 antibody (SEQ ID NO: 11).

[Figure 12] Figure 12 is a diagram showing the amino acid sequence of a light chain of an anti-CDH6 antibody (SEQ ID NO: 12).

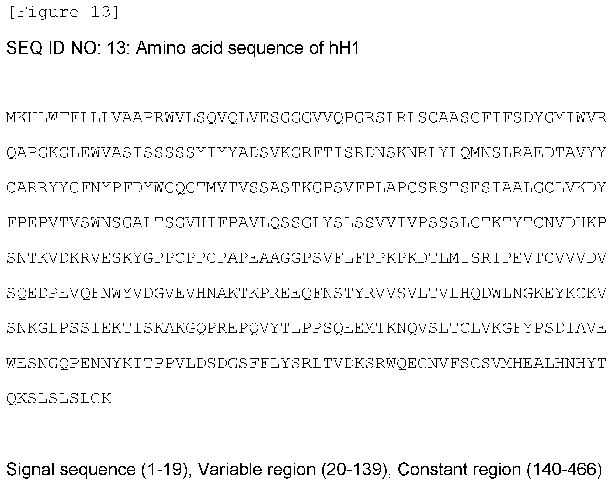

[Figure 13] Figure 13 is a diagram showing the amino acid sequence of the hH1 heavy chain of humanized anti-SIRPα antibody D13 (SEQ ID NO: 13).

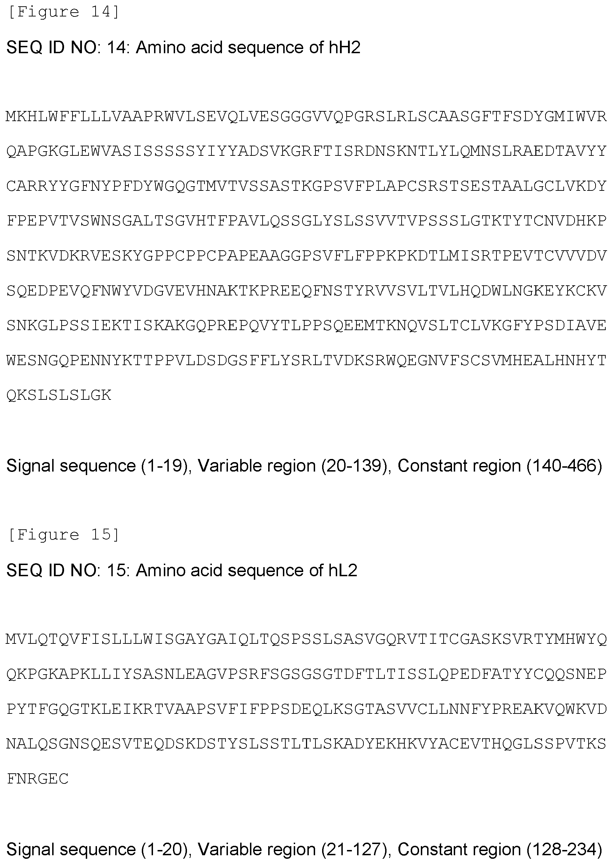

[Figure 14] Figure 14 is a diagram showing the amino acid sequence of the hH2 heavy chain of humanized anti-SIRPα antibody D13 (SEQ ID NO: 14).

[Figure 15] Figure 15 is a diagram showing the amino acid sequence of the hL2 light chain of humanized anti-SIRPα antibody D13 (SEQ ID NO: 15).

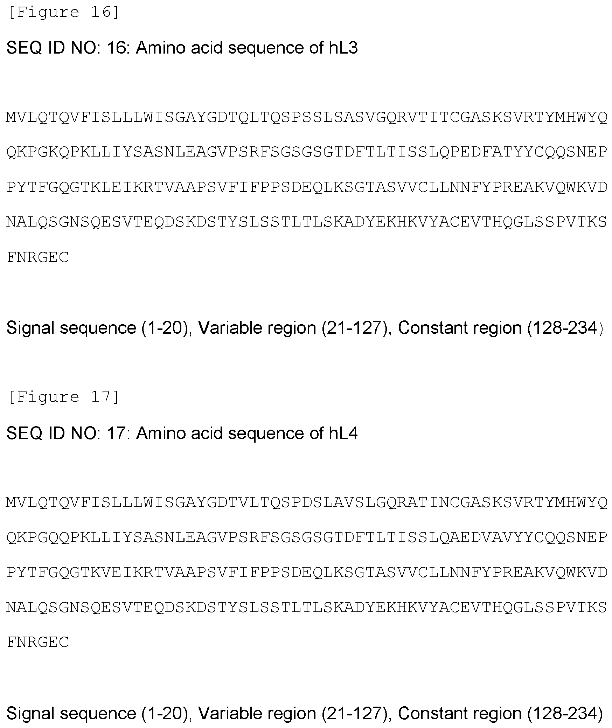

[Figure 16] Figure 16 is a diagram showing the amino acid sequence of the hL3 light chain of humanized anti-SIRPα antibody D13 (SEQ ID NO: 16).

[Figure 17] Figure 17 is a diagram showing the amino acid sequence of the hL4 light chain of humanized anti-SIRPα antibody D13 (SEQ ID NO: 17).

[Figure 18] Figure 18 is a diagram showing CDR sequences of anti-SIRPα antibody cD13 (SEQ ID NO: 18 to 23).

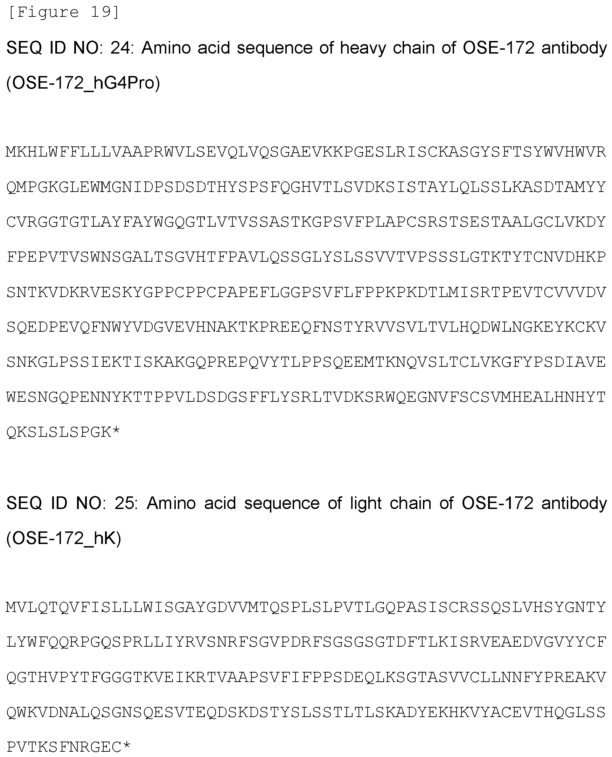

[Figure 19] Figure 19 is a diagram showing the amino acid sequence of an OSE-172 antibody heavy chain (OSE-172_hG4Pro) (SEQ ID NO: 24) and an amino acid sequence of a light chain (OSE-172_hK) thereof (SEQ ID NO: 25).

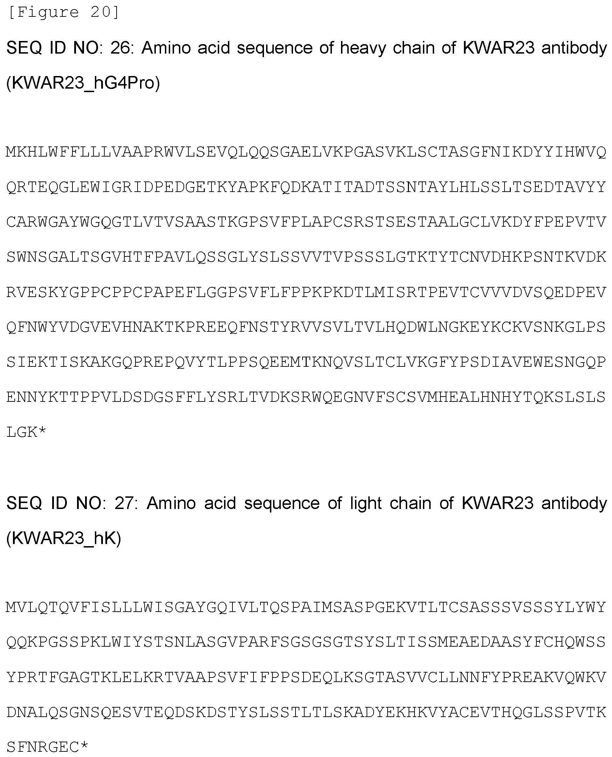

[Figure 20] Figure 20 is a diagram showing the amino acid sequence of a KWAR23 antibody heavy chain (KWAR23_hG4Pro) (SEQ ID NO: 26) and the amino acid sequence of a light chain (KWAR23_hK) thereof (SEQ ID NO: 27).

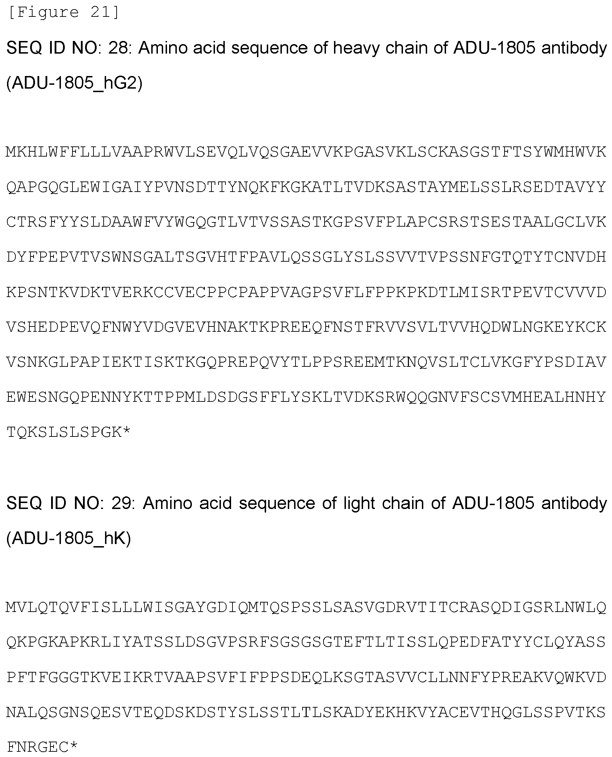

[Figure 21] Figure 21 is a diagram showing the amino acid sequence of an ADU-1805 antibody heavy chain (ADU-1805_hG2) (SEQ ID NO: 28) and the amino acid sequence of a light chain (ADU-1805_hK) thereof (SEQ ID NO: 29).

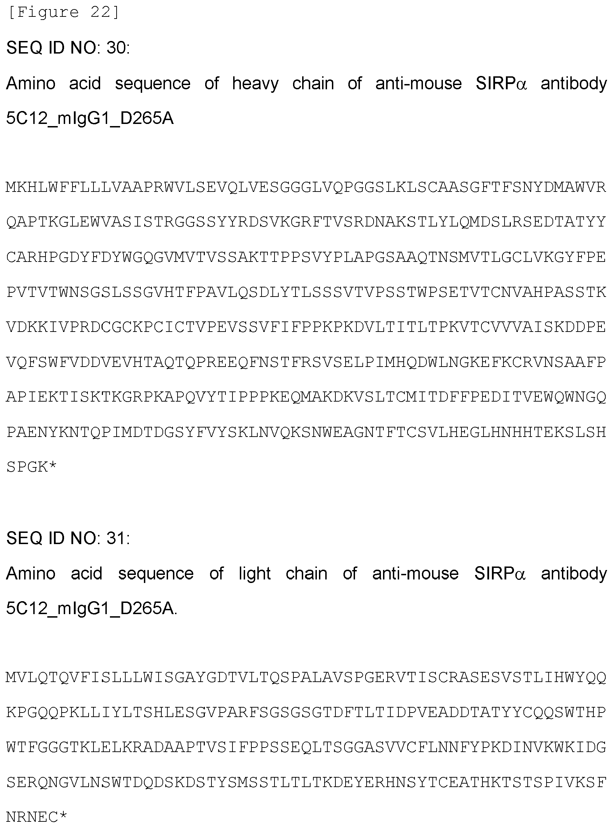

[Figure 22] Figure 22 is a diagram showing the amino acid sequence of a heavy chain of a 5C12 anti-mouse SIRPα antibody (SEQ ID NO: 30) and the amino acid sequence of a light chain thereof (SEQ ID NO: 31).

[Figure 23]

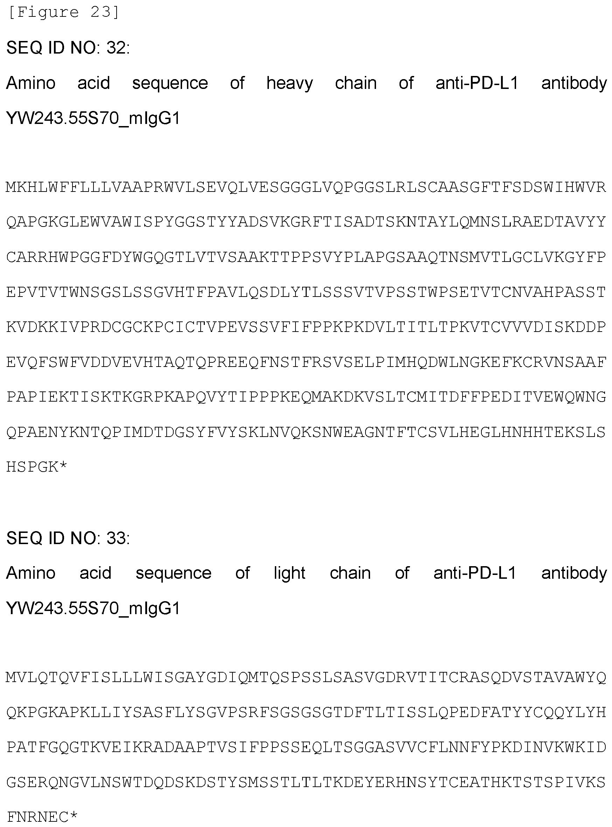

Figure 23 is a diagram showing the amino acid sequence of YW243.55S70 anti-mouse human anti-PD-L1 antibody heavy chain (SEQ ID NO: 32) and a light chain thereof (SEQ ID NO: 33).

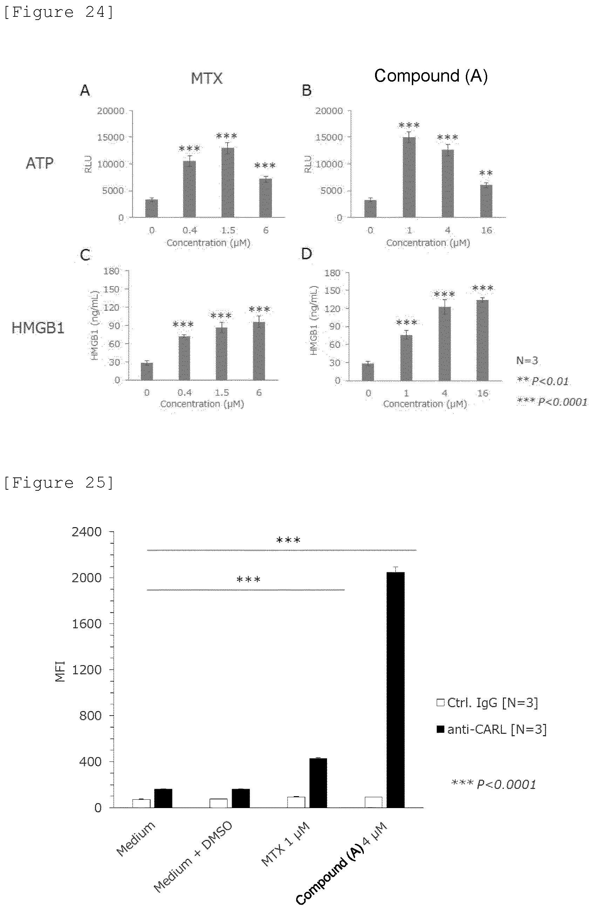

[Figure 24] Figure 24 includes graphs showing release of ATP and HMGB1 in in vitro ICD induction by compound (A).

[Figure 25] Figure 25 is a graph showing expression of Calreticulin (CRT) on the cell surface in in vitro ICD induction by compound (A).

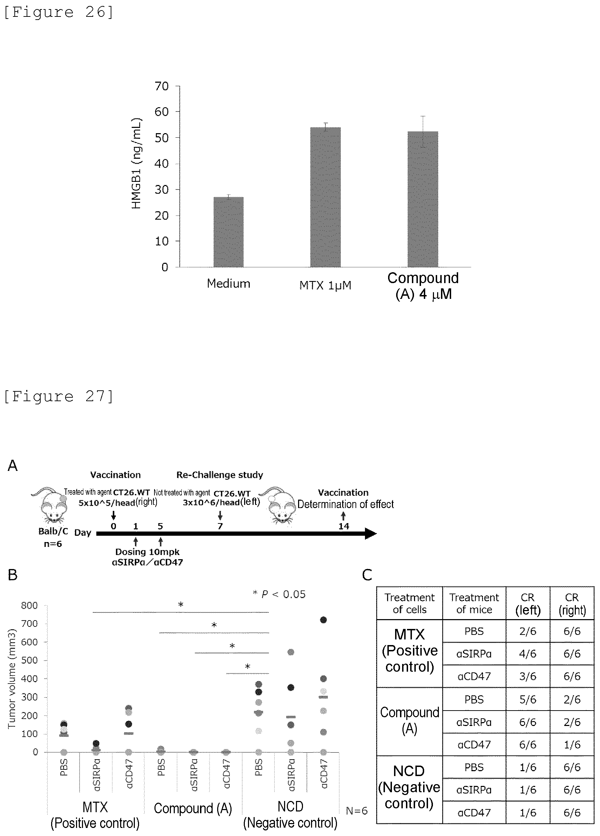

[Figure 26] Figure 26 is a graph showing release of HMGB1 in in vitro ICD induction by compound (A). Mouse colorectal cancer cells in which ICD has been induced are inoculated into a mouse in a study shown in Figure 27.

[Figure 27] Figure 27 includes views showing formation of immunological memory (vaccination effect) to a tumor by in vivo ICD induction by the compound (A).

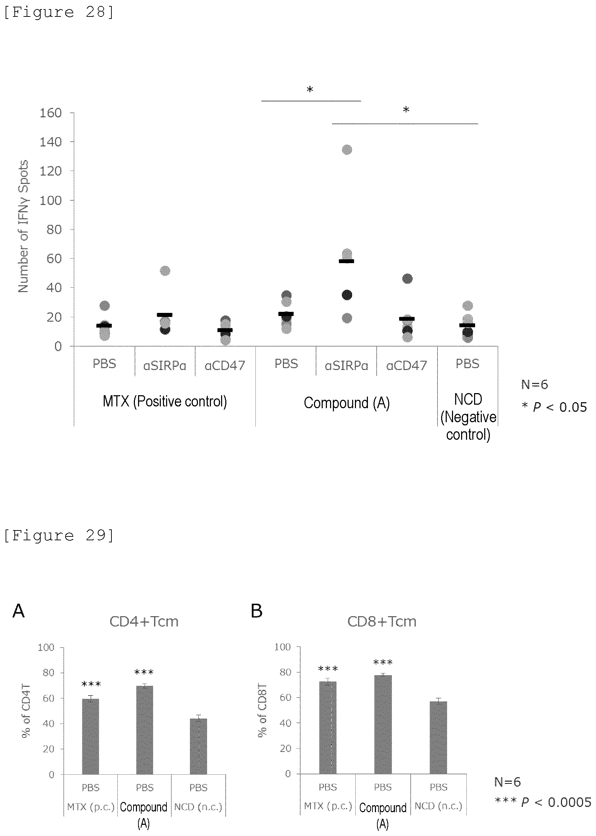

[Figure 28] Figure 28 is a graph showing the number of IFNγ-producing spleen cells in the spleen excised out from a mouse into which mouse colorectal cancer cells have been inoculated in the study shown in Figure 27.

[Figure 29] Figure 29 includes graphs showing the analysis results of T cell population in the spleen excised out from the mouse into which mouse colorectal cancer cells have been inoculated and administered with PBS in the study shown in Figure 27.

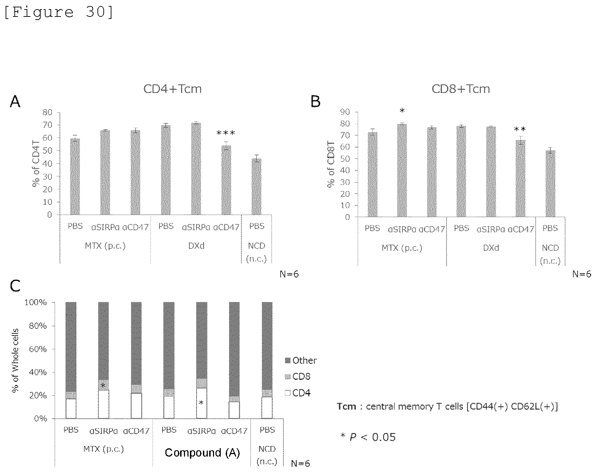

[Figure 30] Figure 30 includes graphs showing the analysis results of T cell population in the spleen excised out from the mouse into which mouse colorectal cancer cells have been inoculated in the study shown in Figure 27.

[Figure 31]

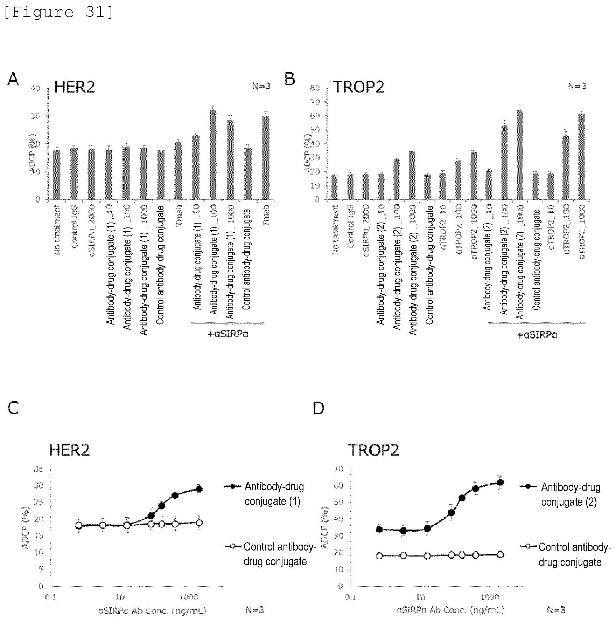

(Figure 31A) Figure 31A is a graph showing ADCP activity of an anti-SIRPα antibody and/or an antibody-drug conjugate (1) to human gastric cancer cells.

(Figure 31B) Figure 31B is a graph showing ADCP activity of an anti-SIRPα antibody and/or an antibody-drug

conjugate (2) in human gastric cancer cells.

(Figure 31C) Figure 31C is a graph showing enhancement of ADCP activity of an anti-SIRPα antibody and an antibody-drug conjugate (1) in human gastric cancer cells in an anti-SIRPα antibody concentration dependent manner.

(Figure 31D) Figure 31D is a graph showing enhancement of ADCP activity of an anti-SIRPα antibody and an antibody-drug conjugate (2) in human gastric cancer cells in an anti-SIRPα antibody concentration dependent manner.

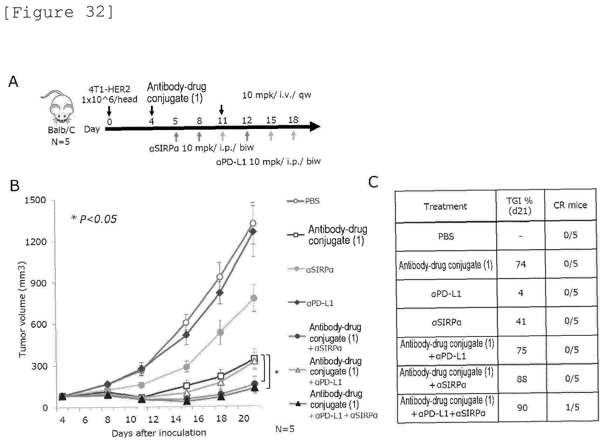

[Figure 32] Figure 32 includes views showing an antitumor effect by an anti-SIRPα antibody, an anti-PD-L1 antibody and/or an antibody-drug conjugate (1) in a mouse into which HER2-expressing mouse breast cancer cells have been inoculated.

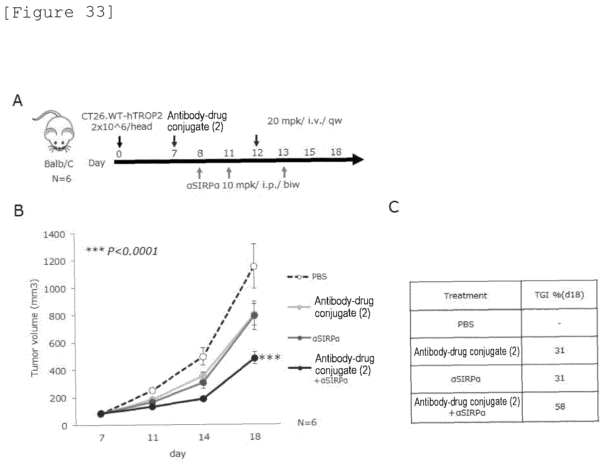

[Figure 33] Figure 33 includes views showing an antitumor effect by an anti-SIRPα antibody and/or an antibody-drug conjugate (2) in a mouse into which TROP2 expressing mouse colorectal cancer cells have been inoculated.

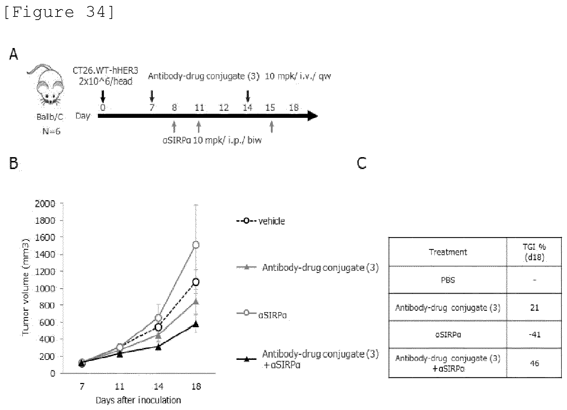

[Figure 34] Figure 34 includes views showing an antitumor effect by an anti-SIRPα antibody and/or an antibody-drug conjugate (3) in a mouse into which HER3 expressing mouse colorectal cancer cells have been inoculated.

[Description of Embodiments]

Hereinafter, preferred modes for carrying out the present invention are described. The embodiments described below are given merely for illustrating one example of a typical embodiment of the present invention and are not intended to limit the scope of the present invention.

1. Antibody-drug conjugate

The antibody-drug conjugate used in the present invention is an antibody-drug conjugate in which a drug-linker represented by the following formula:

[Formula 9]

wherein A represents the connecting position to an antibody,
is conjugated to the antibody via a thioether bond.

In the present invention, the partial structure consisting of a linker and a drug in the antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4' :6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4' :6,7]indolizino[1,2-b]quinolin-10,13(9H,15H)-dione)), which is a topoisomerase I inhibitor, as a component.

Exatecan is a camptothecin derivative having an antitumor effect, represented by the following formula:

[Formula 10]

The antibody-drug conjugate used in the present invention can also be represented by the following formula:

[Formula 11]

wherein, the drug-linker is conjugated to an antibody via a thioether bond. The meaning of n is the same as that of what is called the average number of conjugated drug molecules (DAR; Drug-to-Antibody Ratio), and indicates the average number of units of the drug-linker conjugated per antibody molecule.

After migrating into cancer cells, the antibody-drug conjugate used in the present invention is cleaved at the linker portion to release the compound represented by the following formula
(hereinafter referred to as compound (A)):

[Formula 12]

The aforementioned compound is inferred to be the original source of the antitumor activity of the antibody-drug conjugate used in the present invention, and has been confirmed to have a topoisomerase I inhibitory effect (Ogitani Y. et al., Clinical Cancer Research, 2016, Oct 15;22 (20) :5097-5108, Epub 2016 Mar 29.)

Topoisomerase I is an enzyme that cleaves single strands of DNA and rejoins cleaved fragments, thereby transforming the higher-order structure of DNA for participation in DNA synthesis. Thus, a drug having a topoisomerase

I inhibitory effect inhibits DNA synthesis to terminate cell division during the S phase (DNA synthesis phase) of the cell cycle and induces apoptosis (cell death) to suppress cancer-cell proliferation.

The antibody-drug conjugate used in the present invention is also known to have a bystander effect (Ogitani Y. et al., Cancer Science (2016) 107, 1039-1046) .

The bystander effect is exerted through a process such that the antibody-drug conjugate used in the present invention is internalized in cancer cells expressing the target, and the aforementioned compound is released and then exerts an antitumor effect also on cancer cells which are present therearound and not expressing the target.

The bystander effect is also exerted as an excellent antitumor effect when the antibody-drug conjugate according to the present invention is used in combination with an anti-SIRPα antibody.

2. Antibody in the antibody-drug conjugate

The antibody in the antibody-drug conjugate used in the present invention may be derived from any species and is preferably an antibody derived from a human, a rat, a mouse, or a rabbit. In cases when the antibody is derived from species other than human species, it is preferably chimerized or humanized using a well-known technique. The antibody of the present invention may be a polyclonal antibody or a monoclonal antibody and is preferably a monoclonal antibody.

The antibody in the antibody-drug conjugate used in the present invention is an antibody preferably having the characteristic of being able to target cancer cells, and is preferably an antibody possessing, for example, the property of being able to recognize a cancer cell, the property of binding to a cancer cell, the property of being internalized in a cancer cell, and/or cytocidal activity against cancer cells.

The binding activity of the antibody against cancer cells can be confirmed using flow cytometry. The internalization of the antibody into tumor cells can be confirmed using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added at varying concentrations into the culture system to determine inhibitory activity against focus formation, colony formation, and spheroid growth. The antitumor activity can be confirmed in vivo, for example, by administering the antibody to a nude mouse with an inoculated cancer cell line highly expressing the target protein, and determining changes in the cancer cells.

Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of being internalized to migrate into cancer cells.

The antibody in the antibody-drug conjugate used in the present invention can be obtained by a procedure known in the art. For example, the antibody of the present invention can be obtained using a method usually carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen are fused with myeloma cells according to a method known in the art (for example, Kohler and Milstein, Nature (1975) 256, p.495-497; Kennet, R. ed., Monoclonal Antibodies, p.365-367, Plenum Press, N.Y. (1980)), to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The antibody in the antibody-drug conjugate used in the present invention is preferably a recombinant antibody obtained by artificial modification for the purpose of decreasing heterologous antigenicity to humans such as a chimeric

antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)).

As the humanized antibody, an antibody obtained by integrating only the complementarity determining region (CDR) of a heterologous antibody into a human-derived antibody (Nature (1986) 321, pp. 522-525), an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002) 43 (7), p.2301-2308; Carmen, S. et. al., Briefings in Functional Genomics and Proteomics (2002), 1 (2), p.189-203; Siriwardena, D. et. al., Ophthalmology (2002) 109 (3), p.427-431, etc.) can be exemplified.

In the antibody in the antibody-drug conjugate used in present invention, modified variants of the antibody are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, International Publication No. WO 99/54342, International Publication No. WO 00/61739, International Publication No. WO 02/31140, International Publication No. WO 2007/133855, and International Publication No. WO 2013/120066, etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (complement activation, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, deletion variants having an amidated residue (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, an antibody in which one amino acid residue at the carboxyl terminus has been deleted in both of the two heavy chains in the antibody according to the present invention can be preferably exemplified.

As isotypes of the antibody according to the present invention, for example, IgG (IgG1, IgG2, IgG3, IgG4) can be exemplified, and IgG1, IgG2 or IgG4 can be exemplified preferably.

Examples of antibodies in the antibody-drug conjugates used in the present invention can include, but are not particularly limited to, an anti-HER2 antibody, an anti-HER3 antibody, an anti-TROP2 antibody, an anti-B7-H3 antibody, an anti-CD3 antibody, an anti-CD30 antibody, an anti-CD33 antibody, an anti-CD37 antibody, an anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, an anti-CD20 antibody, an anti-CD22 antibody, an anti-CD70 antibody, an anti-PSMA antibody, an anti-CEA antibody, an anti-Mesothelin antibody, an anti-A33 antibody, an anti-CanAg antibody, an anti-Cripto antibody, an anti-G250 antibody, an anti-MUC1 antibody, an anti-GPNMB antibody, an anti-Integrin antibody, an anti-Tenascin-C antibody, an anti-SLC44A4 antibody, an anti-GPR20 antibody, and an anti-CDH6 antibody, and an anti-HER2 antibody, an anti-HER3 antibody, an anti-TROP2 antibody, an anti-B7-H3 antibody, an anti-GPR20 antibody, and an anti-CDH6 antibody can be preferably exemplified.
In the present invention, the term "anti-HER2 antibody" refers to an antibody which binds specifically to HER2 (Human Epidermal Growth Factor Receptor Type 2; ErbB-2), and preferably has an activity of internalization in HER2-expressing cells by binding to HER2.
Examples of the anti-HER2 antibody include trastuzumab (U.S. Patent No. 5821337) and pertuzumab (International Publication No. WO 01/00245), and trastuzumab can be preferably exemplified.
In the present invention, the term "anti-HER3 antibody" refers to an antibody which binds specifically to HER3 (Human Epidermal Growth Factor Receptor Type 3; ErbB-3), and preferably has an activity internalization in HER3-expressing cells by binding to HER3.
Examples of the anti-HER3 antibody include patritumab (U3-1287), U1-59 (International Publication No. WO 2007/077028), MM-121 (seribantumab), an anti-ERBB3 antibody described in International Publication No. WO 2008/100624, RG-7116 (lumretuzumab), and LJM-716 (elgemtumab), and patritumab and U1-59 can be preferably exemplified.
In the present invention, the term "anti-TROP2 antibody" refers to an antibody which binds specifically to TROP2 (TACSTD2: Tumor-associated calcium signal transducer 2; EGP-1), and preferably has an activity of internalization in TROP2-expressing cells by binding to TROP2.
Examples of the anti-TROP2 antibody include hTINA1-H1L1 (International Publication No. WO 2015/098099).
In the present invention, the term "anti-B7-H3 antibody" refers to an antibody which binds specifically to B7-H3 (B cell antigen #7 homolog 3; PD-L3; CD276), and preferably has an activity of internalization in B7-H3-expressing cells by binding to B7-H3.
Examples of the anti-B7-H3 antibody include M30-H1-L4 (International Publication No. WO 2014/057687).
In the present invention, the term "anti-GPR20 antibody" refers to an antibody which binds specifically to GPR20 (G Protein-coupled receptor 20), and preferably has an activity of internalization in GPR20-expressing cells by binding to GPR20.
Examples of the anti-GPR20 antibody include h046-H4e/L7 (International Publication No. WO 2018/135501).
In the present invention, the term "anti-CDH6 antibody" refers to an antibody which specifically binds to CDH6 (Cadherin-6), and preferably has an activity of internalizing in CDH6-expressing cells by binding to CDH6.
Examples of the anti-CDH6 antibody include H01L02 (International Publication No. WO 2018/212136).

3. Production of the antibody-drug conjugate

A drug-linker intermediate for use in the production of the antibody-drug conjugate according to the present invention is represented by the following formula.

[Formula 13]

The drug-linker intermediate can be expressed as the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2014/057687, International Publication No. WO 2015/098099, International Publication No. WO 2015/115091, International Publication No. WO 2015/155998, and International Publication No. WO 2019/044947.

The antibody-drug conjugate used in the present invention can be produced by reacting the above-described drug-linker intermediate and an antibody having a thiol group (alternatively referred to as a sulfhydryl group).

The antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

Further, by using 2 to 20 molar equivalents of the drug-linker intermediate per the antibody having a sulfhydryl group, an antibody-drug conjugate in which 2 to 8 drug molecules are conjugated per antibody molecule can be produced.

The average number of conjugated drug molecules per antibody molecule of the antibody-drug conjugate produced can be determined, for example, by a method of calculation based on measurement of UV absorbance for the antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the antibody and the drug-linker intermediate and calculation of the average number of conjugated drug molecules per antibody molecule of the antibody-drug conjugate can be performed with reference to descriptions in International Publication No. WO 2014/057687, International Publication No. WO 2015/098099, International Publication No. WO 2015/115091, International Publication No. WO 2015/155998, International Publication No. WO 2018/135501, and International Publication No. WO 2018/212136, and so on.

In the present invention, "anti-HER2 antibody-drug conjugate" represents an antibody-drug conjugate in which the antibody in the antibody-drug conjugate according to the invention is an anti-HER2 antibody.

The anti-HER2 antibody is preferably an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2, and more preferably an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2, and even more preferably an antibody comprising a heavy chain consisting of an amino acid sequence consisting of amino acid residues 1 to 449 of SEQ ID NO: 1 and

a light chain consisting of an amino acid sequence consisting of amino acid residues 1 to 214 of SEQ ID NO: 2; or an antibody comprising a heavy chain consisting of an amino acid sequence represented by SEQ ID NO: 1 and a light chain consisting of an amino acid sequence represented by SEQ ID NO: 2.

The average number of units of the drug-linker conjugated per antibody molecule in the anti-HER2 antibody-drug conjugate is preferably 2 to 8, more preferably 3 to 8, even more preferably 7 to 8, even more preferably 7.5 to 8, and even more preferably about 8.

The anti-HER2 antibody-drug conjugate can be produced with reference to descriptions in International Publication No. WO 2015/115091 and so on.

In the present invention, the term "anti-HER3 antibody-drug conjugate" refers to an antibody-drug conjugate such that the antibody in the antibody-drug conjugate according to the invention is an anti-HER3 antibody.

The anti-HER3 antibody is preferably an antibody comprising a heavy chain consisting of CDRH1 consisting of an amino acid sequence consisting of amino acid residues 26 to 35 of SEQ ID NO: 3, CDRH2 consisting of an amino acid sequence consisting of amino acid residues 50 to 65 of SEQ ID NO: 3, and CDRH3 consisting of an amino acid sequence consisting of amino acid residues 98 to 106 of SEQ ID NO: 3, and a light chain consisting of CDRL1 consisting of an amino acid sequence consisting of amino acid residues 24 to 39 of SEQ ID NO: 4, CDRL2 consisting of an amino acid sequence consisting of amino acid residues 56 to 62 of SEQ ID NO: 4, and CDRL3 consisting of an amino acid sequence consisting of amino acid residues 95 to 103 of SEQ ID NO: 4;

more preferably, an antibody comprising a heavy chain which comprises a heavy chain variable region consisting of an amino acid sequence consisting of amino acid residues 1 to 117 of SEQ ID NO: 3 and a light chain which comprises a light chain variable region consisting of an amino acid sequence consisting of amino acid residues 1 to 113 of SEQ ID NO: 4; and

even more preferably, an antibody comprising a heavy chain consisting of an amino acid sequence represented by SEQ ID NO: 3 and a light chain consisting of an amino acid sequence represented by SEQ ID NO: 4, or a variant of the antibody in which a lysine residue at the carboxyl terminus of the heavy chain is deleted.

The average number of units of the drug-linker conjugated per antibody molecule in the anti-HER3 antibody-drug conjugate is preferably 2 to 8, more preferably 3 to 8, even more preferably 7 to 8, even more preferably 7.5 to 8, and even more preferably about 8.

The anti-HER3 antibody-drug conjugate can be produced with reference to descriptions in International Publication No. WO 2015/155998 and so on.

In the present invention, the term "anti-TROP2 antibody-drug conjugate" refers to an antibody-drug conjugate such that the antibody in the antibody-drug conjugate according to the invention is an anti-TROP2 antibody.

The anti-TROP2 antibody is preferably an antibody comprising a heavy chain consisting of CDRH1 consisting of an amino acid sequence consisting of amino acid residues 50 to 54 of SEQ ID NO: 5, CDRH2 consisting of an amino acid sequence consisting of amino acid residues 69 to 85 of SEQ ID NO: 5, and CDRH3 consisting of an amino acid sequence consisting of amino acid residues 118 to 129 of SEQ ID NO: 5, and a light chain consisting of CDRL1 consisting of an amino acid sequence consisting of amino acid residues 44 to 54 of SEQ ID NO: 6, CDRL2 consisting of an amino acid sequence consisting of amino acid residues 70 to 76 of SEQ ID NO: 6, and CDRL3 consisting of an amino acid sequence consisting of amino acid residues 109 to 117 of SEQ ID NO: 6;

more preferably, an antibody comprising a heavy chain which comprises a heavy chain variable region consisting of an amino acid sequence consisting of amino acid residues 20 to 140 of SEQ ID NO: 5 and a light chain which comprises a light chain variable region consisting of an amino acid sequence consisting of amino acid residues 21 to 129 of SEQ ID NO: 6; and

even more preferably, an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted.

The average number of units of the drug-linker conjugated per antibody molecule in the anti-TROP2 antibody-drug conjugate is preferably 2 to 8, more preferably 3 to 5, even more preferably 3.5 to 4.5, and even more preferably about 4.

The anti-TROP2 antibody-drug conjugate can be produced with reference to descriptions in International Publication No. WO 2015/098099 and so on.

In the present invention, the term "anti-B7-H3 antibody-drug conjugate" refers to an antibody-drug conjugate such that the antibody in the antibody-drug conjugate according to the invention is an anti-B7-H3 antibody.

The anti-B7-H3 antibody is preferably an antibody comprising a heavy chain consisting of CDRH1 consisting

of an amino acid sequence consisting of amino acid residues 50 to 54 of SEQ ID NO: 7, CDRH2 consisting of an amino acid sequence consisting of amino acid residues 69 to 85 of SEQ ID NO: 7, and CDRH3 consisting of an amino acid sequence consisting of amino acid residues 118 to 130 of SEQ ID NO: 7, and a light chain consisting of CDRL1 consisting of an amino acid sequence consisting of amino acid residues 44 to 53 of SEQ ID NO: 8, CDRL2 consisting of an amino acid sequence consisting of amino acid residues 69 to 75 of SEQ ID NO: 8, and CDRL3 consisting of an amino acid sequence consisting of amino acid residues 108 to 116 of SEQ ID NO: 8;

more preferably, an antibody comprising a heavy chain which comprises a heavy chain variable region consisting of an amino acid sequence consisting of amino acid residues 20 to 141 of SEQ ID NO: 7 and a light chain which comprises a light chain variable region consisting of an amino acid sequence consisting of amino acid residues 21 to 128 of SEQ ID NO: 8; and

even more preferably, an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted.

The average number of units of the drug-linker conjugated per antibody molecule in the anti-B7-H3 antibody-drug conjugate is preferably 2 to 8, more preferably 3 to 5, even more preferably 3.5 to 4.5, and even more preferably about 4.

The anti-B7-H3 antibody-drug conjugate used in the present invention can be produced with reference to descriptions in International Publication No. WO 2014/057687 and so on.

In the present invention, the term "anti-GPR20 antibody-drug conjugate" refers to an antibody-drug conjugate such that the antibody in the antibody-drug conjugate according to the invention is an anti-GPR20 antibody.

The anti-GPR20 antibody is preferably an antibody comprising a heavy chain consisting of CDRH1 consisting of an amino acid sequence consisting of amino acid residues 45 to 54 of SEQ ID NO: 9, CDRH2 consisting of an amino acid sequence consisting of amino acid residues 69 to 78 of SEQ ID NO: 9, and CDRH3 consisting of an amino acid sequence consisting of amino acid residues 118 to 131 of SEQ ID NO: 9, and a light chain consisting of CDRL1 consisting of an amino acid sequence consisting of amino acid residues 44 to 54 of SEQ ID NO: 10, CDRL2 consisting of an amino acid sequence consisting of amino acid residues 70 to 76 of SEQ ID NO: 10, and CDRL3 consisting of an amino acid sequence consisting of amino acid residues 109 to 117 of SEQ ID NO: 10;

more preferably, an antibody comprising a heavy chain which comprises a heavy chain variable region consisting of an amino acid sequence consisting of amino acid residues 20 to 142 of SEQ ID NO: 9 and a light chain which comprises a light chain variable region consisting of an amino acid sequence consisting of amino acid residues 21 to 129 of SEQ ID NO: 10; and

even more preferably, an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10, or a variant of the antibody in which a lysine residue at the carboxyl terminus of the heavy chain is deleted.

The average number of units of the drug-linker conjugated per antibody molecule in the anti-GPR20 antibody-drug conjugate is preferably 2 to 8, more preferably 3 to 8, even more preferably 7 to 8, even more preferably 7.5 to 8, and even more preferably about 8.

The anti-GPR20 antibody-drug conjugate can be produced with reference to descriptions in International Publication No. WO 2018/135501 and so on.

In the present invention, the term "anti-CDH6 antibody-drug conjugate" refers to an antibody-drug conjugate such that the antibody in the antibody-drug conjugate according to the invention is an anti-CDH6 antibody.

The anti-CDH6 antibody is preferably an antibody comprising a heavy chain consisting of CDRH1 consisting of an amino acid sequence consisting of amino acid residues 45 to 54 of SEQ ID NO: 11, CDRH2 consisting of an amino acid sequence consisting of amino acid residues 69 to 78 of SEQ ID NO: 11, and CDRH3 consisting of an amino acid sequence consisting of amino acid residues 118 to 130 of SEQ ID NO: 11, and a light chain consisting of CDRL1 consisting of an amino acid sequence consisting of amino acid residues 44 to 54 of SEQ ID NO: 12, CDRL2 consisting of an amino acid sequence consisting of amino acid residues 70 to 76 of SEQ ID NO: 12 and CDRL3 consisting of an amino acid sequence consisting of amino acid residues 109 to 116 of SEQ ID NO: 12;

more preferably, an antibody comprising a heavy chain which comprises a heavy chain variable region consisting of an amino acid sequence consisting of amino acid residues 20 to 141 of SEQ ID NO: 11 and a light chain which comprises a light chain variable region consisting of an amino acid sequence consisting of amino acid residues 21

to 128 of SEQ ID NO: 12; and

even more preferably, an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12, or a variant of the antibody in which a lysine residue at the carboxyl terminus of the heavy chain is deleted.

The average number of units of the drug-linker conjugated per antibody molecule in the anti-CDH6 antibody-drug conjugate is preferably 2 to 8, more preferably 3 to 8, even more preferably 7 to 8, even more preferably 7.5 to 8, and even more preferably about 8.

The anti-CDH6 antibody-drug conjugate can be produced with reference to descriptions in International Publication No. WO 2018/212136 and so on.

4. Anti-SIRPα antibody

SIRPα (signal regulatory protein α) is a single transmembrane molecule belonging to the Ig superfamily present in myeloid cells such as macrophages, dendritic cells and neutrophils, and glial cells. The extracellular region thereof consists of a single IgV domain and two IgC domains. The IgV domain, in which a connecting position to CD47 is present, is reported to have 10 variants V1 to V10 in humans. The extracellular IgV domain of the SIRPα protein is one of the three extracellular Ig-like domains constituting the SIRPα protein. Of the domains, V1 and V2 are major variants. The anti-SIRPα antibody of the present invention binds to all variants including the major variants V1 and V2. In the present invention, "SIRPα" will sometimes be referred to as "SIRPA".

The amino acid sequence of a human SIRPα protein is disclosed in GenBank Accession No.: NP_001035111.

The anti-SIRPα antibody used in the present invention can be obtained in the same manner as described in "2. Antibody in the antibody-drug conjugate".

The monoclonal antibody used in the present invention can be obtained by immunizing a mammal, such as a mouse, a rat, a rabbit, a hamster, a guinea pig, a horse, a monkey, a dog, a pig, a cow, a goat or a sheep, with SIRPα or a fragment thereof used as an immunogen, fusing the spleen cells and myeloma cells to obtain a hybridoma and allowing the hybridoma to produce and secrete the antibody. The hybridoma can be produced by a method known in the art.

SIRPα serving as an immunogen can be chemically synthesized based on sequence information or can be obtained as a recombinant protein, which is produced based on a DNA sequence encoding a protein and in accordance with a method known in the art.

The antibody can be screened by any method; preferably, by Cell-ELISA using animal cells transfected with DNA encoding SIRPα.

The anti-SIRPα antibody used in the present invention inhibits binding between SIRPα and CD47.

Tumor cells highly express CD47. When SIRPα expressed on phagocytes having phagocytic activity binds to CD47 and they interact, a "Don't-eat-me" signal is transmitted to the phagocytes. In this way, the tumor cells escape from phagocytosis by the phagocytes. The anti-SIRPα antibody inhibits the binding between SIRPα and CD47, thereby inhibiting transmission of a "Don'teat-me" signal from tumor cells to phagocytes, thereby enhancing phagocytosis by phagocytes to engulf tumor cells. As a result, an antitumor effect can be exerted. Examples of the phagocytes having phagocytic activity include macrophages such as M1 and M2 macrophages and dendritic cells such as immature dendritic cells (imDC).

In this case, when an anti-SIRPα antibody having an effector function binds to an Fc receptor such as Fcy receptors of effector cells such as phagocytes (e.g., macrophages), natural killer cells and T cells, the anti-SIRPα antibody attacks autologous effector cells such as PBMC (peripheral blood mononuclear cells) and macrophages due to ADCC (Antibody Dependent Cellular Cytotoxicity) and ADCP (Antibody Dependent Cellular Phagocytosis).

To avoid attacking autologous cells, the anti-SIRPα antibody used in the present invention is reduced in effector function. As a result, the anti-SIRPα antibody used in the present invention only has the function of suppressing the binding between SIRPα and CD47 and does not bind to an Fc receptor of effector cells; that is, an effector function is not produced.

The anti-SIRPα antibody used in the present invention, since it does not attack autologous immune cells, can be safely used as a medicine having no side effects.

However, the anti-SIRPα antibody used in the present invention, since it is reduced in effector function, does not produce a sufficient antitumor effect if it is used alone. Thus, the anti-SIRPα antibody is used in combination with another antitumor agent.

To reduce effector function, it is necessary for an Fc part of an anti-SIRPα antibody not to bind to Fc receptors of macrophages and T cells. For this reason, a subclass of the anti-SIRPα antibody used in the present invention is replaced with that derived from IgG4. Generally, of human IgG subclasses, IgG4 is known as a subclass low in effector function such as ADCC activity, CDC activity and/or ADCP activity (Bruggemann et al., J. Exp. Med., 1351-1361, 1987).

IgG4 is used as one of the IgG formats for avoiding toxicity due to cell damage via effector function, when a therapeutic antibody is used for targeting a molecule expressed in a healthy organ (e.g. Opdivo). Note that the effector function of the IgG4 subclass is low, but not zero. Then, in the anti-SIRPα antibody used in the present invention, a mutation such as a mutation that can further reduce the effector function, more specifically, a mutation such as a substitution with at least one amino acid for reducing ADCC and/or ADCP activity, is introduced into the heavy chain constant region. Examples of such a mutation include a substitution of phenylalanine with alanine at position 234 (F234A) according to the EU index by Kabat et al. (Kabat et. al., Sequences of proteins of immunological interest, 1991 Fifth edition), and a substitution of leucine with alanine at position 235 (L235A) (Parekh et al., mAbs, 310-318, 2012). Such a mutation of an antibody is referred to as a FALA mutation.

In IgG4, the S-S bond between antibody heavy chains is not stably formed. In order to improve the stability, a mutation for promoting the S-S bond between antibody heavy chains is introduced. Examples of the mutation include a substitution of serine with proline at position 228 (S228P) according to the EU index by Kabat et al. (ANGAL et.al., Molecular Immunology, 105-108, 1993). This mutation of an antibody is referred to as a PRO mutation.

In the constant region of the anti-SIRPα antibody used in the present invention, the aforementioned FALA mutation and PRO mutation may be simultaneously introduced (Vafa et.al., Methods, 65, 114-126, 2014). An IgG4 heavy chain having both the FALA mutation and Pro mutation is referred to as "IgG4proFALA" heavy chain, "IgG4PFALA" heavy chain, or "IgG4pf" heavy chain.

Of the human IgG subclasses, human IgG1 has a very strong effector function such as CDC activity via complement binding and an antibody-dependent cellular cytotoxity activity (Bruggemann et al., J. Exp. Med., 1351-1361, 1987), and is used as an IgG format exerting a therapeutic effect by promoting cell death of cancer cells due to cell damage via effector function when a therapeutic antibody is used for targeting a molecule highly expressed in cancer (e.g. trastuzumab, rituximab). When IgG1 is used as an isotype of the anti-SIRPα antibody used in the present invention, the effector function can be controlled by substituting part of the amino acid residues of the constant region (see, WO 88/007089, WO 94/28027, WO 94/29351). Examples of IgG1 mutants attenuated in effector function include IgG1 LALA (IgG1-L234A, L235A) and IgG1 LAGA (IgG1-L235A, G237A). As the constant region of the anti-SIRPα antibody used in the present invention, the IgG1 heavy-chain constant region having these mutations introduced therein can be used.

Of the human IgG subclasses, human IgG2 has a very weak effector function such as a CDC activity via complement binding and an antibody-dependent cellular cytotoxity activity (Bruggemann et al., J. Exp. Med., 1351-1361, 1987), and is used as one of the IgG formats for avoiding toxicity due to cell damage via effector function when a therapeutic antibody is used for targeting a molecule expressed in a healthy organ (e.g. denosumab, evolocumab, brodalumab). As the constant region of the anti-SIRPα antibody used in the present invention, the IgG2 heavy-chain constant region can be used.

In the anti-SIRPα antibody used in the present invention, modified variants of the antibody are also included. The modified variant refers to a variant obtained by subjecting the anti-SIRPα antibody to be used in the present invention to chemical or biological modification. Examples of the chemically modified variant include variants having a linkage of a chemical moiety to an amino acid skeleton and variants having a linkage of a chemical moiety to an N-linked or O-linked carbohydrate chain. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by expression using a prokaryotic host cell. Further, an antibody labeled so as to enable the detection or isolation of the anti-SIRPα antibody or antigen used in the present invention, for example an enzyme-labeled antibody, a fluorescence-labeled antibody and an affinity-labeled antibody, are also included in the meaning of the modified variant. Such a modified variant of the anti-SIRPα antibody used in the present invention is useful for, e.g., improving the stability and blood retention of the antibody, reducing the antigenicity thereof, and detecting or isolating an antibody or an antigen.

Note that it is known that a lysine residue at the carboxyl terminus of the heavy chain of an antibody produced in a cultured mammalian cell is deleted (Journal of Chromatography A, 705: 129-134 (1995)). It is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (e.g., activation of complement, antibody-dependent cellular cytotoxicity,) of the antibody. Therefore, in the anti-SIRPα antibody used in the present invention, antibodies subjected to such modification and functional fragments thereof are also included, and deletion variants in which one or two amino acids have been deleted at the carboxyl terminus of the heavy chain, deletion variants having an amidated residue (for example, a heavy chain in which the carboxyl terminal proline residue has been amidated) are also included. Note that the type of deletion variant having a deletion at the carboxyl terminus of the heavy chain of the anti-SIRPα antibody used in the present invention is not limited to the above variants as long as the antigen-binding affinity and the effector function are conserved. The two heavy chains constituting the anti-SIRPα antibody used in the present invention may be one selected from the group consisting of a full-length heavy chain and the above-described heavy chain having a deletion, or may

be of two types in combination selected therefrom. The ratio of the amount of each deletion variant can be affected by the type of cultured mammalian cells which produce the anti-SIRPα antibody used in the present invention and by the culture conditions; however, an antibody in which one amino acid residue at the carboxyl terminus has been deleted in both of the two heavy chains in the anti-SIRPα antibody used in the present invention can be preferably exemplified.

The anti-SIRPα antibody used in the present invention includes a chimeric antibody modified in order to reduce heterogeneous antigenicity to humans and a humanized antibody. The humanized antibody is also referred to as a CDR transplanted antibody.

The chimeric antibody refers to an antibody consisting of a light chain variable region and a heavy chain variable region of an antibody of a non-human animal, and a light chain constant region and a heavy chain constant region of a human antibody. The chimeric antibody can be prepared by taking cDNA encoding a light chain variable region and cDNA encoding a heavy chain variable region from a hybridoma producing an anti-SIRPα antibody, and inserting the cDNAs into an expression vector having cDNA encoding a light chain constant region and a heavy chain constant region of a human antibody to construct a chimeric antibody expression vector, introducing the chimeric antibody expression vector into host cells and allowing expression of the antibody.

The heavy chain constant region is formed of three domains $C_H1$, $C_H2$ and $C_H3$. In the anti-SIRPα antibody used in the present invention, the human heavy-chain constant region of the chimeric antibody is an IgG4-subclass heavy-chain constant region having a Pro mutation and FALA mutation, that is, a heavy chain constant region IgG4proFALA. The light chain constant region may be sufficient if it belongs to human Ig, and is a κ or λ constant region.

Examples of the chimeric antibody of the anti-SIRPα antibody used in the present invention include a chimeric antibody described in Patent Reference 4 (International Publication No. WO 2020/013170) having a variable region of rat anti-human SIRPα monoclonal antibody D13, i.e. antibody cD13. Antibody cD13 is an antibody having a high binding activity to bind to human SIRPα and having a high inhibitory activity against the binding between SIRPα and CD47.

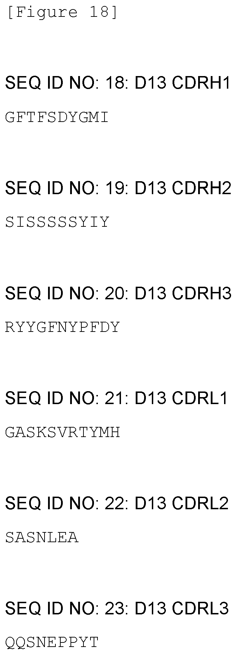

Antibody cD13 comprises, as CDRs (complementarity determining regions) of a light chain variable region, CDRL1 consisting of an amino acid sequence (GASKSVRTYMH) represented by SEQ ID NO: 21, CDRL2 consisting of an amino acid sequence (SASNLEA) represented by SEQ ID NO: 22, and CDRL3 consisting of an amino acid sequence (QQSNEPPYT) represented by SEQ ID NO: 23, and further, as CDRs of a heavy chain variable region, CDRH1 consisting of an amino acid sequence (GFTFSDYGMI) represented by SEQ ID NO: 18, and CDRH2 consisting of an amino acid sequence (SISSSSSYIY) represented by SEQ ID NO: 19, and CDRH3 consisting of an amino acid sequence (RYYGFNYPFDY) represented by SEQ ID NO: 20 (Figure 18).

More specifically, the anti-SIRPα antibody used in the present invention is an antibody comprising CDRL1 consisting of an amino acid sequence represented by SEQ ID NO: 21, CDRL2 consisting of an amino acid sequence represented by SEQ ID NO: 22, and CDRL3 consisting of an amino acid sequence represented by SEQ ID NO: 23, and, as CDRs of the heavy chain variable region, CDRH1 consisting of an amino acid sequence represented by SEQ ID NO: 18, CDRH2 consisting of an amino acid sequence represented by SEQ ID NO: 19, and CDRH3 consisting of an amino acid sequence represented by SEQ ID NO: 20.

The aforementioned CDRs include CDRs consisting of amino acid sequences having deletion, substitution and/or addition of one or several amino acids, preferably one or two amino acids, and further preferably, a single amino acid in each of the amino acid sequences of the CDRs.

A humanized antibody (CDR-transplanted antibody) refers to an antibody prepared by transplanting amino acid sequences of CDRs of a light chain variable region and a heavy chain variable region of an antibody of a non-human animal to appropriate positions of a light chain variable region and a heavy chain variable region of a human antibody.

The humanized anti-SIRPα antibody of the present invention can be produced by constructing cDNA encoding variable regions, which are obtained by transplanting the amino acid sequences of CDRs of a light chain variable region and a heavy chain variable region of an antibody of a non-human animal, which is produced from a hybridoma producing a monoclonal antibody binding to human SIRPα to inhibit the binding between SIRPα and CD47, thereby enhancing macrophage phagocytosis, to framework (FR) regions of a light chain variable region and a heavy chain variable region of a human antibody; inserting the cDNA into an animal-cell expression vector having genes encoding a light chain constant region and a heavy chain constant region of a human antibody to construct a humanized antibody expression vector; introducing the vector into animal cells; and expressing the cDNA.

More specifically, a DNA sequence designed such that the CDRs of an antibody cD13 are linked to framework regions of a human antibody, may be synthesized. The human antibody framework regions to be linked via CDRs are selected such that CDRs form satisfactory antigen connecting positions. If necessary, an amino acid of the framework region in the variable region of the antibody may be substituted such that CDRs of the humanized antibody form an appropriate antigen connecting position. A humanized antibody can be prepared by transplanting CDRs in accordance with CDR grafting technology known in the art.

As the heavy chain of the humanized antibody having CDRs (6 CDRs formed of amino acids represented by SEQ ID NOs: 18 to 23) of heavy chain and light chain variable regions of antibody cD13, which is prepared in accordance with the aforementioned method and has substitution of part of the amino acids in framework regions of the variable

region, humanized antibody heavy chain hH1 and humanized antibody heavy chain hH2 described in Patent Reference 4 (International Publication No. WO 2020/013170) can be exemplified. As the light chain of the humanized antibody having CDRs of a light chain variable region of antibody D13 and having substitution of part of the amino acids in framework regions of the variable region, humanized antibody light chain hL2, humanized antibody light chain hL3 and humanized antibody light chain hL4 described in Patent Reference 4 (International Publication No. WO 2020/013170) can be exemplified.

The full-length amino acid sequence of humanized antibody heavy chain hH1 is represented by SEQ ID NO: 13. The full-length amino acid sequence of humanized antibody heavy chain hH2 is represented by SEQ ID NO: 14. In SEQ ID NOs: 13 and 14, the amino acid sequence consisting of 1st to 19th amino acid residues is an amino acid sequence of a signal sequence; the amino acid sequence consisting of 20 to 139th amino acid residues is an amino acid sequence of a variable region; and the amino acid sequence consisting of 140 to 466th amino acid residues is an amino acid sequence of a constant region.

The anti-SIRPα antibody used in the present invention includes an antibody having a heavy chain variable region consisting of 20 to 139th amino acid residues of SEQ ID NO: 13 or 14 and a heavy chain constant region consisting of 140 to 466th amino acid residues thereof.

The full-length amino acid sequence of humanized antibody light chain hL2 is represented by SEQ ID NO: 15. The full-length amino acid sequence of humanized antibody light chain hL3 is represented by SEQ ID NO: 16. The full-length amino acid sequence of humanized antibody light chain hL4 is represented by SEQ ID NO: 17. In SEQ ID NOs: 15, 16 and 17, the amino acid sequence consisting of 1 to 20th amino acid residues is an amino acid sequence of a signal sequence; the amino acid sequence consisting of 21 to 127th amino acid residues is an amino acid sequence of a variable region; and the amino acid sequence consisting of 128 to 234th amino acid residues is an amino acid sequence of a constant region.

The anti-SIRPα antibody used in the present invention includes antibodies having light chain variable regions consisting of 21 to 127th amino acid residues of SEQ ID NO: 15, 16 and 17 and light chain constant regions consisting of 128 to 234th amino acid residues thereof.

The heavy chain constant region of the humanized antibody is a heavy chain constant region of the IgG4 subclass, more specifically, the heavy chain constant region IgG4proFALA having a Pro mutation and a FALA mutation.

Examples of the antibody having high binding activity to human SIRPα and a high inhibitory activity against the binding between SIRPα and CD47 include an antibody consisting of humanized antibody heavy chain hH1 and humanized antibody light chain hL3 (hD13_H1L3 antibody); an antibody consisting of humanized antibody heavy chain hH1 and humanized antibody light chain hL4 (hD13_H1L4 antibody); an antibody consisting of humanized antibody heavy chain hH2 and humanized antibody light chain hL2 (hD13_H2L2 antibody); and an antibody consisting of humanized antibody heavy chain hH2 and humanized antibody light chain hL3 (hD13_H2L3 antibody).

The hD13_H1L3 antibody is an antibody having a heavy chain consisting of 20 to 466th amino acid residues of SEQ ID NO: 13 and a light chain consisting of 21 to 234th amino acid residues of SEQ ID NO: 16.

The hD13_H1L4 antibody is an antibody having a heavy chain consisting of 20 to 466th amino acid residues of SEQ ID NO: 13 and a light chain consisting of 21 to 234th amino acid residues of SEQ ID NO: 17.

The hD13_H2L2 antibody is an antibody having a heavy chain consisting of 20 to 466th amino acid residues of SEQ ID NO: 14 and a light chain consisting of 21 to 234th amino acid residues of SEQ ID NO: 15.

The hD13_H2L3 antibody is an antibody having a heavy chain consisting of 20 to 466th amino acid residues of SEQ ID NO: 14 and a light chain consisting of 21 to 234th amino acid residues of SEQ ID NO: 16.

As other anti-SIRPα antibodies used in the present invention, antibodies described in Patent References 1 to 3 can be exemplified.

As one of the antibodies described in Patent Reference 1 (International Publication No. WO 2017/178653), OSE-172 can be exemplified. The heavy-chain amino acid sequence of OSE-172 is shown in SEQ ID NO: 24 of the sequence list; whereas the light-chain amino acid sequence of OSE-172 is shown in SEQ ID NO: 25. OSE-172 is an antibody having a heavy chain consisting of 20 to 466th amino acid residues of SEQ ID NO: 24 and a light chain consisting of 21 to 239th amino acid residues of SEQ ID NO: 25.

As one of the antibodies described in Patent Reference 2 (International Publication No. WO 2018/026600), KWAR23 can be exemplified. The heavy-chain amino acid sequence of KWAR23 is shown in SEQ ID NO: 26; whereas the light-chain amino acid sequence of KWAR23 is shown in SEQ ID NO: 27. KWAR23 is an antibody having a heavy chain consisting of 20 to 459th amino acid residues of SEQ ID NO: 26 and a light chain consisting of 21 to 235th amino acid residues of SEQ ID NO: 27.

As one of the antibodies described in Patent Reference 3 (International Publication No. WO 2018/190719), ADU-1805 can be exemplified. The heavy-chain amino acid sequence of ADU-1805 is shown in SEQ ID NO: 28; whereas the light-chain amino acid sequence of ADU-1805 is shown in SEQ ID NO: 29. ADU-1805 is an antibody having a heavy chain consisting of 20 to 467th amino acid residues of SEQ ID NO: 28 and a light chain consisting of 21 to 234th amino acid residues of SEQ ID NO: 29.

Note that, in an antibody produced in a cultured mammalian cell, it is known that a lysine residue at the carboxyl terminus of the heavy chain is deleted (Tsubaki et.al., Int. J. Biol. Macromol, 139-147, 2013). However, the deletion of the heavy chain sequence does not affect the antigen-binding affinity and the effector function (e.g. activation of a complement and antibody-dependent cellular cytotoxicity) of the antibody. Thus, in the present invention, an antibody lacking a lysine residue at the carboxyl terminus of the heavy chain is included.

The cancer therapeutic agent used in the present invention can contain a therapeutically effective amount of an anti-SIRPα antibody and a pharmaceutically acceptable carrier, diluent, solubilizer, emulsifier, preservative, auxiliary agent and others. The "pharmaceutically acceptable carrier" and others can be appropriately selected from a wide range in accordance with the type of target disease and the dosage form of a drug. A method for administering an antitumor agent according to the present invention can be appropriately selected, for example, administration by injection can be selected. Examples of the injection that can be employed include local injection, intraperitoneal injection, selective intravenous injection, intravenous injection, subcutaneous injection and organ-perfusate injection. The injection solution can be formulated by using a carrier consisting of a salt solution, a glucose solution, or a mixture of a salt solution and a glucose solution, and various buffers. Alternatively, the injection solution may be prepared by formulating a powder preparation and mixing the powder preparation with the aforementioned liquid carrier, when used.

Other administration methods can be appropriately selected together with development of formulations. For example, for oral administration, oral liquids, powders, pills, capsules and tablets can be applied. In the case of an oral liquid, an oral liquid preparation such as a suspension and a syrup can be produced by using water; a sugar such as sucrose, sorbitol and fructose; a glycol such as polyethylene glycol; an oil such as sesame oil and soybean oil; a preservative such as alkyl parahydroxybenzoate; and a flavor such as strawberry flavor and peppermint flavor. Powders, pills, capsules and tablets can be formulated by using, e.g., an excipient such as lactose, glucose, sucrose and mannitol; a disintegrant such as starch and soda alginate; a lubricant such as magnesium stearate and talc; a binder such as polyvinyl alcohol, hydroxypropyl cellulose and gelatin; a surfactant such as a fatty acid ester; and a plasticizer such as glycerin. Tablets and capsules are easily administered. In this respect, they are preferable unit dosage forms of the composition of the invention. In producing tablets and capsules, a solid production carrier is used.

5. Relationship between immunogenic cell death and anti-SIRPα antibody

Immunogenic cell death (ICD) is cell death characterized by massive release of intracellular molecules such as ATP and HMGB1 (High-mobility group box1 protein) and exposure of Calreticulin (CRT) on the cell surface. These are Danger signals, which activate immune cells. It is reported that ATP is responsible for recruiting and activating dendritic cells (DC) and macrophages; HMGB1 is responsible for enhancing production of inflammatory cytokines such as Type I IFN; and CRT serves as an "eat-me-signal" and enhances antigen uptake from dead cells (Nature Reviews Immunology. 2017, 17, 97-111). In short, when ICD of cancer cells occurs, immunity (antitumor immunity) against the cancer cells can be induced. As the anti-cancer agent inducing ICD, e.g. an Anthracycline drug, Oxaliplatin and Cyclophosphamide are known; however, e.g., Docetaxel and Mitomycin C are not confirmed to induce ICD. The presence or absence of ICD effect can be evaluated not only in vitro based on whether Danger-signal is detected or not by adding an agent but also in vivo based on vaccination assay. In the latter case, cancer cells are treated with an agent and inoculated into an immunocompetent mouse. A week later, cancer cells not treated with the agent are inoculated into an opposite side. At this time, if immunological memory is already formed by the cancer cells, which caused ICD, engraftment and proliferation of the inoculated cancer cells are inhibited (Cancer Research, 2017, 77, 2686-2698).

For formation of immunological memory by ICD, it is important for myeloid cells such as dendritic cells and macrophages to take up a cancer antigen. At this time, it is presumed that a "Don't-eat-me signal" is transmitted between the myeloid cells and cancer cells by SIRPa-CD47. If an anti-SIRPα antibody is administered, the signal transmission can be inhibited and phagocytosis is enhanced. As a result, uptake of the cancer antigen can be enhanced. The cancer antigen taken up by dendritic cells and/or macrophages is intracellularly processed into 8-30 mer peptide fragments, which are presented on MHC. MHC has two classes, class I and class II. An about 9-mer antigen peptide presented to MHC-class I activates CD8+T cells; whereas an about 15-mer antigen peptide presented to MHC-class II activates CD4+T. Generally, a foreign antigen is processed in myeloid cells and presented to MHC-class II; however, part of DC subsets presents the foreign antigen to MHC-class I and activates CD8+T exerting cytotoxic activity to cancer cells; in short, the part of the DC subsets has cross presentation ability.

DC has subsets of cDC1 and cDC2. cDC1 is negative to SIRPα and has cross presentation ability; whereas cDC2 is positive to SIRPα and does not have cross presentation ability. Recently, it has been reported at a scientific workshop that, in an antitumor study using a mouse syngeneic model, the number of cDC1 subsets increases by administration of an anti-SIRPα antibody, and that cross presentation ability to CD8+T cells is enhanced by addition of an anti-SIRPα antibody to a co-culture system of cancer cells with DC and T cells. From the above, administration of an anti-SIRPα antibody not only promotes phagocytosis of cancer cells and uptake of a cancer antigen but also enhances cross presentation ability, with the result that induction of CD8+T cells, which play a key role in tumor immunity, can be

enhanced.

The relationship of an antibody-drug conjugate, in which a drug having a cytocidal activity is integrated therein, and ICD induction, has been reported in the past in the case of antibody-drug conjugates having Tubulysin, Pyrrolobenzodiazepine (PBD) and MMAE integrated therein (Cancer Research, 2017, 77, 2686-2698 or ONCOIMMUNOLOGY, 2019, 8(4), e1565859). With respect to an antibody-drug conjugate used in the present invention containing a TpoI inhibitor, compound (A), as a payload, it has only been reported that HMGB1 is released from cells treated with compound (A) and a limited antitumor effect is exerted by vaccination of the treated cells (Clin. Invest. 2020: 130(1): 374-388). This time, the following is found: 1) compound (A) induces release of not only HMGB1 but also other Danger signals from dying cancer cells; 2) compound (A) activates immune cells present under the microenvironment of cancer and enhances a cancer antigen specific T cell population to induce an antitumor immunity; and 3) immune response induced by compound (A) is enhanced by an anti-SIRPα antibody.

6. Medicine

Now, a pharmaceutical composition and a method of treatment according to the present invention, wherein an antibody-drug conjugate and an anti-SIRPα antibody are administered in combination, will be described.

The pharmaceutical composition and method of treatment of the present invention may be those in which the antibody-drug conjugate and the anti-SIRPα antibody are separately contained as active components in different formulations and are administered simultaneously or at different times, or may be those in which the antibody-drug conjugate and the anti-SIRPα antibody are contained as active components in a single formulation and administered.

The pharmaceutical composition and method of treatment of the present invention can be used for treating cancer, preferably for treating at least one disease selected from the group consisting of breast cancer, gastric cancer (also called to as gastric adenocarcinoma), colorectal cancer (also called colon and rectal cancer, and including colon cancer and rectal cancer), lung cancer (including small cell lung cancer and non-small cell lung cancer), esophageal cancer, head-and-neck cancer (including salivary gland cancer and pharyngeal cancer), gastroesophageal junction adenocarcinoma, biliary tract cancer (including bile duct cancer), gallbladder cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, thymus cancer, penis cancer, leukemia, lymphoma, malignant lymphoma, plasmacytoma, myeloma, myelodysplastic syndrome, brain tumor, glioma, glioblastoma multiforme, osteosarcoma, and melanoma; more preferably for treating at least one cancer selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, and uterine carcinosarcoma; and even more preferably for treating at least one cancer selected from the group consisting of breast cancer, gastric cancer, lung cancer, and ovarian cancer.

Among the antibody-drug conjugates used in the present invention, the kind of antibody preferably used in the antibody-drug conjugate can be determined by examining the type of cancer and tumor markers. For example, if HER2 expression is found in the cancer, an anti-HER2 antibody-drug conjugate can preferably be used; if HER3 expression is found in the cancer, an anti-HER3 antibody-drug conjugate can preferably be used; if TROP2 expression is found in the cancer, an anti-TROP2 antibody-drug conjugate can preferably be used; if B7-H3 expression is found in the cancer, an anti-B7-H3 antibody-drug conjugate can preferably be used; if GPR20 expression is found in the cancer, an anti-GPR20 antibody-drug conjugate can preferably be used; if CDH6 expression is found in the cancer, an anti-CDH6 antibody-drug conjugate can preferably be used.

The presence or absence of HER2, HER3, TROP2, B7-H3, GPR20, and CDH6, and other tumor markers can be checked by, for example, collecting tumor tissue from a cancer patient, and subjecting the formalin-fixed paraffin-embedded specimen (FFPE) to examination at a gene product (protein) level such as an immunohistochemistry (IHC) method, a flow cytometry, or a western blot method, or examination at a gene translation level such as an in situ hybridization method (ISH), a quantitative PCR method (q-PCR), or a microarray analysis, or alternatively can also be checked by collecting cell-free blood circulating tumor DNA (ctDNA) from a cancer patient and subjecting it to an examination which uses a method such as next generation sequencing (NGS).

The pharmaceutical composition and method of treatment of the present invention can preferably be used for mammals, and can more preferably be used for humans.

The antitumor effect of the pharmaceutical composition and method of treatment of the present invention can be confirmed by, for example, generating a model in which cancer cells are inoculated into a test animal, and measuring reduction in tumor volume, life-prolonging effects due to applying the pharmaceutical composition and method of treatment of the present invention. Furthermore, comparison with the antitumor effect of single administration of each of the antibody-drug conjugate and the anti-SIRPα antibody used in the present invention can provide confirmation of the combined effect of the antibody-drug conjugate and the anti-SIRPα antibody used in the present invention.

In addition, the antitumor effect of the pharmaceutical composition and method of treatment of the present invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS) .

The foregoing methods can provide confirmation of superiority in terms of the antitumor effect of the pharmaceutical composition and method of treatment of the present invention compared to existing pharmaceutical compositions and methods of treatment for cancer therapy.

The pharmaceutical composition and method of treatment of the present invention can retard growth of cancer cells, suppress their proliferation, and further can kill cancer cells. These effects can allow cancer patients to be free from symptoms caused by cancer or achieve an improvement in the QOL of cancer patients and attain a therapeutic effect by sustaining the lives of the cancer patients. Even if the pharmaceutical composition and method of treatment of the present invention do not accomplish the killing of cancer cells, they can achieve higher QOL of cancer patients while achieving longer-term survival, by inhibiting or controlling the growth of cancer cells.

The pharmaceutical composition of the present invention can exert a therapeutic effect by application as systemic therapy to patients, and additionally, by local application to cancer tissues.

The pharmaceutical composition of the present invention may be administered as a pharmaceutical composition containing at least one pharmaceutically suitable ingredient. The pharmaceutically suitable ingredient can be suitably selected and applied from formulation additives or the like that are generally used in the art, in view of the dosage, administration concentration or the like of the antibody-drug conjugate and the anti-SIRPα antibody used in the present invention. For example, the antibody-drug conjugate used in the present invention may be administered as a pharmaceutical composition containing a buffer such as a histidine buffer, an excipient such as sucrose or trehalose, and a surfactant such as polysorbate 80 or 20. The pharmaceutical composition containing the antibody-drug conjugate used in the present invention can preferably be used as an injection, can more preferably be used as an aqueous injection or a lyophilized injection, and can even more preferably be used as a lyophilized injection.

In the case that the pharmaceutical composition containing the antibody-drug conjugate used in the present invention is an aqueous injection, it can preferably be diluted with a suitable diluent and then given as an intravenous infusion. For the diluent, a dextrose solution, physiological saline, and the like, can be exemplified, and a dextrose solution can be exemplified preferably, and a 5% dextrose solution can be exemplified more preferably.

In the case that the pharmaceutical composition containing the antibody-drug conjugate used in the present invention is a lyophilized injection, it can preferably be dissolved in water for injection, subsequently a required amount can be diluted with a suitable diluent and then given as an intravenous infusion. For the diluent, a dextrose solution, physiological saline, and the like, can be exemplified, and a dextrose solution can be exemplified preferably, and a 5% dextrose solution can be exemplified more preferably.

Examples of the administration route which may be used to administer the pharmaceutical composition of the present invention include intravenous, intradermal, subcutaneous, intramuscular, and intraperitoneal routes; and preferably include an intravenous route.

The antibody-drug conjugate used in the present invention can be administered to a human once at intervals of 1 to 180 days, and can preferably be administered once a week, once every 2 weeks, once every 3 weeks, or once every 4 weeks, and can even more preferably be administered, once every 3 weeks. Also, the antibody-drug conjugate used in the present invention can be administered at a dose of about 0.001 to 100 mg/kg, and can preferably be administered at a dose of 0.8 to 12.4 mg/kg. In the case that the antibody-drug conjugate used in the present invention is an anti-HER2 antibody-drug conjugate, it can preferably be administered once every 3 weeks at a dose of 0.8 mg/kg, 1.6 mg/kg, 3.2 mg/kg, 5.4 mg/kg, 6.4 mg/kg, 7.4 mg/kg, or 8 mg/kg. In the case that the antibody-drug conjugate used in the present invention is an anti-HER3 antibody-drug conjugate, it can preferably be administered once every 3 weeks at a dose of 1.6 mg/kg, 3.2 mg/kg, 4.8 mg/kg, 5.6 mg/kg, 6.4 mg/kg, 8.0 mg/kg, 9.6 mg/kg, or 12.8 mg/kg. In the case that the antibody-drug conjugate used in the present invention is an anti-TROP2 antibody-drug conjugate, it can preferably be administered once every 3 weeks at a dose of 0.27 mg/kg, 0.5 mg/kg, 1.0 mg/kg, 2.0 mg/kg, 4.0 mg/kg, 6.0 mg/kg, 8.0 mg/kg, or 10.0 mg/kg. The anti-SIRPα antibody according to the present invention can be administered to a human once at intervals of 1 to 180 days, and can preferably be administered once a week, once every 2 weeks, once every 3 weeks, or once every 4 weeks. Also, the anti-SIRPα antibody according to the present invention can be administered at a dose of about 0.001 to 100 mg/kg per dose.

The pharmaceutical composition and method of treatment of the present invention may further contain a cancer therapeutic agent other than the antibody-drug conjugate and the anti-SIRPα antibody according to the present invention. The pharmaceutical composition and method of treatment of the present invention can also be administered in combination with another cancer therapeutic agent, thereby enhancing the antitumor effect. Other cancer therapeutic agents to be used for such purpose may be administered to a subject simultaneously, separately, or sequentially with the

pharmaceutical composition of the present invention, or may be administered with varying each dosage interval. Such cancer therapeutic agents are not limited as long as they are agents having antitumor activity, and can be exemplified by at least one selected from the group consisting of irinotecan (CPT-11), cisplatin, carboplatin, oxaliplatin, fluorouracil (5-FU), gemcitabine, capecitabine, doxorubicin, epirubicin, cyclophosphamide, mitomycin C, tegafur-gimeracil-oteracil combination, panitumumab, bevacizumab, ramucirumab, regorafenib, trifluridine-tipiracil combination, gefitinib, erlotinib, afatinib, methotrexate, pemetrexed, tamoxifen, toremifene, fulvestrant, leuprorelin, goserelin, letrozole, anastrozole, progesterone formulation, and lapatinib.

In the pharmaceutical composition and method of treatment of the present invention, immune checkpoint inhibitors and antibody drugs specifically binding to a cancer antigen and having ADCC and/or ADCP activity may be further contained as the cancer therapeutic agent to be used in combination, other than the antibody-drug conjugate and the anti-SIRPα antibody according to the present invention. As the immune checkpoint inhibitor, an inhibitor of binding between PD-1 and its ligand, PD-L1, or a CTLA4 inhibitor is exemplified. Specific examples thereof include an anti-PD-1 antibody (nivolumab, pembrolizumab, cemiplimab, spartalizumab, PDR-001, or BI 754091), an anti-PD-L1 antibody (atezolizumab, avelumab, or durbarumab), and an anti-CTLA4 antibody (ipilimumab or tremelimumab). Examples of the antibody drug specifically binding to a cancer antigen and having ADCC and/or ADCP activity include an anti-CD20 antibody (rituximab), an anti-HER2 antibody (trastuzumab or pertuzumab), an anti-EGFR antibody (cetuximab), and an anti-CD52 antibody (alemutuzumab).

ADCC refers to a cell-mediated reaction in which non-specific cytotoxic cells (for example, NK cells, neutrophils and macrophages) expressing an Fcy receptor recognize an antibody bound to a target cell, and then cause lysis of the target cell. In NK cells, which are primary cells responsible for ADCC, FcyRIIC and FcyRIIIA are expressed. In monocytes, FcyRI, FcyRIIA, FcyRIIC and FcyRIIIA are expressed. On the other hand, ADCP refers to a cell-mediated reaction, in which phagocytes (for example, macrophages, neutrophils) expressing an Fc receptor recognize an antibody bound to a target cell, and then engulf the target cell within the cells. In the monocytes, which are primary cells responsible for ADCP, FcyRI, FcyRIIA, FcyRIIC and FcyRIIIA are expressed.

The pharmaceutical composition and method of treatment of the present invention can also be used in combination with radiotherapy. For example, a cancer patient may receive radiotherapy before and/or after or simultaneously with receiving a therapy with the pharmaceutical composition of the present invention.

The pharmaceutical composition and method of treatment of the present invention can also be used as an adjuvant chemotherapy in combination with a surgical procedure. The pharmaceutical composition of the present invention may be administered for the purpose of diminishing the size of a tumor before a surgical procedure (referred to as pre-operative adjuvant chemotherapy or neoadjuvant therapy), or may be administered after a surgical procedure for the purpose of preventing the recurrence of a tumor (referred to as post-operative adjuvant chemotherapy or adjuvant therapy).

[Examples]

The present invention is specifically described in view of the examples shown below. However, the present invention is not limited to these. Further, it is by no means to be interpreted in a limited way.

Production Example 1: Production of the antibody-drug conjugate (1)

In accordance with a production method described in International Publication No. WO 2015/115091 with use of a humanized anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, hereinafter will be "referred to as humanized anti-HER2 antibody (1)"), an antibody-drug conjugate in which a drug-linker represented by the following formula:

[Formula 14]

wherein A represents the connecting position to an antibody,
is conjugated to an anti-HER2 antibody via a thioether bond (hereinafter referred to as "antibody-drug conjugate (1)") was produced. The DAR of the antibody-drug conjugate (1) is 7.7 or 7.8.

Production Example 2: Production of the antibody-drug conjugate (2)

In accordance with a production method described in International Publication Nos. WO 2015/098099 and 2017/002776 with use of a humanized anti-TROP2 antibody (an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, hereinafter will be referred to as humanized anti-TROP2 antibody (1)), an antibody-drug conjugate in which a drug-linker represented by the following formula:

[Formula 15]

wherein A represents the connecting position to an antibody,
is conjugated to an anti-TROP2 antibody via a thioether bond (hereinafter referred to as "antibody-drug conjugate (2)") was produced. Although DAR of the antibody-drug conjugate (2) can be controlled within the range of from 0 to 8, an antibody-drug conjugate having an average number of conjugated drugs of 3.5 to 4.5 was produced herein.

Production Example 3: Preparation of Compound (A)

In accordance with a production method described in International Publication No. WO 2014/057687, a compound represented by the following formula:

[Formula 16]

(Compound (A)) was produced.

Production Example 4: Preparation of anti-mouse SIRPα antibody (clone: 5C12)

Anti-mouse SIRPα antibody 5C12 was established by the following method. For immunization, WKY/Izm or female Wister rats (Japan SLC, Inc.) were used. A mouse SIRPα protein (manufactured by Sino Biological Inc.) and Freund's Complete Adjuvant (manufactured by Wako Pure Chemical Industries, Ltd.) were mixed and subcutaneously administered to the tail of the rat. The lymph node of the rat was taken and used for production of a hybridoma.

The lymph node cells and mouse myeloma SP2/0-ag14 cells (ATCC: CRL-1581) were fused in accordance with a polyethylene glycol method. The fused cells were suspended in HAT selective medium and cultured in accordance with the limiting dilution method. The hybridoma colonies that emerged were collected. In this manner, monoclonal hybridomas were prepared. The collected hybridoma colonies were cultured. Using the resultant hybridoma culture supernatant, an antibody-producing hybridoma was screened based on binding activity to a mouse SIRPα protein as an index. In this manner, clone: 5C12 was screened.

For amplifying cDNA encoding a variable region of 5C12, total RNA was prepared from the 5C12-producing hybridoma by use of TRIzol Reagent (Ambion, Inc.). Based on the total RNA, cDNA encoding heavy chain and light chain variable regions was amplified by use of SMARTer RACE 5'/3' Kit (Clontech, Inc.). The cDNA encoding heavy chain and light chain variable regions amplified by 5'-RACE PCR were cloned to a plasmid. Subsequently, the nucleotide sequence of the cDNA encoding heavy chain and light chain variable regions was analyzed. The amino acid sequences of 5C12 heavy chain and light chain are shown in SEQ ID NOs: 30 and 31, respectively.

The homology between human SIRPα and mouse SIRPα is as low as 60%. It is reported that humans have 10 types of variants in the IgV domain of SIRPα, which is an interaction site with CD47; and mice have at least 4 types of variants due to differences in genetic background. Because of this, it is presumably difficult to obtain a functional antibody having cross-reactivity to the orthologue of a human and a mouse, at which SIRPα-CD47 interaction can be inhibited. Actually, functional antibodies having a cross-reactivity to a mouse have not yet been found in a rat immunized with a human antigen.

SIRPα is a molecule that is present in myeloid cells such as macrophages and dendritic cells. In the case of a drug directly producing an anti-cancer effect on cancer, generally, the drug can be evaluated by use of a xenograft model prepared by inoculating a human cancer cell line into an immunodeficient mouse. However, in order to evaluate an antitumor effect of a target molecule like SIRPα expressed in host immune cells, any one of the following methods must be employed: (1) using a model, which is prepared by inoculating into an immunocompetent mouse a mouse cancer cell line having a compatible genetic background, and a surrogate antibody having a cross-reactivity to mouse SIRPα; (2) using a model, which is prepared by introducing a human SIRPα gene into an immunodeficient mouse and inoculating a human cancer cell line into it, and an anti-human SIRPα antibody; (3) using a mouse model, which is prepared by inoculating a mouse cancer cell line having a human CD47 gene expressed therein into an immunocompetent mouse having an SIRPα target gene introduced therein, and an anti-human SIRPα antibody. Of them, the method (2) has a problem in that, since a T cell defective immunodeficient mouse is used, the effect on an acquired immune system (immune response mainly by T cells) when SIRPα is inhibited cannot be evaluated; and the method (3) has a problem in that preparation takes a long time and the effect of, e.g., control of SIRPα gene expression level on the antitumor

effect is not easily estimated. On the other hand, in the case of the method (1) using a surrogate antibody, evaluation can be made in a general mouse model; in addition, it has been reported that a combined effect is obtained when it is used in combination with an anti-cancer antibody and an immune checkpoint antibody in clones such as MY-1 and P84 (International Publication No. WO 2017/178653, or Yanagita et al., JCI Insight, 2017 (2)1, 1-15). From this, in this experiment, 5C12 clone was obtained as an anti-mouse SIRPα antibody and the antitumor effect was evaluated. 5C12 has a high binding affinity to the mouse SIRPα antigen. It is confirmed that the binding affinity is at the same level as that of an anti-human SIRPα antibody hD13_H1L3 to a human SIRPα antigen. Note that a plurality of PD-1 antibodies already marketed were evaluated by use of a surrogate antibody in non-clinical pharmacology studies, and thereafter, the effect of the antibodies was clinically confirmed. From the foregoing, it can be expected to be able to extrapolate the immunological evaluation results and evaluation results of antitumor studies using an anti-SIRPα surrogate antibody to results in humans.

Production Example 5: Preparation of humanized anti-SIRPα antibody (clone: hD13_H1L3)

In accordance with a production method described in International Publication No. WO 2020/013170, humanized anti- SIRPα antibody (clone: hD13_H1L3) was produced.

Production Example 6: Production of the antibody-drug conjugate (3)

In accordance with a production method described in International Publication No. WO 2015/155998 with use of a humanized anti-HER3 antibody (an antibody comprising a heavy chain consisting of an amino acid sequence consisting of SEQ ID NO: 3 and a light chain consisting of an amino acid sequence consisting of SEQ ID NO: 4, hereinafter will be "referred to as humanized anti-HER3 antibody (1)"), an antibody-drug conjugate in which a drug-linker represented by the following formula:

[Formula 16]

wherein A represents the connecting position to an antibody,

is conjugated to an anti-HER3 antibody via a thioether bond (hereinafter referred to as "antibody-drug conjugate (3)") was produced. The DAR of the antibody-drug conjugate (3) is 7.7 or 7.8.

Example 1: ICD induction evaluation (in vitro):

Measurement of ATP/HMGB1 in culture supernatant

In cancer cells treated with different compounds, the ATP level, and the expression level of HMGB1, which serve as an index of Immunogenic cell death (ICD), were measured. Cells of mouse colorectal cancer cell line CT26.WT were seeded in a 6-well plate and cultured overnight. After the supernatant was removed, the cells were washed twice with PBS. Compound (A) was dissolved in RPMI1640 culture solution containing 10% FBS (R10) to prepare solutions having a final concentration of 0, 1, 4 and 16 μM. As a positive control, Mitoxantrone (MTX) was dissolved in DMSO and added at a final concentration of 0.4, 1.5 and 6 μM. To the control (0 μM) herein, DMSO was added in the same

amount as that of compound (A) (R10-DMSO). Twenty four hours later, the culture supernatant was collected, and the levels of ATP and HMGB1 were measured. The level of ATP was measured by adding the culture supernatant in a 96-well white plate, adding the same amount of Back titer-Glo (manufactured by Promega Corporation) and determining the amount of luminescence by a plate reader. The level of HMGB1 was detected by a HMGB1 ELISA-kit (manufactured by Shino-Test Corporation) in accordance with the Sandwich ELISA method. In the plate provided in the kit, a test buffer was added, and thereafter, an HMGB1 standard solution diluted with the test buffer and the culture supernatant were added thereto. It was allowed to stand at 37°C for 24 hours. After the supernatant was removed and the cells were washed five times with PBS/Tween 20 (200 μE/well), a secondary-antibody solution provided in the kit was added in an amount of 100 μL/well. It was allowed to stand at room temperature for 2 hours. After the supernatant was removed, the cells were washed five times with PBS/Tween 20 (200 μL/well). Thereafter, a color-producing solution provided in the kit was added in an amount of 100 μL/well. The plate was allowed to stand at room temperature for 30 minutes. A stop solution (100 μL/well) was added and stirred. The absorbance at 405 nm was measured by a plate reader. The results of the control group and compound (A) group were compared in accordance with the Dunnett's test. P value was expressed to four decimal places. The results satisfying $P < 0.05$ (two-sided test) were determined as being significant.

The results are shown in Figure 24A-D. In MTX treatment groups, release of ATP at a maximum of about 4 times as large as those of the control (0 μM) was confirmed; and release of HMGB1 at a maximum of about 3 time as large as those of the control (0 μM) was confirmed in the culture supernatant after 24 hours ($P < 0.0001$). On the other hand, in compound (A)-treatment groups, release of ATP and HMGB1 at a maximum of about 4.6 times as large as that of R10 or R10-DMSO was confirmed ($P < 0.0001$).

From the above, it was suggested that compound (A) significantly increases release of ATP and HMGB1, which serve as an index of ICD induction, from cancer cells, in vitro.

Example 2: ICD induction evaluation (in vitro):

Measurement of Calreticulin (CRT) on cell surface

In cancer cells treated with different compounds, the expression level of Calreticulin (CRT), which serves as an index of Immunogenic cell death (ICD), was measured. Cells of mouse colorectal cancer cell line CT26.WT were seeded in a 6-well plate and cultured overnight. After the supernatant was removed, the cells were washed twice with PBS. In RPMI1640 culture solution containing 10% FBS (R10), compound (A), (4 μM) or Mitoxantrone (MTX) (1 μM) was added. As a control, DMSO was added in the same amount as that of compound (A) (R10-DMSO). After completion of drug treatment, CT26.WT cells were collected and suspended in PBS, and then, seeded in a 96-well (round bottom) plate at $1 \times 10^6$ cells/well. After centrifugation at 1200 rpm for 3 minutes, the supernatant was removed, and then, LIVE/DEAD Fixable Violet Dead Cell Stain Kit (manufactured by Thermo Fisher Scientific Inc.) and Mouse FcyR Blocker (manufactured by Biolegend, Inc.) were diluted with PBS to 1/1000 and 1/50 and added at 100 μL/well respectively. After standing at room temperature for 30 minutes, centrifugation was carried out at 1200 rpm for 3 minutes. The supernatant was removed, Mildform (manufactured by Wako Pure Chemical Industries, Ltd.) was added in an amount of 100 μE/well and allowed to stand at 37°C for 10 minutes. After centrifugation, the supernatant was removed. The cells were washed once with FACS buffer (1 mM EDTA, 5% FBS) (200 μL/well). After centrifugation, the supernatant was removed. A primary-antibody solution diluted with FACS buffer (anti-CRT labeled with PE or AF647: both manufactured by Abcam plc.) was added in an amount of 50 μL/well. After standing in a dark place at 4°C for 25 minutes, centrifugation was carried out at 1200 rpm for 3 minutes. The supernatant was removed. The cells were washed twice with FACS buffer (200 μL/well) and centrifugation was carried out. After the supernatant was removed, the cells were suspended in Stabilizing Fixative (manufactured by Becton, Dickinson and Company) and allowed to stand at room temperature for 10 minutes. The expression level of CRT on the cancer cells was measured by flow cytometry (FACS CantoII: manufactured by Becton, Dickinson and Company). Data were analyzed by FlowJo (manufactured by TreeStar Inc). Note that, LIVE/DEAD Fixable Violet-positive cells were determined as dead cells and excluded from the analysis. The mean fluorescence intensity (MFI) of CRT was calculated. The value, which was obtained by subtracting the MFI of the cells treated with Isotype control from the MFI of stained cells, was determined as the adjusted MFI. Note that the experiment was performed in triplicate. The results of the control group (0 μM) and the drug groups were compared in accordance with the Dunnett's test. P value was expressed to four decimal places. The results satisfying $P < 0.05$ (two-sided test) were determined as being significant (***: $P < 0.001$, **: $P < 0.01$).

The results are shown in Figure 25. In the surface of CT26.WT cells after 24 hours, a significant increase of CRT expression was confirmed. The CRT expression of a sample treated with MTX was about 2.6 times ($P < 0.0001$) as large as that of the control group. The CRT expression of a sample treated with compound (A) was about 12.7 times ($P < 0.0001$) as large as that of the control group.

From the above, it was demonstrated that compound (A) significantly promotes ICD induction of cancer cells, in vitro, based on exposure of CRT on the cell surfaces.

Example 3: Evaluation of in vivo ICD induction

Cells of mouse colorectal cancer cell line CT26.WT were seeded in a cell-culture flask and cultured overnight. The supernatant was removed and the cells were washed twice with PBS. To induce ICD, compound (A) (4 μM) or MTX (1 μM) was added in a RPMI1640 culture solution containing 10% FBS (R10). Twenty four hours later, HMGB1 in the culture supernatant was measured in accordance with the method shown in Example 1 in order to confirm ICD induction.

As shown in Figure 26, it was confirmed that HMGB1 was released in the culture supernatant after 24 hours in the drug treatment groups.

For evaluating in vivo ICD, the culture supernatant was removed from each of the culture flasks treated with drugs and the cells were washed twice with PBS, and then cells were collected. The supernatant was removed and the cells were washed once with PBS to prepare a suspension containing $5.0 \times 10^6$ cells/mL. As a control, a suspension was prepared by adding CT26.WT cells, which were cultured in a RPMI1640 culture solution containing 10% FBS without a drug, in an amount of $5.0 \times 10^6$ cells/mL, in the same manner as above. After centrifugation, the supernatant was removed. The cells were subjected to a freeze-thaw process repeatedly three times and suspended again in PBS to obtain Necrotic cell death (NCD) cells.

The cells ($5.0 \times 10^6$ cells) of each group mentioned above were subcutaneously inoculated into the right axilla of each of female 6 week-old BALB/c mice (BALB/c AnNCrlCrlj) (bred by CHARLES RIVER LABORATORIES JAPAN, INC.) (inoculation, Day 0). An anti-SIRPα antibody (clone: 5C12) or an anti-CD47 antibody (clone: MIAP410, manufactured by Bio X Cell, Inc.) was intraperitoneally administered at a dose of 10 mg/kg on Day 1 and 5 (two times in total). As a control, the same amount of PBS was administered. Seven days later, CT26.WT cells ($3.0 \times 10^6$ cells) not treated with a drug were subcutaneously inoculated into the left axilla of each of the mice (rechallenge, Day 0). On Day 14, tumor volumes were measured to evaluate vaccination effect. Note that the number of mice per group was 6. The results of the NCD-PBS group and individual groups were compared in accordance with the Dunnett's test, P value was expressed to four decimal places. The results satisfying P < 0.05 (two-sided test) were determined as being significant.

Figure 27A shows an overview of the evaluation of in vivo ICD induction. In the graph of Figure 27B, the vertical axis represents tumor volume ($mm^3$) and the horizontal axis represents the names of the individual groups. MTX treatment groups were positive controls and the NCD treatment groups were negative controls. Figure 27C shows general information of the individual treatment groups and the number of CR individuals. As shown in Figure 27B, in each of the NCD groups into which CT26.WT cells were re-challenged, tumor growth was observed. On the other hand, in a MTX-PBS administration group to which CT26.WT cells were re-challenged, engraftment of a tumor was rejected in 2/6 cases (CR). In a MTX-anti-SIRPα antibody administration group, the number of CR individuals significantly increased up to 4/6 (P = 0.0486), compared to NCD. In a compound (A)-PBS group into which CT26.WT cells were re-challenged, 5/6 cases showed CR (P = 0.0362), compared to NCD. In a compound (A)-anti-SIRPα antibody administration group or an anti-CD47 antibody administration group, all cases show CR (both P = 0.0325). The number of CR individuals significantly increased.

As described above, HMGB1, which serves as an index of ICD, was released from CT26.WT cells by treating the cells with compound (A) or MTX. Further, it was demonstrated that a vaccination effect (immunological memory of a tumor is formed) is produced by inoculating cells, which were induced into ICD by these drugs, into a mouse, and that the vaccination effect is enhanced by administration of the anti-SIRPα antibody or the anti-CD47 antibody.

Example 4: ELISPOT analysis

The number of CT26.WT-reactive T cells in mouse spleen cells was determined by Murine IFNy Single-Color Enzymatic ELISPOT Assay (manufactured by CELLULAR TECHNOLOGY LIMITED), more specifically, counting of the number of IFNy spots produced from individual spleen cells. From each of the mice used in Example 3, the spleen was harvested and the number of spleen cells was adjusted to be $1.0 \times 10^6$ cells/mL by use of CTL test medium. CT26.WT cells were treated with 10 μg/mL mitomycin C for 2 hours and washed. Thereafter, the cells were collected, adjusted to be $1.0 \times 10^6$ cells/mL by CTL test medium, and used as an antigen. The spleen cells and the antigen were added each in an amount of 100 μL/well in a PVDF-membrane plate pre-coated with an anti-IFNy antibody and co-cultured at 37°C for 24 hours. Thereafter, the color was developed using the attached antibody and coloring reagent and the number of IFNy-producing spleen cells was determined. Comparison between the NCD cell inoculation group and compound (A) or MTX treatment group was made in accordance with the Wilcoxon rank sum test. P value was expressed to four decimal places and the results satisfying P < 0.05 (two-sided test) were determined as being significant.

The results are shown in Figure 28. In the spleen cells of a mouse of [compound (A) _PBS], which is a "compound (A) treated CT26.WT cells inoculated-PBS administration group", the number of IFNy-producing spleen cells showed a tendency to increase, compared to that of the NCD cell inoculated-PBS administration group [NCD_PBS]. In the spleen cells of a compound (A)_anti-SIRPα antibody mouse, the number of IFNy-producing spleen cells significantly increased (P = 0.0043), compared to that of the spleen cells of the NCD_PBS administration group. When the compound (A) _PBS

was compared to the compound (A)_αSIRPα group, the number of IFNy-producing spleen cells significantly increased (P = 0.013).

From the results, in the mice treated with compound (A), it was suggested that T cells recognizing a CT26.WT cell-derived antigen were induced. The number of T cells was significantly increased by administration with an anti-SIRPα antibody. The antigen specific T cell induction showed a tendency to further enhance in the compound (A) treatment group compared to the MTX treatment group.

Example 5: FCM analysis of spleen cells

From each of the mice used in Example 3, the spleen was excised out and spleen cells were prepared by use of PBS and seeded in a 96-well (round bottom) plate at $1 \times 10^6$ cells/well. After centrifugation was carried out at 1200 rpm for 3 minutes, the supernatant was removed. Human FcyR Blocker (manufactured by Biolegend, Inc.) diluted to 1/20 with PBS was added up to 100 μL/well. After standing at room temperature for 30 minutes, centrifugation was carried out at 1200 rpm for 3 minutes. The supernatant was removed, and primary-antibody solutions (FITC anti-CD3ε antibody, PerCP anti-CD4 antibody, PE/Cy7 anti-CD8α antibody, APC anti-CD62L antibody, and APC/Cy7 anti-CD44 antibody: all manufactured by Biolegend, Inc.) were diluted with FACS buffer (1 mM EDTA, 5% FBS) and added in an amount of 50 μL/well. After standing in a dark place at 4°C for 25 minutes, centrifugation was carried out at 1200 rpm for 3 minutes, and then the supernatant was removed. Washing was carried out twice with FACS buffer (200 μL/wells). After centrifugation, the supernatant was removed. A suspension was made with Stabilizing Fixative (manufactured by Becton, Dickinson and Company) and allowed to stand at room temperature for 10 minutes. Measurement was carried out by flow cytometry (FACS CantoII: manufactured by Becton, Dickinson and Company). Data were analyzed by use of FlowJo (manufactured by TreeStar Inc). Comparison between the NCD group and each of the drug treatment groups, or comparison among the PBS administration group, the anti-SIRPα antibody group and an anti-CD47 antibody group in each of the cell treatment groups was made in accordance with the Dunnett's multiple comparison. P value was expressed to four decimal places and the results satisfying P < 0.05 (two-sided test) were determined as being significant.

Note that individual populations in the CD4-positive T cells and CD8-positive T cells were identified in the following conditions.

Tcm: central memory T cells [CD44(+) CD62L(+)]

The results are shown in Figures 29 and 30. As shown in Figure 29A, the ratio occupied by Tcm in CD4-positive T cells in the MTX treatment group (P = 0.0005) or compound (A) treatment group (P < 0.0001) significantly increased compared to that in the NCD treatment group.

As shown in Figure 29B, the ratio occupied by Tcm in the CD8-positive T cells in the MTX treatment group (P = 0.0004) or compound (A) treatment group (P < 0.0001) significantly increased compared to that in the NCD treatment group.

As shown in Figure 30A, the ratio occupied by Tcm in the CD4-positive T cells showed a tendency to increase in the MTX_anti-SIRPα antibody group or MTX_anti-CD47 antibody group, compared to the MTX_PBS group. The ratio in the compound (A)_anti-SIRPα antibody group showed a tendency to increase; whereas the ratio in the compound (A)_anti-CD47 antibody group significantly decreased (P = 0.0002), compared to the compound (A)_PBS group.

As shown in Figure 30B, the ratio occupied by Tcm in the CD8-positive T cells significantly increased in the MTX_anti-SIRPα antibody group and showed a tendency to increase (P = 0.035) in the MTX_anti-CD47 antibody group, compared to that of MTX_PBS group. The ratio in the compound (A)_anti-SIRPα antibody group shows substantially the same value; whereas the ratio in the compound (A)_anti-CD47 antibody group significantly decreased (P = 0.0017), compared to the compound (A)_PBS group.

As shown in Figure 30C, the ratio occupied by CD8-positive T cells in the whole cells significantly increased in the MTX treated-anti-SIRPα antibody administration group (P = 0.015); whereas the ratio in the compound (A)_anti-SIRPα antibody administration group showed a tendency to increase. The ratio occupied by CD4-positive T cells in the whole cells showed a tendency to increase in the MTX treated_anti-SIRPα antibody administration group; whereas the ratio significantly increased in the compound (A) treated_anti-SIRPα antibody administration group (P = 0.029).

When naive CD4-positive T cells/CD8-positive T cells recognize cancer cells in an antigen specific manner, the T cells are activated to differentiate into CD4-positive Teff cells, which serve as a control tower of a wide variety of immune responses, or CD8-positive Teff cells, which have a cytotoxic effect on target cells. The Tcm cells are CD4/CD8-positive T cells, in which Teff cells partly maintain an ability to respond to target cells and acquire an ability to survive for a long time. These cells retain memory of an antigen once encountered and quickly and effectively induce an immune response when these are exposed to the same antigen.

From the results, it was demonstrated that the number of long survival CD4/CD8-positive Tcm cells significantly increases by treatment with MTX or compound (A) (Figure 29); and that a T cell population that can maintain in vivo antitumor immunity for a long time can be induced.

These effects can be enhanced by administration of the anti-SIRPα antibody. From this, it was demonstrated

that the anti-SIRPα antibody is advantageous for inducing an antitumor immunity, if used in combination with compound (A).

From the results of Examples 3, 4 and 5, it was demonstrated that compound (A) used in the present invention acts on tumor cells to promote production of, e.g., HMGB1 and ATP, induces ICD and following expression of CRT on cell surfaces, and accelerates immunological memory formation in vivo by ICD molecule induction. It was also demonstrated that these effects are enhanced by administration of the anti-CD47 antibody and the anti-SIRPα antibody, and remarkably enhanced particularly by administration of the anti-SIRPα antibody. From this, it was demonstrated that the anti-SIRPα antibody is advantageous for inducing an antitumor immunity, if used in combination with compound (A).

Example 6: ADCP activity of antibody-drug conjugate of compound (A) and the anti-human SIRPα antibody to cancer cell line

6-1 Preparation of target cells

CD47, HER2 and TROP2 positive human gastric cancer cell line AGS cells were collected, washed twice with PBS and resuspended in PBS. The number of living cells was counted in accordance with the trypan blue pigment exclusion test. A CellTrace Far Red (manufactured by Thermo Fisher Scientific Inc.) solution was added in a volume of 1 μL per 1 × $10^6$ cells/mL. The mixture was allowed to stand at room temperature for 10 minutes. To this, 20 mL of RPMI1640 culture medium containing 10% FBS (R10/manufactured by Thermo Fisher Scientific Inc.) was added. The mixture was allowed to stand for 5 minutes. Then, 20 ml of R10 was added and washing was carried out twice. The cells were resuspended so as to reach 1 × $10^6$ cells/mL and used as target cells.

6-2 Preparation of effector cells

To SepMate-50 tubes (manufactured by STEMCELL Technologies Inc.), Ficoll-Paque PLUS (manufactured by GE) was dispensed in an amount of 15 mL/tube. The whole blood diluted 2-fold with PBS containing 2% FBS was overlaid in an amount of 17 mL/tube. Centrifugation was carried out at room temperature at 1200 g for 10 minutes. The supernatant containing PBMC was collected in fresh 50 mL tubes by decantation and centrifugation was carried out at room temperature at 1200 rpm for 8 minutes. After the supernatant was removed, 25 mL of PBS containing 2% FBS was added. Centrifugation was carried out at room temperature and 300 g for 8 minutes to perform washing. Suspending with 1 mL of Robosep buffer (STEMCELL Technologies Inc.) was carried out, and then the number of PBMC living cells was counted in accordance with the trypan blue pigment exclusion test. Preparation was carried out with RoboSep buffer (manufactured by STEMCELL Technologies Inc.) so as to reach 5 × $10^7$ cells/mL and EasySep human Monocyte enrichment cocktail provided in Human monocyte Enrichment Kit Without CD16 Depletion (manufactured by STEMCELL Technologies Inc.) was added in an amount of 50 μL per mL of the PBMC cell suspension. After reaction at 4°C for 10 minutes, EasySep Magnetic Particles were added to the PBMC cell suspension in an amount of 50 μL per mL. After reaction at 4°C for 5 minutes, RoboSep buffer (manufactured by STEMCELL Technologies Inc.) was added up to 2.5 mL before setting on an EasySep Magnet. Two minutes and 30 seconds later, the supernatant was collected. Centrifugation was carried out at 1200 rpm × 5 minutes, and then, monocyte fractions were collected. R10 was added; and washing was carried out once; R10 containing 20 ng/mL M-CSF (manufactured by PEPROTEC, Inc.) was added. Seeding was carried out in a floating 225 $cm^2$ flask (manufactured by Sumitomo Bakelite Co., Ltd.). Culturing was carried out in the conditions of 37°C and 5% $CO_2$ for 11 days. The culture supernatant was removed, R10 containing 20 ng/mL IL-10 and 20 ng/mL M-CSF (manufactured by PEPROTEC, Inc.) was added. Culturing was carried out for a further two days. Thirteen days later, TrypLE Express (manufactured by Life Technology) was added to macrophages induced by differentiation. A reaction was carried out at 37°C for 15 minutes, the macrophages were removed. R10 (25 mL) was added and collection was carried out. Washing was carried out twice with PBS and resuspension was carried out with PBS so as to reach 1 × $10^6$ cells/mL. As a standard solution, 1 μL/$10^6$ cells/mL CFSE solution (manufactured by Thermo Fisher Scientific Inc.) was added before allowing to stand at room temperature for 10 minutes. R10 (20 ml) was added and washing was carried out twice. The cells were resuspended so as to reach 1 × $10^6$ cells/mL and used as effector cells.

6-3 Evaluation of ADCP activity

The target cells (50 μL/well) prepared in accordance with the method of step 6-1 were added to a 96-well (U-bottom) microplate (manufactured by Sumitomo Bakelite Co., Ltd.) having an ultra-low adhesive surface. To the plate, an antibody-drug conjugate (1) or (2), a humanized anti-HER2 (1) antibody or a humanized anti-TROP2 antibody (1), or each of different control antibodies diluted with R10 was added so as to reach a final concentration of 0 to 1000 ng/mL, in an amount of 50 μL/well. In single treatment groups, R10 was added in an amount of 50 μL/well; whereas in combined administration groups, a humanized anti-SIRPα antibody (clone: hD13_H1L3) diluted with R10 (as final concentration

of 2000 ng/mL) was added in an amount of 50 μL/well. In another experiment, an antibody-drug conjugate (1) or (2), an anti-HER2 antibody or an anti-TROP2 antibody, and each of different control antibodies diluted with R10 was added so as to reach a final concentration of 1000 ng/mL, in an amount of 50 μL/well. In the single administration groups, R10 was added in an amount of 50 μL/well; whereas in the combined administration group, an anti-SIRPα antibody diluted with R10 (as final concentration of 0 to 2000 ng/mL) was added in an amount of 50 μL/well. To the wells, effector cells ($1 \times 10^6$ cells/mL) prepared in step 6-2 were added in an amount of 50 μL/well before allowing to stand in the conditions of 37°C and 5% $CO_2$ for 4 hours. After centrifugation at 4°C and 1200 rpm for 5 minutes, the supernatant was removed. The cells were washed with FACS buffer (200 μL/well) and suspended with 50 μL/well 1 × BD Stabilizing Fixative (manufactured by Becton, Dickinson and Company) before allowing to stand at room temperature for 10 minutes. Measurement was carried out by flow cytometry (AttuneNxT: manufactured by Thermo Fisher Scientific Inc.). Data were analyzed by use of FlowJo (manufactured by TreeStar Inc.). After development by FSC (forward scattered light)/SSC (laterally scattered light), the numbers of APC-positive cells (A) and APC- and FITC-co-positive cells (B) were calculated. APC- and FITC-co-positive cells (B) were determined as the target cells engulfed by the effector cells. The ratio of the cells engulfed by phagocytosis due to ADCP activity was calculated in accordance with the following formula:

$$\text{Ratio of cells engulfed (\%)} = B/(A+B) \times 100$$

As shown in Figure 31A-D, the antibody-drug conjugate (1) or (2) showed ADCP activity to CD47, HER2 and TROP2 positive human gastric cancer cell line AGS cells in an antibody-(added thereto) concentration dependent manner (Figure 31A, B) and showed a higher ADCP activity when the humanized anti-SIRPα antibody hD13_H1L3 was used in combination (Figure 31A, B). Since these effects were not observed in Control_antibody-drug conjugate, both were demonstrated as effects induced in a target-specific manner. Note that these exerted the ADCP activity at the same level or more as that of a parent antibody, i.e. the humanized anti-HER2 antibody (1) or humanized anti-TROP2 antibody (1), which was not conjugated with a drug. From this, it was suggested that an adverse effect is not produced by conjugation with a drug. On the other hand, as shown in Figure 31C and D, when the concentration of the humanized anti-SIRPα antibody hD13_H1L3 was examined while the concentration of the antibody-drug conjugate (1) or (2) was maintained constant, the ADCP activity increased in an anti-SIRPα antibody concentration dependent manner.

Example 7: Antitumor study (1)

Mouse: Female 6-week-old BALB/c mice (BALB/c AnNCrlCrlj) (bred by CHARLES RIVER LABORATORIES JAPAN, INC.) were subjected to experiments.

Measurement and calculation formula: the long diameter and short diameter of tumors were measured twice a week with an electronic digital caliper (CD15-CX, manufactured by Mitutoyo Corporation), and the tumor volumes ($mm^3$) were calculated. The calculation formula is shown below:

$$\text{Tumor volume (mm}^3\text{)} = 0.5 \times \text{Long diameter (mm)} \times [\text{Short diameter (mm)}]^2$$

Individuals having a tumor volume of beyond 3000 $mm^3$ were humanly killed from an ethical point of view for animal experiments.

The antibody-drug conjugate (1) (Drug-to-Antibody Ratio: 7.6) was diluted with PBS, and a dose of 10 mL/kg was intravenously administered to the tail vein. An anti-mouse SIRPα antibody (clone: 5C12) and an anti-PD-L1 antibody (clone: YW243.55S70, prepared in accordance with US Patent Publication, US 2013/0045201 A1) were diluted with PBS, and a dose of 10 mL/kg was intraperitoneally administered.

To mouse breast cancer cell line 4T1 (CRL-2539) purchased from the American Type Culture Collection, a human/mouse chimera HER2 gene was transfected by use of a lentivirus vector to prepare 4T1-hmHER2 cells, which were put in use. The cells express a human/mouse chimera HER2 protein on the cell membrane. Then, 4T1-hmHER2 cells were suspended in PBS and $1.0 \times 10^6$ cells were subcutaneously inoculated into the right axilla of each of the BALB/c mice (Day 0). Four days later, the mice were randomly grouped (Day 4). The antibody-drug conjugate (1) was intravenously administered at a dose of 10 mg/kg on Day 4 and 11 (two times in total) to the tail vein. The anti-SIRPα antibody (clone: 5C12) or the anti-PD-L1 antibody (clone: YW243.55S70) was intraperitoneally administered at a dose of 10 mg/kg on Day 5, 8 and 12 (three times in total). A combined administration group of the antibody-drug conjugate

(1), the anti-SIRPα antibody and the anti-PD-L1 antibody was set up; and a PBS administration group was set up as a control group. The number of mice per group was 5. Measurement of the tumor volume was continued until Day 21. Comparison among the control group, each of the single administration groups, and the combined administration groups, or comparison between the antibody-drug conjugate (1) group and each of the combined administration groups was carried out in accordance with the Dunnett's multiple comparison. P value was expressed to four decimal places and the results satisfying $P < 0.05$ (two-sided test) were determined as being significant.

The results are shown in Figure 32. Figure 32A shows the overview of an antitumor study. Figure 32B shows tumor growth curves of individual administration groups. The vertical axis represents tumor volume ($mm^3$) and the horizontal axis represents the number of days after tumor inoculation. Figure 32C shows general data of the treatment groups, the tumor growth inhibition (TGI/%) and the number of CR individuals. On Day 21, the antibody-drug conjugate (1) group ($P < 0.0001$) and the anti-SIRPα antibody group ($P = 0.0087$) showed a significantly stronger antitumor effect compared to the control group. The antibody-drug conjugate (1)-anti-SIRPα antibody combined group or the three-drug combined group showed a significantly stronger antitumor effect (both $p < 0.0001$) compared to the control group. The antibody-drug conjugate (1)-anti-SIRPα antibody combined group ($P = 0.0497$) and the three-drug combined group ($P = 0.0237$) showed a significantly stronger antitumor effect, compared to the antibody-drug conjugate (1) group. In all groups of the experiment, no weight loss of mice was observed. From the above, it was confirmed that an antitumor effect is produced by single administration of each of the antibody-drug conjugate (1) or the anti-SIRPα antibody, and enhanced significantly by the combined administration of these.

Example 8: Antitumor study (2)

Mouse: Female 6-week-old BALB/c mice (BALB/c AnNCrlCrlj) (bred by CHARLES RIVER LABORATORIES JAPAN, INC.) were subjected to experiments.

Measurement and calculation formula: the long diameter and short diameter of tumors were measured twice a week with an electronic digital caliper (CD15-CX, manufactured by Mitutoyo Corporation), and the tumor volumes ($mm^3$) were calculated. The calculation formula is shown below:

$$\text{Tumor volume (mm}^3\text{)} = 0.5 \times \text{Long diameter (mm)} \times [\text{Short diameter (mm)}]^2$$

Individuals having a tumor volume of beyond 3000 $mm^3$ were humanely killed from an ethical point of view for animal experiments.

The antibody-drug conjugate (2) (Drug-to-Antibody Ratio: 4) was diluted with PBS, and a dose of 10 mL/kg was intravenously administered to the tail vein. An anti-SIRPα antibody (clone: 5C12) was diluted with PBS, and a dose of 10 mL/kg was intraperitoneally administered.

To mouse colorectal cancer cell line CT26.WT (CRL2638) purchased from the American Type Culture Collection, a human TROP2 gene was tranfected by use of a lentivirus vector to prepare CT26.WT-hTROP2 cells, which were put in use. The cells express a human TROP2 protein on the cell membrane. CT26.WT-hTROP2 cells were suspended in saline, and $2.0 \times 10^6$ cells were subcutaneously inoculated into the right axilla of each of the BALB/c mice (Day 0). Seven days later, the mice were randomly grouped (Day 7). The antibody-drug conjugate (2) was intravenously administered at a dose of 10 mg/kg on Day 7 and 12 (two times in total) to the tail vein. The anti-SIRPα antibody (clone: 5C12) was intraperitoneally administered at a dose of 10 mg/kg on Day 8, 11 and 13 (three times in total). A combined administration group of the antibody-drug conjugate (2) and the anti-SIRPα antibody was set up and a PBS administration group was set up as a control group. The number of mice per group was 6. Measurement of the tumor volume was continued until Day 18. Comparison among the control group, each of the single administration groups and the combined administration groups was made in accordance with the Dunnett's multiple comparison. P value was expressed to four decimal places and the results satisfying $P < 0.05$ (two-sided test) were determined as being significant.

The results are shown in Figure 33. Figure 33A shows the overview of an antitumor study. Figure 33B shows tumor growth curves of individual administration groups. The vertical axis represents tumor volume ($mm^3$) and the horizontal axis represents the number of days after tumor inoculation. Figure 33C shows general data of the treatment groups and the tumor growth inhibition (TGI/%). The vertical axis represents tumor volume ($mm^3$) and the horizontal axis represents the number of days from the first day of tumor inoculation. On Day 18, the antibody-drug conjugate (2) group and the anti-SIRPα antibody group showed a partial antitumor effect compared to the control group. The combined administration group showed a significantly stronger antitumor effect ($P = 0.0007$). In all groups of the experiment, no weight loss of mice was observed. From the above, it was confirmed that an antitumor effect is produced by single

administration of each of the drugs, and enhanced by the combined administration of these.

Example 9: Antitumor study (3)

Mouse: Female 6-week-old BALB/c mice (BALB/c AnNCrlCrlj) (bred by CHARLES RIVER LABORATORIES JAPAN, INC.) were subjected to experiments.

Measurement and calculation formula: the long diameter and short diameter of tumors were measured twice a week with an electronic digital caliper (CD15-CX, manufactured by Mitutoyo Corporation), and the tumor volumes ($mm^3$) were calculated. The calculation formula is shown below:

$$\text{Tumor volume } (mm^3) = 0.5 \times \text{Long diameter (mm)} \times [\text{Short diameter (mm)}]^2$$

Individuals having a tumor volume of beyond 3000 $mm^3$ were humanely killed from an ethical point of view for animal experiments.

The antibody-drug conjugate (3) (Drug-to-Antibody Ratio: 8) and an anti-SIRPα antibody (clone: 5C12) were diluted with PBS, and used for administration.

To mouse colorectal cancer cell line CT26.WT (CRL2638) purchased from the American Type Culture Collection, a human HER3 gene was transfected by use of a lentivirus vector to prepare CT26.WT-HER3 cells, which were put in use. The cells express a human HER3 protein on the cell membrane. CT26.WT-HER3 cells were suspended in saline, and $2.0 \times 10^6$ cells were subcutaneously inoculated into the right axilla of each of the BALB/c mice (Day 0). Seven days later, the mice were randomly grouped (Day 7). The antibody-drug conjugate (3) was intravenously administered at a dose of 10 mg/kg on Day 7 and 14 (two times in total) via the tail vein. The anti-SIRPα antibody (clone: 5C12) was intraperitoneally administered at a dose of 10 mg/kg on Day 8, 11 and 15 (three times in total). A combined administration group of the antibody-drug conjugate (3) and the anti-SIRPα antibody was set up and a PBS administration group was set up as a control group. The number of mice per group was 6. Measurement of the tumor volume was continued until Day 18. Comparison among the control group, each of the single administration groups and the combined administration groups was made in accordance with the Dunnett's multiple comparison. P value was expressed to four decimal places and the results satisfying $P < 0.05$ (two-sided test) were determined as being significant.

The results are shown in Figure 34. Figure 34A shows the overview of an antitumor study. Figure 34B shows tumor growth curves of individual administration groups. The vertical axis represents tumor volume ($mm^3$) and the horizontal axis represents the number of days after tumor inoculation. Figure 34C shows general data of the treatment groups and the tumor growth inhibition (TGI/%). On Day 18, the anti-SIRPα antibody single-agent group showed no therapeutic effect and the antibody-drug conjugate (3) group showed a partial antitumor effect compared to the control group. In addition, the combined administration group showed a significantly stronger antitumor effect ($P = 0.046$) compared to the anti-SIRPα antibody single-agent group, and exhibited an enhanced trend in antitumor efficacy compared to the antibody-drug conjugate (3) group. In all groups of the experiment, no weight loss of mice was observed.

[Industrial Applicability]

From the above experimental results, it was found that the antibody-drug conjugate according to the present invention shows an excellent antitumor effect if administered in combination with the anti-SIRPα antibody.

[Free Text of Sequence Listing]

SEQ ID NO: 1 - Amino acid sequence of a heavy chain of the anti-HER2 antibody
SEQ ID NO: 2 - Amino acid sequence of a light chain of the anti-HER2 antibody
SEQ ID NO: 3 - Amino acid sequence of a heavy chain of the anti-HER3 antibody
SEQ ID NO: 4 - Amino acid sequence of a light chain of the anti-HER3 antibody
SEQ ID NO: 5 - Amino acid sequence of a heavy chain of the anti-TROP2 antibody
SEQ ID NO: 6 - Amino acid sequence of a light chain of the anti-TROP2 antibody
SEQ ID NO: 7 - Amino acid sequence of a heavy chain of the anti-B7-H3 antibody
SEQ ID NO: 8 - Amino acid sequence of a light chain of the anti-B7-H3 antibody

SEQ ID NO: 9 - Amino acid sequence of a heavy chain of the anti-GPR20 antibody
SEQ ID NO: 10 - Amino acid sequence of a light chain of the anti-GPR20 antibody
SEQ ID NO: 11 - Amino acid sequence of a heavy chain of the anti-CDH6 antibody
SEQ ID NO: 12 - Amino acid sequence of a light chain of the anti-CDH6 antibody
SEQ ID NO: 13 - Amino acid sequence of hH1 heavy chain of the humanized anti-SIRPα antibody hD13
SEQ ID NO: 14 - Amino acid sequence of hH2 heavy chain of the humanized anti-SIRPα antibody hD13
SEQ ID NO: 15 - Amino acid sequence of hL2 light chain of the humanized anti-SIRPα antibody hD13
SEQ ID NO: 16 - Amino acid sequence of hL3 light chain of the humanized anti-SIRPα antibody hD13
SEQ ID NO: 17 - Amino acid sequence of hL4 light chain of the humanized anti-SIRPα antibody hD13
SEQ ID NO: 18 - Amino acid sequence of the chimera SIRPα antibody cD13 CDR-H1
SEQ ID NO: 19 - Amino acid sequence of the chimera SIRPα antibody cD13 CDR-H2
SEQ ID NO: 20 - Amino acid sequence of the chimera SIRPα antibody cD13 CDR-H3
SEQ ID NO: 21 - Amino acid sequence of the chimera SIRPα antibody cD13 CDR-L1
SEQ ID NO: 22- Amino acid sequence of the chimera SIRPα antibody cD13 CDR-L2
SEQ ID NO: 23 - Amino acid sequence of the chimera SIRPα antibody cD13 CDR-L3
SEQ ID NO: 24 - Amino acid sequence of a heavy chain of the OSE-172 antibody (OSE-172_hG4Pro)
SEQ ID NO: 25 - Amino acid sequence of a light chain of the OSE-172 antibody (OSE-172_hK)
SEQ ID NO: 26 - Amino acid sequence of a heavy chain of the KWAR23 antibody (KWAR23_hG4Pro)
SEQ ID NO: 27 - Amino acid sequence of a light chain of the KWAR23 antibody (KWAR23_hK)
SEQ ID NO: 28 - Amino acid sequence of a heavy chain of the ADU-1805 antibody (ADU-1805_hG2)
SEQ ID NO: 29 - Amino acid sequence of a light chain of the ADU-1805 antibody (ADU-1805_hK)
SEQ ID NO: 30 - Amino acid sequence of a heavy chain of the 5C12 anti-mouse SIRPα antibody
SEQ ID NO: 31 - Amino acid sequence of a light chain of the 5C12 anti-mouse SIRPα antibody
SEQ ID NO: 32 - Amino acid sequence of a heavy chain of the YW243.55S70 anti-mouse human anti-PD-L1 antibody
SEQ ID NO: 33 - Amino acid sequence of a light chain of the YW243.55S70 anti-mouse human anti-PD-L1 antibody

## Claims

**1.** A pharmaceutical composition, wherein

an antibody-drug conjugate and an anti-SIRPα antibody are administered in combination, and
the antibody-drug conjugate is an antibody-drug conjugate in which a drug-linker represented by the following formula:

[Formula 1]

wherein A represents the connecting position to an antibody,
is conjugated to the antibody via a thioether bond.

**2.** The pharmaceutical composition according to claim 1, wherein the antibody in the antibody-drug conjugate is an

anti-HER2 antibody, an anti-HER3 antibody, an anti-TROP2 antibody, an anti-B7-H3 antibody, an anti-GPR20 antibody, or an anti-CDH6 antibody.

**3.** The pharmaceutical composition according to claim 2, wherein the antibody in the antibody-drug conjugate is an anti-HER2 antibody.

**4.** The pharmaceutical composition according to claim 3, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**5.** The pharmaceutical composition according to claim 3, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**6.** The pharmaceutical composition according to any one of claims 3 to 5, wherein the average number of units of the drug-linker conjugated per antibody molecule in the antibody-drug conjugate is in the range of from 7 to 8.

**7.** The pharmaceutical composition according to claim 2, wherein the antibody in the antibody-drug conjugate is an anti-HER3 antibody.

**8.** The pharmaceutical composition according to claim 7, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**9.** The pharmaceutical composition according to claim 8, wherein the anti-HER3 antibody lacks a lysine residue at the carboxyl terminus of the heavy chain.

**10.** The pharmaceutical composition according to any one of claims 7 to 9, wherein the average number of units of the drug-linker conjugated per antibody molecule in the antibody-drug conjugate is in the range of from 7 to 8.

**11.** The pharmaceutical composition according to claim 2, wherein the antibody in the antibody-drug conjugate is an anti-TROP2 antibody.

**12.** The pharmaceutical composition according to claim 11, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**13.** The pharmaceutical composition according to claim 12, wherein the anti-TROP2 antibody lacks a lysine residue at the carboxyl terminus of the heavy chain.

**14.** The pharmaceutical composition according to any one of claims 11 to 13, wherein the average number of units of the drug-linker conjugated per antibody molecule in the antibody-drug conjugate is in the range of from 3.5 to 4.5.

**15.** The pharmaceutical composition according to claim 2, wherein the antibody in the antibody-drug conjugate is an anti-B7-H3 antibody.

**16.** The pharmaceutical composition according to claim 15, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**17.** The pharmaceutical composition according to claim 16, wherein the anti-B7-H3 antibody lacks a lysine residue at the carboxyl terminus of the heavy chain.

**18.** The pharmaceutical composition according to any one of claims 15 to 17, wherein the average number of units of the drug-linker conjugated per antibody molecule in the antibody-drug conjugate is in the range of from 3.5 to 4.5.

**19.** The pharmaceutical composition according to claim 2, wherein the antibody in the antibody-drug conjugate is an anti-GPR20 antibody.

**20.** The pharmaceutical composition according to claim 19, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

**21.** The pharmaceutical composition according to claim 20, wherein the anti-GPR20 antibody lacks a lysine residue at the carboxyl terminus of the heavy chain.

**22.** The pharmaceutical composition according to any one of claims 19 to 21, wherein the average number of units of the drug-linker conjugated per antibody molecule in the antibody-drug conjugate is in the range of from 7 to 8.

**23.** The pharmaceutical composition according to claim 2, wherein the antibody in the antibody-drug conjugate is an anti-CDH6 antibody.

**24.** The pharmaceutical composition according to claim 23, wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

**25.** The pharmaceutical composition according to claim 24, wherein the anti-CDH6 antibody lacks a lysine residue at the carboxyl terminus of the heavy chain.

**26.** The pharmaceutical composition according to any one of claims 23 to 25, wherein the average number of units of the drug-linker conjugated per antibody molecule in the antibody-drug conjugate is in the range of from 7 to 8.

**27.** The pharmaceutical composition according to any one of claims 1 to 26, wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

**28.** The pharmaceutical composition according to any one of claims 1 to 27, wherein the antibody-drug conjugate and the anti-SIRPα antibody are separately contained as active components in different formulations, and are administered simultaneously or at different times.

**29.** The pharmaceutical composition according to any one of claims 1 to 28, wherein the composition is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine cancer, sarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

**30.** A pharmaceutical composition, wherein an antibody-drug conjugate and an anti-SIRPα antibody are administered in combination, and
the antibody-drug conjugate is an antibody-drug conjugate represented by the following formula:

[Formula 2]

wherein a drug-linker is conjugated to an antibody via a thioether bond; and n represents the average number of units of the drug-linker conjugated per antibody molecule.

**31.** The pharmaceutical composition according to claim 30, wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

**32.** The pharmaceutical composition according to claim 31, wherein the antibody in the antibody-drug conjugate is an anti-HER2 antibody.

**33.** The pharmaceutical composition according to claim 32, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**34.** The pharmaceutical composition according to claim 32, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**35.** The pharmaceutical composition according to any one of claims 32 to 34, wherein the average number of units of the drug-linker conjugated per antibody molecule in the antibody-drug conjugate is in the range of from 7 to 8.

**36.** The pharmaceutical composition according to claim 31, wherein the antibody in the antibody-drug conjugate is an anti-HER3 antibody.

**37.** The pharmaceutical composition according to claim 36, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**38.** The pharmaceutical composition according to claim 37, wherein the anti-HER3 antibody lacks a lysine residue at the carboxyl terminus of the heavy chain.

**39.** The pharmaceutical composition according to any one of claims 36 to 38, wherein the average number of units of the drug-linker conjugated per antibody molecule in the antibody-drug conjugate is in the range of from 7 to 8.

**40.** The pharmaceutical composition according to claim 31, wherein the antibody in the antibody-drug conjugate is an anti-TROP2 antibody.

**41.** The pharmaceutical composition according to claim 40, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**42.** The pharmaceutical composition according to claim 41, wherein the anti-TROP2 antibody lacks a lysine residue at the carboxyl terminus of the heavy chain.

**43.** The pharmaceutical composition according to any one of claims 40 to 42, wherein the average number of units of the drug-linker conjugated per antibody molecule in the antibody-drug conjugate is in the range of from 3.5 to 4.5.

**44.** The pharmaceutical composition according to claim 31, wherein the antibody in the antibody-drug conjugate is an anti-B7-H3 antibody.

**45.** The pharmaceutical composition according to claim 44, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**46.** The pharmaceutical composition according to claim 45, wherein the anti-B7-H3 antibody lacks a lysine residue at the carboxyl terminus of the heavy chain.

**47.** The pharmaceutical composition according to any one of claims 44 to 46, wherein the average number of units of the drug-linker conjugated per antibody molecule in the antibody-drug conjugate is in the range of from 3.5 to 4.5

**48.** The pharmaceutical composition according to claim 31, wherein the antibody in the antibody-drug conjugate is an anti-GPR20 antibody

**49.** The pharmaceutical composition according to claim 48, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

**50.** The pharmaceutical composition according to claim 49, wherein the anti-GPR20 antibody lacks a lysine residue at the carboxyl terminus of the heavy chain.

**51.** The pharmaceutical composition according to any one of claims 48 to 50, wherein the average number of units of the drug-linker conjugated per antibody molecule in the antibody-drug conjugate is in the range of from 7 to 8.

**52.** The pharmaceutical composition according to claim 31, wherein the antibody in the antibody-drug conjugate is an anti-CDH6 antibody.

**53.** The pharmaceutical composition according to claim 52, wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

**54.** The pharmaceutical composition according to claim 53, wherein the anti-CDH6 antibody lacks a lysine residue at the carboxyl terminus of the heavy chain.

**55.** The pharmaceutical composition according to any one of claims 52 to 54, wherein the average number of units of the drug-linker conjugated per antibody molecule in the antibody-drug conjugate is in the range of from 7 to 8.

**56.** The pharmaceutical composition according to any one of claims 30 to 55, wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues

20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

**57.** The pharmaceutical composition according to any one of claims 30 to 56, wherein the antibody-drug conjugate and the anti-SIRPα antibody are separately contained as active components in different formulations, and are administered simultaneously or at different times.

**58.** The pharmaceutical composition according to any one of claims 30 to 57, wherein the composition is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine cancer, sarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

**59.** A method of treatment, comprising administering an antibody-drug conjugate and an anti-SIRPα antibody in combination to a subject in need of treatment, wherein the antibody-drug conjugate is an antibody-drug conjugate in which a drug-linker represented by the following formula:

[Formula 3]

wherein A represents the connecting position to an antibody,
is conjugated to the antibody via a thioether bond.

**60.** The method of treatment according to claim 59, wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

**61.** The method of treatment according to claim 60, wherein the antibody in the antibody-drug conjugate is an anti-HER2 antibody.

**62.** The method of treatment according to claim 61, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**63.** The method of treatment according to claim 61, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**64.** The method of treatment according to any one of claims 61 to 63, wherein the average number of units of the drug-linker conjugated per antibody molecule in the antibody-drug conjugate is in the range of from 7 to 8.

**65.** The method of treatment according to claim 60, wherein the antibody in the antibody-drug conjugate is an anti-HER3 antibody.

**66.** The method of treatment according to claim 65, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**67.** The method of treatment according to claim 66, wherein the anti-HER3 antibody lacks a lysine residue at the carboxyl terminus of the heavy chain.

**68.** The method of treatment according to any one of claims 65 to 67, wherein the average number of units of the drug-linker conjugated per antibody molecule in the antibody-drug conjugate is in the range of from 7 to 8.

**69.** The method of treatment according to claim 60, wherein the antibody in the antibody-drug conjugate is an anti-TROP2 antibody.

**70.** The method of treatment according to claim 69, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**71.** The method of treatment according to claim 70, wherein the anti-TROP2 antibody lacks a lysine residue at the carboxyl terminus of the heavy chain.

**72.** The method of treatment according to any one of claims 69 to 71, wherein the average number of units of the drug-linker conjugated per antibody molecule in the antibody-drug conjugate is in the range of from 3.5 to 4.5.

**73.** The method of treatment according to claim 60, wherein the antibody in the antibody-drug conjugate is an anti-B7-H3 antibody.

**74.** The method of treatment according to claim 73, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**75.** The method of treatment according to claim 74, wherein the anti-B7-H3 antibody lacks a lysine residue at the carboxyl terminus of the heavy chain.

**76.** The method of treatment according to any one of claims 73 to 75, wherein the average number of units of the drug-linker conjugated per antibody molecule in the antibody-drug conjugate is in the range of from 3.5 to 4.5.

**77.** The method of treatment according to claim 60, wherein the antibody in the antibody-drug conjugate is an anti-GPR20 antibody.

**78.** The method of treatment according to claim 77, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

**79.** The method of treatment according to claim 78, wherein the anti-GPR20 antibody lacks a lysine residue at the carboxyl terminus of the heavy chain.

**80.** The method of treatment according to any one of claims 77 to 79, wherein the average number of units of the drug-linker conjugated per antibody molecule in the antibody-drug conjugate is in the range of from 7 to 8.

**81.** The method of treatment according to claim 60, wherein the antibody in the antibody-drug conjugate is an anti-CDH6 antibody.

**82.** The method of treatment according to claim 81, wherein the anti-CDH6 antibody is an antibody comprising a heavy

chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

**83.** The method of treatment according to claim 82, wherein the anti-CDH6 antibody lacks a lysine residue at the carboxyl terminus of the heavy chain.

**84.** The method of treatment according to any one of claims 81 to 83, wherein the average number of units of the drug-linker conjugated per antibody molecule in the antibody-drug conjugate is in the range of from 7 to 8.

**85.** The method of treatment according to any one of claims 59 to 84, wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

**86.** The method of treatment according to any one of claims 59 to 85, wherein the antibody-drug conjugate and the anti-SIRPα antibody are separately contained as active components in different formulations, and are administered simultaneously or at different times.

**87.** The method of treatment according to any one of claims 59 to 86, wherein the method is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine cancer, sarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome.

**88.** A method of treatment, comprising administering an antibody-drug conjugate and an anti-SIRPα antibody in combination to a subject in need of treatment, wherein the antibody-drug conjugate is an antibody-drug conjugate represented by the following formula:

[Formula 4]

Antibody — [ ... ]n

wherein a drug-linker is conjugated to an antibody via a thioether bond; and n represents the average number of units of the drug-linker conjugated per antibody molecule.

**89.** The method of treatment according to claim 88, wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

**90.** The method of treatment according to claim 89, wherein the antibody in the antibody-drug conjugate is an anti-HER2 antibody.

**91.** The method of treatment according to claim 90, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**92.** The method of treatment according to claim 90, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**93.** The method of treatment according to any one of claims 90 to 92, wherein the average number of units of the drug-linker conjugated per antibody molecule in the antibody-drug conjugate is in the range of from 7 to 8.

**94.** The method of treatment according to claim 89, wherein the antibody in the antibody-drug conjugate is an anti-HER3 antibody.

**95.** The method of treatment according to claim 94, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**96.** The method of treatment according to claim 95, wherein the anti-HER3 antibody lacks a lysine residue at the carboxyl terminus of the heavy chain.

**97.** The method of treatment according to any one of claims 94 to 96, wherein the average number of units of the drug-linker conjugated per antibody molecule in the antibody-drug conjugate is in the range of from 7 to 8.

**98.** The method of treatment according to claim 89, wherein the antibody in the antibody-drug conjugate is an anti-TROP2 antibody.

**99.** The method of treatment according to claim 98, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**100.** The method of treatment according to claim 99, wherein the anti-TROP2 antibody lacks a lysine residue at the carboxyl terminus of the heavy chain.

**101.** The method of treatment according to any one of claims 98 to 100, wherein the average number of units of the drug-linker conjugated per antibody molecule in the antibody-drug conjugate is in the range of from 3.5 to 4.5.

**102.** The method of treatment according to claim 89, wherein the antibody in the antibody-drug conjugate is an anti-B7-H3 antibody.

**103.** The method of treatment according to claim 102, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**104.** The method of treatment according to claim 103, wherein the anti-B7-H3 antibody lacks a lysine residue at the carboxyl terminus of the heavy chain.

**105.** The method of treatment according to any one of claims 102 to 104, wherein the average number of units of the

drug-linker conjugated per antibody molecule in the antibody-drug conjugate is in the range of from 3.5 to 4.5.

**106.** The method of treatment according to claim 89, wherein the antibody in the antibody-drug conjugate is an anti-GPR20 antibody.

**107.** The method of treatment according to claim 106, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

**108.** The method of treatment according to claim 107, wherein the anti-GPR20 antibody lacks a lysine residue at the carboxyl terminus of the heavy chain.

**109.** The method of treatment according to any one of claims 106 to 108, wherein the average number of units of the drug-linker conjugated per antibody molecule in the antibody-drug conjugate is in the range of from 7 to 8.

**110.** The method of treatment according to claim 89, wherein the antibody in the antibody-drug conjugate is an anti-CDH6 antibody.

**111.** The method of treatment according to claim 110, wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

**112.** The method of treatment according to claim 111, wherein the anti-CDH6 antibody lacks a lysine residue at the carboxyl terminus of the heavy chain.

**113.** The method of treatment according to any one of claims 110 to 112, wherein the average number of units of the drug-linker conjugated per antibody molecule in the antibody-drug conjugate is in the range of from 7 to 8.

**114.** The method of treatment according to any one of claims 88 to 113, wherein the anti-SIRPα antibody is any one of the following antibodies (1) to (5):

(1) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 16;
(2) an antibody comprising a heavy chain consisting of an amino acid sequence consisting of amino acid residues 20 to 466 of SEQ ID NO: 13 and a light chain consisting of an amino acid sequence consisting of amino acid residues 21 to 234 of SEQ ID NO: 17;
(3) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 15;
(4) an antibody comprising a heavy chain consisting of an amino acid sequence consisting of amino acid residues 20 to 466 of SEQ ID NO: 14 and a light chain consisting of an amino acid sequence consisting of amino acid residues 21 to 234 of SEQ ID NO: 16; and
(5) an antibody according to any one of (1) to (4), lacking a lysine residue at the carboxyl terminus of the heavy chain.

**115.** The method of treatment according to any one of claims 88 to 114, wherein the antibody-drug conjugate and the anti-SIRPα antibody are separately contained as active components in different formulations, and are administered simultaneously or at different times.

**116.** The method of treatment according to any one of claims 88 to 115, wherein the method is for treating at least one selected from the group consisting of breast cancer, gastric cancer, colorectal cancer, lung cancer, esophageal cancer, salivary gland cancer, gastroesophageal junction adenocarcinoma, biliary tract cancer, Paget's disease, pancreatic cancer, ovarian cancer, bladder cancer, prostate cancer, uterine carcinosarcoma, head and neck cancer, hepatocellular cancer, cervical cancer, brain tumor, glioma, eye tumor, thyroid cancer, thymus cancer, gallbladder cancer, lymphoma, leukemia, and myelodysplastic syndrome

[Figure 1]

SEQ ID NO: 1: Amino acid sequence of heavy chain of anti-HER2 antibody

EVQLVESGGGLVQPGGSLRLSCAASGFNIKDTYIHWVRQAPGKGLEWVARIYPTNGY
TRYADSVKGRFTISADTSKNTAYLQMNSLRAEDTAVYYCSRWGGDGFYAMDYWGQGT
LVTVSSASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK

[Figure 2]

SEQ ID NO: 2: Amino acid sequence of light chain of anti-HER2 antibody

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC

[Figure 3]

SEQ ID NO: 3: Amino acid sequence of heavy chain of anti-HER3 antibody

QVQLQQWGAGLLKPSETLSLTCAVYGGSFSGYYWSWIRQPPGKGLEWIGEINHSGST
NYNPSLKSRVTISVETSKNQFSLKLSSVTAADTAVYYCARDKWTWYFDLWGRGTLVT
VSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPP
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPGK

[Figure 4]

SEQ ID NO: 4: Amino acid sequence of light chain of anti-HER3 antibody

DIEMTQSPDSLAVSLGERATINCRSSQSVLYSSSNRNYLAWYQQNPGQPPKLLIYWA
STRESGVPDRFSGSGSGTDFTLTISSLQAEDVAVYYCQQYYSTPRTFGQGTKVEIKR
TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

[Figure 5]

SEQ ID NO: 5: Amino acid sequence of heavy chain of anti-TROP2 antibody

MKHLWFFLLLVAAPRWVLSQVQLVQSGAEVKKPGASVKVSCKASGYTFTTAGMQWVR
QAPGQGLEWMGWINTHSGVPKYAEDFKGRVTISADTSTSTAYLQLSSLKSEDTAVYY
CARSGFGSSYWYFDVWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMHEALH
NHYTQKSLSLSPGK

Signal sequence (1-19), Variable region (20-140), Constant region (141-470)

[Figure 6]

SEQ ID NO: 6: Amino acid sequence of light chain of anti-TROP2 antibody

MVLQTQVFISLLLWISGAYGDIQMTQSPSSLSASVGDRVTITCKASQDVSTAVAWYQ
QKPGKAPKLLIYSASYRYTGVPSRFSGSGSGTDFTLTISSLQPEDFAVYYCQQHYIT
PLTFGQGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

Signal sequence (1-20), Variable region (21-129), Constant region (130-234)

[Figure 7]

SEQ ID NO: 7: Amino acid sequence of heavy chain of anti-B7-H3 antibody

MKHLWFFLLLVAAPRWVLSQVQLVQSGAEVKKPGSSVKVSCKASGYTFTNYVMHWVR
QAPGQGLEWMGYINPYNDDVKYNEKFKGRVTITADESTSTAYMELSSLRSEDTAVYY
CARWGYYGSPLYYFDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNH
KPSNTKVDKRVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEAL
HNHYTQKSLSLSPGK

Signal sequence (1-19), Variable region (20-141), Constant region (142-471)

[Figure 8]

SEQ ID NO: 8: Amino acid sequence of light chain of anti-B7-H3 antibody

MVLQTQVFISLLLWISGAYGEIVLTQSPATLSLSPGERATLSCRASSRLIYMHWYQQ
KPGQAPRPLIYATSNLASGIPARFSGSGSGTDFTLTISSLEPEDFAVYYCQQWNSNP
PTFGQGTKVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVTKSF
NRGEC

Signal sequence (1-20), Variable region (21-128), Constant region (129-233)

[Figure 9]

SEQ ID NO: 9: Amino acid sequence of heavy chain of anti-GPR20 antibody

MKHLWFFLLLVAAPRWVLSEVQLVQSGAEVKKPGASVKVSCKASGYTFTSYYISWIR
QAPGQGLKYMGFINPGSGHTNYNEKFKGRVTITADKSSSTATMELSSLRSEDTAVYY
CARGAGGFLRIITKFDYWGQGTLVTVSSASTKGPSVFPLAPSSKSTSGGTAALGCLV
KDYFPEPVTVSWNSGALTSGVHTFPAVLQSSGLYSLSSVVTVPSSSLGTQTYICNVN
HKPSNTKVDKRVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQDWLNGK
EYKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHEA
LHNHYTQKSLSLSPGK

Signal sequence (1-19), Variable region (20-142), Constant region (143-472)

[Figure 10]

SEQ ID NO: 10: Amino acid sequence of light chain of anti-GPR20 antibody

MVLQTQVFISLLLWISGAYGDTQLTQSPSSLSASVGDRVTITCRASKSVSTYIHWYQ
QKPGKQPKLLIYSAGNLESGVPSRFSGSGSGTDFTLTISSLQPEDFANYYCQQINEL
PYTFGQGTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

Signal sequence (1-20), Variable region (21-129), Constant region (130-234)

[Figure 11]

SEQ ID NO: 11: Amino acid sequence of heavy chain of anti-CDH6 antibody

MKHLWFFLLLVAAPRWVLSEVQLVQSGAEVKKPGASVKVSCKASGYTFTRNFMHWVR
QAPGQGLEWMGWIYPGDGETEYAQKFQGRVTITADTSTSTAYMELSSLRSEDTAVYY
CARGVYGGFAGGYFDFWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNH
KPSNTKVDKRVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEAL
HNHYTQKSLSLSPGK

Signal sequence (1-19), Variable region (20-141), Constant region (142-471)

[Figure 12]

SEQ ID NO: 12: Amino acid sequence of light chain of anti-CDH6 antibody

MVLQTQVFISLLLWISGAYGDIQMTQSPSSLSASVGDRVTITCKASQNIYKNLAWYQ
QKPGKAPKLLIYDANTLQTGVPSRFSGSGSGSDFTLTISSLQPEDFATYFCQQYYSG
WAFGQGTKVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVTKSF
NRGEC

Signal sequence (1-20), Variable region (21-128), Constant region (129-233)

[Figure 13]

SEQ ID NO: 13: Amino acid sequence of hH1

MKHLWFFLLLVAAPRWVLSQVQLVESGGGVVQPGRSLRLSCAASGFTFSDYGMIWVR
QAPGKGLEWVASISSSSSYIYYADSVKGRFTISRDNSKNRLYLQMNSLRAEDTAVYY
CARRYYGFNYPFDYWGQGTMVTVSSASTKGPSVFPLAPCSRSTSESTAALGCLVKDY
FPEPVTVSWNSGALTSGVHTFPAVLQSSGLYSLSSVVTVPSSSLGTKTYTCNVDHKP
SNTKVDKRVESKYGPPCPPCPAPEAAGGPSVFLFPPKPKDTLMISRTPEVTCVVVDV
SQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALHNHYT
QKSLSLSLGK

Signal sequence (1-19), Variable region (20-139), Constant region (140-466)

[Figure 14]

SEQ ID NO: 14: Amino acid sequence of hH2

MKHLWFFLLLVAAPRWVLSEVQLVESGGGVVQPGRSLRLSCAASGFTFSDYGMIWVR
QAPGKGLEWVASISSSSSYIYYADSVKGRFTISRDNSKNTLYLQMNSLRAEDTAVYY
CARRYYGFNYPFDYWGQGTMVTVSSASTKGPSVFPLAPCSRSTSESTAALGCLVKDY
FPEPVTVSWNSGALTSGVHTFPAVLQSSGLYSLSSVVTVPSSSLGTKTYTCNVDHKP
SNTKVDKRVESKYGPPCPPCPAPEAAGGPSVFLFPPKPKDTLMISRTPEVTCVVVDV
SQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALHNHYT
QKSLSLSLGK

Signal sequence (1-19), Variable region (20-139), Constant region (140-466)

[Figure 15]

SEQ ID NO: 15: Amino acid sequence of hL2

MVLQTQVFISLLLWISGAYGAIQLTQSPSSLSASVGQRVTITCGASKSVRTYMHWYQ
QKPGKAPKLLIYSASNLEAGVPSRFSGSGSGTDFTLTISSLQPEDFATYYCQQSNEP
PYTFGQGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

Signal sequence (1-20), Variable region (21-127), Constant region (128-234)

[Figure 16]

SEQ ID NO: 16: Amino acid sequence of hL3

MVLQTQVFISLLLWISGAYGDTQLTQSPSSLSASVGQRVTITCGASKSVRTYMHWYQ
QKPGKQPKLLIYSASNLEAGVPSRFSGSGSGTDFTLTISSLQPEDFATYYCQQSNEP
PYTFGQGTKLEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

Signal sequence (1-20), Variable region (21-127), Constant region (128-234)

[Figure 17]

SEQ ID NO: 17: Amino acid sequence of hL4

MVLQTQVFISLLLWISGAYGDTVLTQSPDSLAVSLGQRATINCGASKSVRTYMHWYQ
QKPGQQPKLLIYSASNLEAGVPSRFSGSGSGTDFTLTISSLQAEDVAVYYCQQSNEP
PYTFGQGTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

Signal sequence (1-20), Variable region (21-127), Constant region (128-234)

[Figure 18]

SEQ ID NO: 18: D13 CDRH1

GFTFSDYGMI

SEQ ID NO: 19: D13 CDRH2

SISSSSSYIY

SEQ ID NO: 20: D13 CDRH3

RYYGFNYPFDY

SEQ ID NO: 21: D13 CDRL1

GASKSVRTYMH

SEQ ID NO: 22: D13 CDRL2

SASNLEA

SEQ ID NO: 23: D13 CDRL3

QQSNEPPYT

[Figure 19]

SEQ ID NO: 24: Amino acid sequence of heavy chain of OSE-172 antibody (OSE-172_hG4Pro)

MKHLWFFLLLVAAPRWVLSEVQLVQSGAEVKKPGESLRISCKASGYSFTSYWVHWVR
QMPGKGLEWMGNIDPSDSDTHYSPSFQGHVTLSVDKSISTAYLQLSSLKASDTAMYY
CVRGGTGTLAYFAYWGQGTLVTVSSASTKGPSVFPLAPCSRSTSESTAALGCLVKDY
FPEPVTVSWNSGALTSGVHTFPAVLQSSGLYSLSSVVTVPSSSLGTKTYTCNVDHKP
SNTKVDKRVESKYGPPCPPCPAPEFLGGPSVFLFPPKPKDTLMISRTPEVTCVVVDV
SQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALHNHYT
QKSLSLSPGK*

SEQ ID NO: 25: Amino acid sequence of light chain of OSE-172 antibody (OSE-172_hK)

MVLQTQVFISLLLWISGAYGDVVMTQSPLSLPVTLGQPASISCRSSQSLVHSYGNTY
LYWFQQRPGQSPRLLIYRVSNRFSGVPDRFSGSGSGTDFTLKISRVEAEDVGVYYCF
QGTHVPYTFGGGTKVEIKRTVAAPSVFIFPPSDEQLKSGTASVVCLLNNFYPREAKV
QWKVDNALQSGNSQESVTEQDSKDSTYSLSSTLTLSKADYEKHKVYACEVTHQGLSS
PVTKSFNRGEC*

[Figure 20]

SEQ ID NO: 26: Amino acid sequence of heavy chain of KWAR23 antibody (KWAR23_hG4Pro)

MKHLWFFLLLVAAPRWVLSEVQLQQSGAELVKPGASVKLSCTASGFNIKDYYIHWVQ
QRTEQGLEWIGRIDPEDGETKYAPKFQDKATITADTSSNTAYLHLSSLTSEDTAVYY
CARWGAYWGQGTLVTVSAASTKGPSVFPLAPCSRSTSESTAALGCLVKDYFPEPVTV
SWNSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVSNKGLPS
SIEKTISKAKGQPREPQVYTLPPSQEEMTKNQVSLTCLVKGFYPSDIAVEWESNGQP
ENNYKTTPPVLDSDGSFFLYSRLTVDKSRWQEGNVFSCSVMHEALHNHYTQKSLSLS
LGK*

SEQ ID NO: 27: Amino acid sequence of light chain of KWAR23 antibody (KWAR23_hK)

MVLQTQVFISLLLWISGAYGQIVLTQSPAIMSASPGEKVTLTCSASSSVSSSYLYWY
QQKPGSSPKLWIYSTSNLASGVPARFSGSGSGTSYSLTISSMEAEDAASYFCHQWSS
YPRTFGAGTKLELKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTK
SFNRGEC*

[Figure 21]

SEQ ID NO: 28: Amino acid sequence of heavy chain of ADU-1805 antibody (ADU-1805_hG2)

MKHLWFFLLLVAAPRWVLSEVQLVQSGAEVVKPGASVKLSCKASGSTFTSYWMHWVK
QAPGQGLEWIGAIYPVNSDTTYNQKFKGKATLTVDKSASTAYMELSSLRSEDTAVYY
CTRSFYYSLDAAWFVYWGQGTLVTVSSASTKGPSVFPLAPCSRSTSESTAALGCLVK
DYFPEPVTVSWNSGALTSGVHTFPAVLQSSGLYSLSSVVTVPSSNFGTQTYTCNVDH
KPSNTKVDKTVERKCCVECPPCPAPPVAGPSVFLFPPKPKDTLMISRTPEVTCVVVD
VSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAV
EWESNGQPENNYKTTPPMLDSDGSFFLYSKLTVDKSRWQQGNVFSCSVMHEALHNHY
TQKSLSLSPGK*

SEQ ID NO: 29: Amino acid sequence of light chain of ADU-1805 antibody (ADU-1805_hK)

MVLQTQVFISLLLWISGAYGDIQMTQSPSSLSASVGDRVTITCRASQDIGSRLNWLQ
QKPGKAPKRLIYATSSLDSGVPSRFSGSGSGTEFTLTISSLQPEDFATYYCLQYASS
PFTFGGGTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC*

[Figure 22]

SEQ ID NO: 30:

Amino acid sequence of heavy chain of anti-mouse SIRPα antibody 5C12_mIgG1_D265A

MKHLWFFLLLVAAPRWVLSEVQLVESGGGLVQPGGSLKLSCAASGFTFSNYDMAWVR
QAPTKGLEWVASISTRGGSSYYRDSVKGRFTVSRDNAKSTLYLQMDSLRSEDTATYY
CARHPGDYFDYWGQGVMVTVSSAKTTPPSVYPLAPGSAAQTNSMVTLGCLVKGYFPE
PVTVTWNSGSLSSGVHTFPAVLQSDLYTLSSSVTVPSSTWPSETVTCNVAHPASSTK
VDKKIVPRDCGCKPCICTVPEVSSVFIFPPKPKDVLTITLTPKVTCVVVAISKDDPE
VQFSWFVDDVEVHTAQTQPREEQFNSTFRSVSELPIMHQDWLNGKEFKCRVNSAAFP
APIEKTISKTKGRPKAPQVYTIPPPKEQMAKDKVSLTCMITDFFPEDITVEWQWNGQ
PAENYKNTQPIMDTDGSYFVYSKLNVQKSNWEAGNTFTCSVLHEGLHNHHTEKSLSH
SPGK*

SEQ ID NO: 31:

Amino acid sequence of light chain of anti-mouse SIRPα antibody 5C12_mIgG1_D265A.

MVLQTQVFISLLLWISGAYGDTVLTQSPALAVSPGERVTISCRASESVSTLIHWYQQ
KPGQQPKLLIYLTSHLESGVPARFSGSGSGTDFTLTIDPVEADDTATYYCQQSWTHP
WTFGGGTKLELKRADAAPTVSIFPPSSEQLTSGGASVVCFLNNFYPKDINVKWKIDG
SERQNGVLNSWTDQDSKDSTYSMSSTLTLTKDEYERHNSYTCEATHKTSTSPIVKSF
NRNEC*

[Figure 23]

SEQ ID NO: 32:

Amino acid sequence of heavy chain of anti-PD-L1 antibody YW243.55S70_mIgG1

MKHLWFFLLLVAAPRWVLSEVQLVESGGGLVQPGGSLRLSCAASGFTFSDSWIHWVR
QAPGKGLEWVAWISPYGGSTYYADSVKGRFTISADTSKNTAYLQMNSLRAEDTAVYY
CARRHWPGGFDYWGQGTLVTVSAAKTTPPSVYPLAPGSAAQTNSMVTLGCLVKGYFP
EPVTVTWNSGSLSSGVHTFPAVLQSDLYTLSSSVTVPSSTWPSETVTCNVAHPASST
KVDKKIVPRDCGCKPCICTVPEVSSVFIFPPKPKDVLTITLTPKVTCVVVDISKDDP
EVQFSWFVDDVEVHTAQTQPREEQFNSTFRSVSELPIMHQDWLNGKEFKCRVNSAAF
PAPIEKTISKTKGRPKAPQVYTIPPPKEQMAKDKVSLTCMITDFFPEDITVEWQWNG
QPAENYKNTQPIMDTDGSYFVYSKLNVQKSNWEAGNTFTCSVLHEGLHNHHTEKSLS
HSPGK*

SEQ ID NO: 33:

Amino acid sequence of light chain of anti-PD-L1 antibody YW243.55S70_mIgG1

MVLQTQVFISLLLWISGAYGDIQMTQSPSSLSASVGDRVTITCRASQDVSTAVAWYQ
QKPGKAPKLLIYSASFLYSGVPSRFSGSGSGTDFTLTISSLQPEDFATYYCQQYLYH
PATFGQGTKVEIKRADAAPTVSIFPPSSEQLTSGGASVVCFLNNFYPKDINVKWKID
GSERQNGVLNSWTDQDSKDSTYSMSSTLTLTKDEYERHNSYTCEATHKTSTSPIVKS
FNRNEC*

[Figure 24]

MTX
Compound (A)
A
B
C
D
ATP
HMGB1
RLU
HMGB1 (ng/mL)
Concentration (μM)
N=3
** P<0.01
*** P<0.0001

[Figure 25]

MFI
Ctrl. IgG [N=3]
anti-CARL [N=3]
*** P<0.0001
Medium
Medium + DMSO
MTX 1 μM
Compound (A) 4 μM

[Figure 26]

HMGB1 (ng/mL)
70
60
50
40
30
20
10
0
Medium
MTX 1μM
Compound (A) 4 μM

[Figure 27]

A
Vaccination
Re-Challenge study
Treated with agent CT26.WT 5x10^5/head(right)
Not treated with agent CT26.WT 3x10^6/head(left)
Vaccination Determination of effect
Balb/C n=6
Day 0 1 5 7 14
Dosing 10mpk aSIRPα/aCD47
B
* P < 0.05
Tumor volume (mm3)
800
700
600
500
400
300
200
100
0
PBS
aSIRPα
aCD47
MTX (Positive control)
Compound (A)
NCD (Negative control)
N=6
C

| Treatment of cells | Treatment of mice | CR (left) | CR (right) |
|---|---|---|---|
| MTX (Positive control) | PBS | 2/6 | 6/6 |
| | aSIRPα | 4/6 | 6/6 |
| | aCD47 | 3/6 | 6/6 |
| Compound (A) | PBS | 5/6 | 2/6 |
| | aSIRPα | 6/6 | 2/6 |
| | aCD47 | 6/6 | 1/6 |
| NCD (Negative control) | PBS | 1/6 | 6/6 |
| | aSIRPα | 1/6 | 6/6 |
| | aCD47 | 1/6 | 6/6 |

[Figure 28]

*
*
160
140
120
100
80
60
40
20
0
Number of IFNγ Spots
PBS
aSIRPa
aCD47
PBS
aSIRPa
aCD47
PBS
MTX (Positive control)
Compound (A)
NCD (Negative control)
N=6
* P < 0.05

[Figure 29]

A
CD4+Tcm
100
80
60
40
20
0
% of CD4T
***
***
PBS
PBS
PBS
MTX (p.c.)
Compound (A)
NCD (n.c.)
B
CD8+Tcm
100
80
60
40
20
0
% of CD8T
***
***
PBS
PBS
PBS
MTX (p.c.)
Compound (A)
NCD (n.c.)
N=6
*** P < 0.0005

[Figure 30]

A
CD4+Tcm
% of CD4T
PBS
aSIRPa
aCD47
MTX (p.c.)
DXd
NCD (n.c.)
***
N=6
B
CD8+Tcm
% of CD8T
*
**
N=6
C
% of Whole cells
Other
CD8
CD4
Compound (A)
N=6
Tcm : central memory T cells [CD44(+) CD62L(+)]
* P < 0.05

[Figure 31]

A
HER2
N=3
ADCP (%)
No treatment
Control IgG
αSIRPα_2000
Antibody-drug conjugate (1) _10
Antibody-drug conjugate (1) _100
Antibody-drug conjugate (1) _1000
Control antibody-drug conjugate
Tmab
+αSIRPα
B
TROP2
Antibody-drug conjugate (2) _10
Antibody-drug conjugate (2) _100
Antibody-drug conjugate (2) _1000
αTROP2_10
αTROP2_100
αTROP2_1000

C
HER2
ADCP (%)
Antibody-drug conjugate (1)
Control antibody-drug conjugate
αSIRPα Ab Conc. (ng/mL)
N=3
D
TROP2
Antibody-drug conjugate (2)

[Figure 32]

A

4T1-HER2
1x10^6/head
Antibody-drug
conjugate (1)
10 mpk/ i.v./ qw
Balb/C
N=5
Day
0
4
5
8
11
12
15
18
αSIRPα 10 mpk/ i.p./ biw
αPD-L1 10 mpk/ i.p./ biw

B

* P<0.05
Tumor volume (mm3)
Days after inoculation
PBS
Antibody-drug conjugate (1)
αSIRPα
αPD-L1
Antibody-drug conjugate (1) +αSIRPα
Antibody-drug conjugate (1) +αPD-L1
Antibody-drug conjugate (1) +αPD-L1+αSIRPα
N=5

C

| Treatment | TGI % (d21) | CR mice |
|---|---|---|
| PBS | - | 0/5 |
| Antibody-drug conjugate (1) | 74 | 0/5 |
| αPD-L1 | 4 | 0/5 |
| αSIRPα | 41 | 0/5 |
| Antibody-drug conjugate (1) +αPD-L1 | 75 | 0/5 |
| Antibody-drug conjugate (1) +αSIRPα | 88 | 0/5 |
| Antibody-drug conjugate (1) +αPD-L1+αSIRPα | 90 | 1/5 |

[Figure 33]

A
CT26.WT-hTROP2
2x10^6/head
Antibody-drug
conjugate (2)
20 mpk/ i.v./ qw
Balb/C
N=6
Day
0
7
8
11
12
13
15
18
αSIRPα 10 mpk/ i.p./ biw
B
*** P<0.0001
Tumor volume (mm3)
1400
1200
1000
800
600
400
200
0
7
11
14
18
day
***
PBS
Antibody-drug
conjugate (2)
αSIRPα
Antibody-drug
conjugate (2)
+αSIRPα
N=6
C

| Treatment | TGI %(d18) |
| --- | --- |
| PBS | - |
| Antibody-drug conjugate (2) | 31 |
| αSIRPα | 31 |
| Antibody-drug conjugate (2) +αSIRPα | 58 |

[Figure 34]

A
CT26.WT-hHER3
2x10^6/head
Antibody-drug conjugate (3) 10 mpk/ i.v./ qw
Balb/C
N=6
Day
0
7
8
11
12
14
15
18
αSIRPα 10 mpk/ i.p./ biw

B

Tumor volume (mm3)

Days after inoculation

--O-- vehicle

Antibody-drug conjugate (3)

αSIRPα

Antibody-drug conjugate (3) +αSIRPα

C

| Treatment | TGI % (d18) |
|---|---|
| PBS | - |
| Antibody-drug conjugate (3) | 21 |
| αSIRPα | -41 |
| Antibody-drug conjugate (3) +αSIRPα | 46 |

## INTERNATIONAL SEARCH REPORT

International application No. **PCT/JP2021/041255**

**A. CLASSIFICATION OF SUBJECT MATTER**

***A61K 47/68***(2017.01)i; ***A61K 31/4745***(2006.01)i; ***A61K 39/395***(2006.01)i; ***A61K 47/55***(2017.01)i; ***A61P 35/00***(2006.01)i; ***A61P 35/02***(2006.01)i; ***A61P 43/00***(2006.01)i
FI: A61K47/68; A61P35/02; A61P43/00 121; A61K47/55; A61K39/395 T; A61K39/395 E; A61K31/4745; A61P35/00 ZNA

According to International Patent Classification (IPC) or to both national classification and IPC

**B. FIELDS SEARCHED**

Minimum documentation searched (classification system followed by classification symbols)

A61K47/00-47/69; A61K31/00-33/44; A61K39/00-39/44

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY/MEDLINE/EMBASE/BIOSIS (STN); GenBank/EMBL/DDBJ/GeneSeq; UniProt/GeneSeq; SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | WO 2019/044947 A1 (DAIICHI SANKYO CO LTD) 07 March 2019 (2019-03-07) claims 51-52, paragraphs [0157]-[0161], [0400], [0404] | 1-22, 27-51, 56-80, 85-109, 114-116 |
| Y | WO 2020/013170 A1 (UNIV KOBE NAT UNIV CORP) 16 January 2020 (2020-01-16) claims 1, 22, 32-33 | 1-116 |
| Y | WO 2020/022363 A1 (DAIICHI SANKYO CO LTD) 30 January 2020 (2020-01-30) claims 1, 22, 24-35, 45, 60, 62-73, paragraphs [0257], [0261] | 1-10, 19-39, 48-68, 77-97, 106-116 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 December 2021** | **14 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/041255**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2021/041255**

| Patent document cited in search report | Publication date (day/month/year) | Patent family member(s) | Publication date (day/month/year) |
|---|---|---|---|
| WO 2019/044947 A1 | 07 March 2019 | US 2020/0385422 A1 claims 51-52, paragraph [0021] (paragraphs [0079]-[0095]) paragraphs [0253], [0257] | |
| | | EP 3677589 A1 | |
| | | CN 111051330 A | |
| | | KR 10-2020-0041993 A | |
| WO 2020/013170 A1 | 16 January 2020 | US 2021/0155707 A1 claims 1, 22, 32-33 | |
| | | EP 3822289 A1 | |
| WO 2020/022363 A1 | 30 January 2020 | EP 3828206 A1 claims 1, 22, 24-35, 45, 60, 62-73, paragraphs [0164], [0168] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017178653 A
- WO 2018026600 A
- WO 2018190719 A
- WO 2020013170 A
- WO 2014057687 A
- WO 2014061277 A
- WO 2015098099 A
- WO 2015115091 A
- WO 2015146132 A
- WO 2015155976 A
- WO 2015155998 A
- WO 9007861 A
- US 5821337 A
- WO 9954342 A
- WO 0061739 A
- WO 0231140 A
- WO 2007133855 A
- WO 2013120066 A
- WO 0100245 A
- WO 2007077028 A
- WO 2008100624 A
- WO 2018135501 A
- WO 2018212136 A
- WO 2019044947 A
- WO 88007089 A
- WO 9428027 A
- WO 9429351 A
- WO 2017002776 A
- US 20130045201 A1


### Non-patent literature cited in the description


- **MATOZAKI et al.** *Trends in cell biol.,* 2009, vol. 2 (19), 72-80
- **TAKENAKA et al.** *Nat Immunol.,* 2007, vol. 12 (8), 1313-1323
- **MATOZAKI et al.** *Biochem.,* 2014, vol. 6 (155), 335-344
- **OGITANI Y. et al.** *Clinical Cancer Research,* 2016, vol. 22 (20), 5097-5108
- **OGITANI Y. et al.** *Cancer Science,* 2016, vol. 107, 1039-1046
- **DOI T et al.** *Lancet Oncol,* 2017, vol. 18, 1512-22
- **TAKEGAWA N et al.** *Int. J. Cancer,* 2017, vol. 141, 1682-1689
- **OGITANI Y. et al.** *Clinical Cancer Research,* 29 March 2016, vol. 22 (20), 5097-5108
- *Cell Death and Differentiation,* 2008, vol. 15, 751-761
- *Molecular Biology of the Cell,* December 2004, vol. 15, 5268-5282
- *Bio Techniques,* January 2000, vol. 28, 162-165
- **KOHLER ; MILSTEIN.** *Nature,* 1975, vol. 256, 495-497
- Monoclonal Antibodies. Plenum Press, 1980, 365-367
- *Proc. Natl. Acad. Sci. USA,* 1984, vol. 81, 6851-6855
- *Nature,* 1986, vol. 321, 522-525
- **TOMIZUKA, K. et al.** *Nature Genetics,* 1997, vol. 16, 133-143
- **KUROIWA, Y.** *Nucl. Acids Res.,* 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- **WORMSTONE, I. M.** *Investigative Ophthalmology & Visual Science,* 2002, vol. 43 (7), 2301-2308
- **CARMEN, S.** *Briefings in Functional Genomics and Proteomics,* 2002, vol. 1 (2), 189-203
- **SIRIWARDENA, D.** *Ophthalmology,* 2002, vol. 109 (3), 427-431
- *Journal of Chromatography A,* 1995, vol. 705, 129-134
- *Analytical Biochemistry,* 2007, vol. 360, 75-83
- **HERMANSON, G. T.** Bioconjugate Techniques. Academic Press, 1996, 56-136, 456-493
- **BRUGGEMANN et al.** *J. Exp. Med.,* 1987, 1351-1361
- **KABAT.** Sequences of proteins of immunological interest. 1991
- **PAREKH et al.** *mAbs,* 2012, 310-318

- **ANGAL.** *Molecular Immunology,* 1993, 105-108
- **VAFA.** *Methods,* 2014, vol. 65, 114-126
- **TSUBAKI.** *Int. J. Biol. Macromol,* 2013, 139-147
- *Nature Reviews Immunology,* 2017, vol. 17, 97-111
- *Cancer Research,* 2017, vol. 77, 2686-2698
- *ONCOIMMUNOLOGY,* 2019, vol. 8 (4), e1565859
- *Clin. Invest.,* 2020, vol. 130 (1), 374-388
- **YANAGITA et al.** *JCI Insight,* 2017, vol. 1 (2), 1-15